# EUROPEAN PATENT APPLICATION

(11) **EP 3 715 358 A1**
(43) Date of publication of application: **30.09.2020**
(21) Application number: 19165681.8
(22) Date of filing: 28.03.2019
(51) Int. Cl.: C07K 14/015, C12N 15/35, C12N 5/10, C12N 15/86, C07K 14/005, C12N 15/10

(54) **MUTATED ADENO-ASSOCIATED VIRUS CAPSID PROTEINS, AAV PARTICLE COMPRISING THE SAME AND LIVER DIRECTED AAV VECTOR GENE THERAPY**

(71) Applicant: Universität zu Köln, 50923 Köln (DE)
(72) Inventor: BÜNING, Hildegard, 30625 Hannover (DE); MEUMANN, Nadja, 30159 Hannover (DE); GONZALEZ-CARMONA, Maria, 80686 München (DE); STRASSBURG, Christian P., 80686 München (DE); VOGT, Annabelle, 80686 München (DE); PIIPER, Albrecht, 80686 München (DE); SCHWÄBLE, Joachim, 80686 München (DE); HUBER, Karin, 80686 München (DE); SEIFRIED, Erhard, 80686 München (DE)
(74) Representative: Gramm, Lins & Partner Patent- und Rechtsanwälte PartGmbB

(57) **Abstract**

The present invention relates in a first aspect to a mutated adeno-associated virus (AAV) capsid protein or fragment thereof having an insert composed of an oligopeptide optionally having further amino acids representing linker sequences flanking both sites of said oligopeptide. These mutated AAV capsid proteins are more efficient in targeting hepatic tissue, hepatocytes, hepatic cells and cell lines or hepatocellular carcinoma (HCC) with partially higher specificity. Further, a mutated AAV particle comprising the mutated AAV capsid protein according to the present invention is provided. In addition, a nucleic acid encoding the mutated AAV capsid protein according to the present invention is identified together with a corresponding nucleic acid vector, in particular, a plasmid. In addition, a host cell containing the nucleic acid vector or the nucleic acid molecule according to the present invention. Further, a use of the AAV particle or the nucleic acid or the nucleic acid vector according to the present invention in the manufacture of an AAV particle, or in the manufacture of a medicament for gene therapy is described. Moreover, the described protein, particle molecules as well as nucleic acid vectors for use in targeting hepatocytes and/or HCC are described. In particular, said components for use in treating diseases involving hepatocytes or for treating HCC are disclosed, in particular, for use in gene therapy, e.g. for use in a transfer of a gene of interest into hepatocytes, heptatic tissue or HCC.

SEQUENCE LISTING

<110>  Universitaet zu Koeln

<120>  Mutated adeno-associated virus capsid proteins, AAV particle
       comprising the same and liver directed AAV  vector gene therapy

<130>  4677-009 EP-1

<160>  538

<170>  PatentIn version 3.5

<210>  1
<211>  736
<212>  PRT
<213>  AAV-1

<400>  1

Met Ala Ala Asp Gly Tyr Leu Pro Asp Trp Leu Glu Asp Asn Leu Ser
1               5                   10                  15

Glu Gly Ile Arg Glu Trp Trp Asp Leu Lys Pro Gly Ala Pro Lys Pro
            20                  25                  30

Lys Ala Asn Gln Gln Lys Gln Asp Asp Gly Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Ala Ala Asp Ala Ala Ala Leu Glu His Asp Lys Ala Tyr Asp
65                  70                  75                  80

Gln Gln Leu Lys Ala Gly Asp Asn Pro Tyr Leu Arg Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Gly Ala Lys Thr Ala Pro Gly Lys Lys Arg
    130                 135                 140

Pro Val Glu Gln Ser Pro Gln Glu Pro Asp Ser Ser Ser Gly Ile Gly
145                 150                 155                 160

Lys Thr Gly Gln Gln Pro Ala Lys Lys Arg Leu Asn Phe Gly Gln Thr
                165                 170                 175

Gly Asp Ser Glu Ser Val Pro Asp Pro Gln Pro Leu Gly Glu Pro Pro
            180                 185                 190

Ala Thr Pro Ala Ala Val Gly Pro Thr Thr Met Ala Ser Gly Gly Gly
        195                 200                 205

Ala Pro Met Ala Asp Asn Asn Glu Gly Ala Asp Gly Val Gly Asn Ala
    210                 215                 220

Ser Gly Asn Trp His Cys Asp Ser Thr Trp Leu Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Ser Ala Ser Thr Gly Ala Ser Asn Asp Asn His
            260                 265                 270

Tyr Phe Gly Tyr Ser Thr Pro Trp Gly Tyr Phe Asp Phe Asn Arg Phe
        275                 280                 285

His Cys His Phe Ser Pro Arg Asp Trp Gln Arg Leu Ile Asn Asn Asn
    290                 295                 300

Trp Gly Phe Arg Pro Lys Arg Leu Asn Phe Lys Leu Phe Asn Ile Gln
305                 310                 315                 320

Val Lys Glu Val Thr Thr Asn Asp Gly Val Thr Thr Ile Ala Asn Asn
                325                 330                 335

Leu Thr Ser Thr Val Gln Val Phe Ser Asp Ser Glu Tyr Gln Leu Pro
            340                 345                 350

Tyr Val Leu Gly Ser Ala His Gln Gly Cys Leu Pro Pro Phe Pro Ala
        355                 360                 365

Asp Val Phe Met Ile Pro Gln Tyr Gly Tyr Leu Thr Leu Asn Asn Gly
    370                 375                 380

Ser Gln Ala Val Gly Arg Ser Ser Phe Tyr Cys Leu Glu Tyr Phe Pro
385                 390                 395                 400

Ser Gln Met Leu Arg Thr Gly Asn Asn Phe Thr Phe Ser Tyr Thr Phe
                405                 410                 415

Glu Glu Val Pro Phe His Ser Ser Tyr Ala His Ser Gln Ser Leu Asp
            420                 425                 430

Arg Leu Met Asn Pro Leu Ile Asp Gln Tyr Leu Tyr Tyr Leu Asn Arg
        435                 440                 445

Thr Gln Asn Gln Ser Gly Ser Ala Gln Asn Lys Asp Leu Leu Phe Ser
    450                 455                 460

Arg Gly Ser Pro Ala Gly Met Ser Val Gln Pro Lys Asn Trp Leu Pro
465                 470                 475                 480

Gly Pro Cys Tyr Arg Gln Gln Arg Val Ser Lys Thr Lys Thr Asp Asn
                485                 490                 495

Asn Asn Ser Asn Phe Thr Trp Thr Gly Ala Ser Lys Tyr Asn Leu Asn
            500                 505                 510

Gly Arg Glu Ser Ile Ile Asn Pro Gly Thr Ala Met Ala Ser His Lys
        515                 520                 525

Asp Asp Glu Asp Lys Phe Phe Pro Met Ser Gly Val Met Ile Phe Gly
    530                 535                 540

Lys Glu Ser Ala Gly Ala Ser Asn Thr Ala Leu Asp Asn Val Met Ile
545                 550                 555                 560

Thr Asp Glu Glu Glu Ile Lys Ala Thr Asn Pro Val Ala Thr Glu Arg
                565                 570                 575

Phe Gly Thr Val Ala Val Asn Phe Gln Ser Ser Ser Thr Asp Pro Ala
            580                 585                 590

Thr Gly Asp Val His Ala Met Gly Ala Leu Pro Gly Met Val Trp Gln
        595                 600                 605

Asp Arg Asp Val Tyr Leu Gln Gly Pro Ile Trp Ala Lys Ile Pro His
    610                 615                 620

Thr Asp Gly His Phe His Pro Ser Pro Leu Met Gly Gly Phe Gly Leu
625                 630                 635                 640

Lys Asn Pro Pro Pro Gln Ile Leu Ile Lys Asn Thr Pro Val Pro Ala
                645                 650                 655

Asn Pro Pro Ala Glu Phe Ser Ala Thr Lys Phe Ala Ser Phe Ile Thr
            660                 665                 670

Gln Tyr Ser Thr Gly Gln Val Ser Val Glu Ile Glu Trp Glu Leu Gln

675                 680                 685

Lys Glu Asn Ser Lys Arg Trp Asn Pro Glu Val Gln Tyr Thr Ser Asn
    690                 695                 700

Tyr Ala Lys Ser Ala Asn Val Asp Phe Thr Val Asp Asn Asn Gly Leu
705                 710                 715                 720

Tyr Thr Glu Pro Arg Pro Ile Gly Thr Arg Tyr Leu Thr Arg Pro Leu
                725                 730                 735

<210>  2
<211>  735
<212>  PRT
<213>  AAV-2

<400>  2

<210>  3
<211>  736
<212>  PRT
<213>  AAV-3b

<400>  3

Met Ala Ala Asp Gly Tyr Leu Pro Asp Trp Leu Glu Asp Asn Leu Ser
1               5                   10                  15

Glu Gly Ile Arg Glu Trp Trp Ala Leu Lys Pro Gly Val Pro Gln Pro
            20                  25                  30

Lys Ala Asn Gln Gln His Gln Asp Asn Arg Arg Gly Leu Val Leu Pro
        35                  40                  45

Gly Tyr Lys Tyr Leu Gly Pro Gly Asn Gly Leu Asp Lys Gly Glu Pro
    50                  55                  60

Val Asn Glu Ala Asp Ala Ala Ala Leu Glu His Asp Lys Ala Tyr Asp
65                  70                  75                  80

Gln Gln Leu Lys Ala Gly Asp Asn Pro Tyr Leu Lys Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Ile Leu Glu Pro
        115                 120                 125

Leu Gly Leu Val Glu Glu Ala Ala Lys Thr Ala Pro Gly Lys Lys Arg
    130                 135                 140

Pro Val Asp Gln Ser Pro Gln Glu Pro Asp Ser Ser Ser Gly Val Gly
145                 150                 155                 160

Lys Ser Gly Lys Gln Pro Ala Arg Lys Arg Leu Asn Phe Gly Gln Thr
                165                 170                 175

Gly Asp Ser Glu Ser Val Pro Asp Pro Gln Pro Leu Gly Glu Pro Pro
            180                 185                 190

Ala Ala Pro Thr Ser Leu Gly Ser Asn Thr Met Ala Ser Gly Gly Gly
        195                 200                 205

Ala Pro Met Ala Asp Asn Asn Glu Gly Ala Asp Gly Val Gly Asn Ser
    210                 215                 220

Ser Gly Asn Trp His Cys Asp Ser Gln Trp Leu Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Ser Gln Ser Gly Ala Ser Asn Asp Asn His Tyr
            260                 265                 270

Phe Gly Tyr Ser Thr Pro Trp Gly Tyr Phe Asp Phe Asn Arg Phe His
        275                 280                 285

Cys His Phe Ser Pro Arg Asp Trp Gln Arg Leu Ile Asn Asn Asn Trp
    290                 295                 300

Gly Phe Arg Pro Lys Lys Leu Ser Phe Lys Leu Phe Asn Ile Gln Val
305                 310                 315                 320

Lys Glu Val Thr Gln Asn Asp Gly Thr Thr Thr Ile Ala Asn Asn Leu
                325                 330                 335

Thr Ser Thr Val Gln Val Phe Thr Asp Ser Glu Tyr Gln Leu Pro Tyr
            340                 345                 350

Val Leu Gly Ser Ala His Gln Gly Cys Leu Pro Pro Phe Pro Ala Asp
        355                 360                 365

Val Phe Met Val Pro Gln Tyr Gly Tyr Leu Thr Leu Asn Asn Gly Ser
    370                 375                 380

Gln Ala Val Gly Arg Ser Ser Phe Tyr Cys Leu Glu Tyr Phe Pro Ser
385                 390                 395                 400

Gln Met Leu Arg Thr Gly Asn Asn Phe Gln Phe Ser Tyr Thr Phe Glu
                405                 410                 415

Asp Val Pro Phe His Ser Ser Tyr Ala His Ser Gln Ser Leu Asp Arg
            420                 425                 430

Leu Met Asn Pro Leu Ile Asp Gln Tyr Leu Tyr Tyr Leu Asn Arg Thr
        435                 440                 445

Gln Gly Thr Thr Ser Gly Thr Thr Asn Gln Ser Arg Leu Leu Phe Ser
    450                 455                 460

Gln Ala Gly Pro Gln Ser Met Ser Leu Gln Ala Arg Asn Trp Leu Pro
465                 470                 475                 480

Gly Pro Cys Tyr Arg Gln Gln Arg Leu Ser Lys Thr Ala Asn Asp Asn
                485                 490                 495

Asn Asn Ser Asn Phe Pro Trp Thr Ala Ala Ser Lys Tyr His Leu Asn
            500                 505                 510

Gly Arg Asp Ser Leu Val Asn Pro Gly Pro Ala Met Ala Ser His Lys
        515                 520                 525

Asp Asp Glu Glu Lys Phe Phe Pro Met His Gly Asn Leu Ile Phe Gly
    530                 535                 540

Lys Glu Gly Thr Thr Ala Ser Asn Ala Glu Leu Asp Asn Val Met Ile
545                 550                 555                 560

Thr Asp Glu Glu Glu Ile Arg Thr Thr Asn Pro Val Ala Thr Glu Gln
                565                 570                 575

Tyr Gly Thr Val Ala Asn Asn Leu Gln Ser Ser Asn Thr Ala Pro Thr
            580                 585                 590

Thr Arg Thr Val Asn Asp Gln Gly Ala Leu Pro Gly Met Val Trp Gln
        595                 600                 605

Asp Arg Asp Val Tyr Leu Gln Gly Pro Ile Trp Ala Lys Ile Pro His
    610                 615                 620

Thr Asp Gly His Phe His Pro Ser Pro Leu Met Gly Gly Phe Gly Leu
625                 630                 635                 640

Lys His Pro Pro Pro Gln Ile Met Ile Lys Asn Thr Pro Val Pro Ala
                645                 650                 655

Asn Pro Pro Thr Thr Phe Ser Pro Ala Lys Phe Ala Ser Phe Ile Thr

660                 665                 670

Gln Tyr Ser Thr Gly Gln Val Ser Val Glu Ile Glu Trp Glu Leu Gln
        675                 680                 685

Lys Glu Asn Ser Lys Arg Trp Asn Pro Glu Ile Gln Tyr Thr Ser Asn
    690                 695                 700

Tyr Asn Lys Ser Val Asn Val Asp Phe Thr Val Asp Thr Asn Gly Val
705                 710                 715                 720

Tyr Ser Glu Pro Arg Pro Ile Gly Thr Arg Tyr Leu Thr Arg Asn Leu
                725                 730                 735

<210>  4
<211>  734
<212>  PRT
<213>  AAV-4

<400>  4

Met Thr Asp Gly Tyr Leu Pro Asp Trp Leu Glu Asp Asn Leu Ser Glu
1               5                   10                  15

Gly Val Arg Glu Trp Trp Ala Leu Gln Pro Gly Ala Pro Lys Pro Lys
            20                  25                  30

Ala Asn Gln Gln His Gln Asp Asn Ala Arg Gly Leu Val Leu Pro Gly
        35                  40                  45

Tyr Lys Tyr Leu Gly Pro Gly Asn Gly Leu Asp Lys Gly Glu Pro Val
    50                  55                  60

Asn Ala Ala Asp Ala Ala Ala Leu Glu His Asp Lys Ala Tyr Asp Gln
65                  70                  75                  80

Gln Leu Lys Ala Gly Asp Asn Pro Tyr Leu Lys Tyr Asn His Ala Asp
                85                  90                  95

Ala Glu Phe Gln Gln Arg Leu Gln Gly Asp Thr Ser Phe Gly Gly Asn
            100                 105                 110

Leu Gly Arg Ala Val Phe Gln Ala Lys Lys Arg Val Leu Glu Pro Leu
        115                 120                 125

Gly Leu Val Glu Gln Ala Gly Glu Thr Ala Pro Gly Lys Lys Arg Pro
    130                 135                 140

Leu Ile Glu Ser Pro Gln Gln Pro Asp Ser Ser Thr Gly Ile Gly Lys

145                 150                 155                 160

Lys Gly Lys Gln Pro Ala Lys Lys Lys Leu Val Phe Glu Asp Glu Thr
                165                 170                 175

Gly Ala Gly Asp Gly Pro Pro Glu Gly Ser Thr Ser Gly Ala Met Ser
            180                 185                 190

Asp Asp Ser Glu Met Arg Ala Ala Ala Gly Gly Ala Ala Val Glu Gly
        195                 200                 205

Gly Gln Gly Ala Asp Gly Val Gly Asn Ala Ser Gly Asp Trp His Cys
    210                 215                 220

Asp Ser Thr Trp Ser Glu Gly His Val Thr Thr Thr Ser Thr Arg Thr
225                 230                 235                 240

Trp Val Leu Pro Thr Tyr Asn Asn His Leu Tyr Lys Arg Leu Gly Glu
                245                 250                 255

Ser Leu Gln Ser Asn Thr Tyr Asn Gly Phe Ser Thr Pro Trp Gly Tyr
            260                 265                 270

Phe Asp Phe Asn Arg Phe His Cys His Phe Ser Pro Arg Asp Trp Gln
        275                 280                 285

Arg Leu Ile Asn Asn Asn Trp Gly Met Arg Pro Lys Ala Met Arg Val
    290                 295                 300

Lys Ile Phe Asn Ile Gln Val Lys Glu Val Thr Thr Ser Asn Gly Glu
305                 310                 315                 320

Thr Thr Val Ala Asn Asn Leu Thr Ser Thr Val Gln Ile Phe Ala Asp
                325                 330                 335

Ser Ser Tyr Glu Leu Pro Tyr Val Met Asp Ala Gly Gln Glu Gly Ser
            340                 345                 350

Leu Pro Pro Phe Pro Asn Asp Val Phe Met Val Pro Gln Tyr Gly Tyr
        355                 360                 365

Cys Gly Leu Val Thr Gly Asn Thr Ser Gln Gln Gln Thr Asp Arg Asn
    370                 375                 380

Ala Phe Tyr Cys Leu Glu Tyr Phe Pro Ser Gln Met Leu Arg Thr Gly
385                 390                 395                 400

Asn Asn Phe Glu Ile Thr Tyr Ser Phe Glu Lys Val Pro Phe His Ser
                405                 410                 415

Met Tyr Ala His Ser Gln Ser Leu Asp Arg Leu Met Asn Pro Leu Ile
            420                 425                 430

Asp Gln Tyr Leu Trp Gly Leu Gln Ser Thr Thr Thr Gly Thr Thr Leu
        435                 440                 445

Asn Ala Gly Thr Ala Thr Thr Asn Phe Thr Lys Leu Arg Pro Thr Asn
    450                 455                 460

Phe Ser Asn Phe Lys Lys Asn Trp Leu Pro Gly Pro Ser Ile Lys Gln
465                 470                 475                 480

Gln Gly Phe Ser Lys Thr Ala Asn Gln Asn Tyr Lys Ile Pro Ala Thr
                485                 490                 495

Gly Ser Asp Ser Leu Ile Lys Tyr Glu Thr His Ser Thr Leu Asp Gly
            500                 505                 510

Arg Trp Ser Ala Leu Thr Pro Gly Pro Pro Met Ala Thr Ala Gly Pro
        515                 520                 525

Ala Asp Ser Lys Phe Ser Asn Ser Gln Leu Ile Phe Ala Gly Pro Lys
    530                 535                 540

Gln Asn Gly Asn Thr Ala Thr Val Pro Gly Thr Leu Ile Phe Thr Ser
545                 550                 555                 560

Glu Glu Glu Leu Ala Ala Thr Asn Ala Thr Asp Thr Asp Met Trp Gly
                565                 570                 575

Asn Leu Pro Gly Gly Asp Gln Ser Asn Ser Asn Leu Pro Thr Val Asp
            580                 585                 590

Arg Leu Thr Ala Leu Gly Ala Val Pro Gly Met Val Trp Gln Asn Arg
        595                 600                 605

Asp Ile Tyr Tyr Gln Gly Pro Ile Trp Ala Lys Ile Pro His Thr Asp
    610                 615                 620

Gly His Phe His Pro Ser Pro Leu Ile Gly Gly Phe Gly Leu Lys His
625                 630                 635                 640

Pro Pro Pro Gln Ile Phe Ile Lys Asn Thr Pro Val Pro Ala Asn Pro
                645                 650                 655

Ala Thr Thr Phe Ser Ser Thr Pro Val Asn Ser Phe Ile Thr Gln Tyr
                660                 665                 670

Ser Thr Gly Gln Val Ser Val Gln Ile Asp Trp Glu Ile Gln Lys Glu
            675                 680                 685

Arg Ser Lys Arg Trp Asn Pro Glu Val Gln Phe Thr Ser Asn Tyr Gly
    690                 695                 700

Gln Gln Asn Ser Leu Leu Trp Ala Pro Asp Ala Ala Gly Lys Tyr Thr
705                 710                 715                 720

Glu Pro Arg Ala Ile Gly Thr Arg Tyr Leu Thr His His Leu
                725                 730

<210>  5
<211>  724
<212>  PRT
<213>  AAV-5

<400>  5

Met Ser Phe Val Asp His Pro Pro Asp Trp Leu Glu Glu Val Gly Glu
1               5                   10                  15

Gly Leu Arg Glu Phe Leu Gly Leu Glu Ala Gly Pro Pro Lys Pro Lys
            20                  25                  30

Pro Asn Gln Gln His Gln Asp Gln Ala Arg Gly Leu Val Leu Pro Gly
        35                  40                  45

Tyr Asn Tyr Leu Gly Pro Gly Asn Gly Leu Asp Arg Gly Glu Pro Val
    50                  55                  60

Asn Arg Ala Asp Glu Val Ala Arg Glu His Asp Ile Ser Tyr Asn Glu
65                  70                  75                  80

Gln Leu Glu Ala Gly Asp Asn Pro Tyr Leu Lys Tyr Asn His Ala Asp
                85                  90                  95

Ala Glu Phe Gln Glu Lys Leu Ala Asp Asp Thr Ser Phe Gly Gly Asn
            100                 105                 110

Leu Gly Lys Ala Val Phe Gln Ala Lys Lys Arg Val Leu Glu Pro Phe
        115                 120                 125

Gly Leu Val Glu Glu Gly Ala Lys Thr Ala Pro Thr Gly Lys Arg Ile
    130                 135                 140

Asp Asp His Phe Pro Lys Arg Lys Lys Ala Arg Thr Glu Glu Asp Ser
145                 150                 155                 160

Lys Pro Ser Thr Ser Ser Asp Ala Glu Ala Gly Pro Ser Gly Ser Gln
                165                 170                 175

Gln Leu Gln Ile Pro Ala Gln Pro Ala Ser Ser Leu Gly Ala Asp Thr
            180                 185                 190

Met Ser Ala Gly Gly Gly Gly Pro Leu Gly Asp Asn Asn Gln Gly Ala
        195                 200                 205

Asp Gly Val Gly Asn Ala Ser Gly Asp Trp His Cys Asp Ser Thr Trp
    210                 215                 220

Met Gly Asp Arg Val Val Thr Lys Ser Thr Arg Thr Trp Val Leu Pro
225                 230                 235                 240

Ser Tyr Asn Asn His Gln Tyr Arg Glu Ile Lys Ser Gly Ser Val Asp
                245                 250                 255

Gly Ser Asn Ala Asn Ala Tyr Phe Gly Tyr Ser Thr Pro Trp Gly Tyr
            260                 265                 270

Phe Asp Phe Asn Arg Phe His Ser His Trp Ser Pro Arg Asp Trp Gln
        275                 280                 285

Arg Leu Ile Asn Asn Tyr Trp Gly Phe Arg Pro Arg Ser Leu Arg Val
    290                 295                 300

Lys Ile Phe Asn Ile Gln Val Lys Glu Val Thr Val Gln Asp Ser Thr
305                 310                 315                 320

Thr Thr Ile Ala Asn Asn Leu Thr Ser Thr Val Gln Val Phe Thr Asp
                325                 330                 335

Asp Asp Tyr Gln Leu Pro Tyr Val Val Gly Asn Gly Thr Glu Gly Cys
            340                 345                 350

Leu Pro Ala Phe Pro Pro Gln Val Phe Thr Leu Pro Gln Tyr Gly Tyr
        355                 360                 365

Ala Thr Leu Asn Arg Asp Asn Thr Glu Asn Pro Thr Glu Arg Ser Ser
    370                 375                 380

Phe Phe Cys Leu Glu Tyr Phe Pro Ser Lys Met Leu Arg Thr Gly Asn
385                 390                 395                 400

Asn Phe Glu Phe Thr Tyr Asn Phe Glu Glu Val Pro Phe His Ser Ser
                405                 410                 415

Phe Ala Pro Ser Gln Asn Leu Phe Lys Leu Ala Asn Pro Leu Val Asp
            420                 425                 430

Gln Tyr Leu Tyr Arg Phe Val Ser Thr Asn Asn Thr Gly Gly Val Gln
        435                 440                 445

Phe Asn Lys Asn Leu Ala Gly Arg Tyr Ala Asn Thr Tyr Lys Asn Trp
    450                 455                 460

Phe Pro Gly Pro Met Gly Arg Thr Gln Gly Trp Asn Leu Gly Ser Gly
465                 470                 475                 480

Val Asn Arg Ala Ser Val Ser Ala Phe Ala Thr Thr Asn Arg Met Glu
                485                 490                 495

Leu Glu Gly Ala Ser Tyr Gln Val Pro Pro Gln Pro Asn Gly Met Thr
            500                 505                 510

Asn Asn Leu Gln Gly Ser Asn Thr Tyr Ala Leu Glu Asn Thr Met Ile
        515                 520                 525

Phe Asn Ser Gln Pro Ala Asn Pro Gly Thr Thr Ala Thr Tyr Leu Glu
    530                 535                 540

Gly Asn Met Leu Ile Thr Ser Glu Ser Glu Thr Gln Pro Val Asn Arg
545                 550                 555                 560

Val Ala Tyr Asn Val Gly Gly Gln Met Ala Thr Asn Asn Gln Ser Ser
                565                 570                 575

Thr Thr Ala Pro Ala Thr Gly Thr Tyr Asn Leu Gln Glu Ile Val Pro
            580                 585                 590

Gly Ser Val Trp Met Glu Arg Asp Val Tyr Leu Gln Gly Pro Ile Trp
        595                 600                 605

Ala Lys Ile Pro Glu Thr Gly Ala His Phe His Pro Ser Pro Ala Met
    610                 615                 620

Gly Gly Phe Gly Leu Lys His Pro Pro Pro Met Met Leu Ile Lys Asn
625                 630                 635                 640

Thr Pro Val Pro Gly Asn Ile Thr Ser Phe Ser Asp Val Pro Val Ser

645                 650                 655

Ser Phe Ile Thr Gln Tyr Ser Thr Gly Gln Val Thr Val Glu Met Glu
            660                 665                 670

Trp Glu Leu Lys Lys Glu Asn Ser Lys Arg Trp Asn Pro Glu Ile Gln
        675                 680                 685

Tyr Thr Asn Asn Tyr Asn Asp Pro Gln Phe Val Asp Phe Ala Pro Asp
    690                 695                 700

Ser Thr Gly Glu Tyr Arg Thr Thr Arg Pro Ile Gly Thr Arg Tyr Leu
705                 710                 715                 720

Thr Arg Pro Leu

<210>  6
<211>  736
<212>  PRT
<213>  AAV-6

<400>  6

Met Ala Ala Asp Gly Tyr Leu Pro Asp Trp Leu Glu Asp Asn Leu Ser
1               5                   10                  15

Glu Gly Ile Arg Glu Trp Trp Asp Leu Lys Pro Gly Ala Pro Lys Pro
            20                  25                  30

Lys Ala Asn Gln Gln Lys Gln Asp Asp Gly Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Ala Ala Asp Ala Ala Ala Leu Glu His Asp Lys Ala Tyr Asp
65                  70                  75                  80

Gln Gln Leu Lys Ala Gly Asp Asn Pro Tyr Leu Arg Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Val Leu Glu Pro
        115                 120                 125

Phe Gly Leu Val Glu Glu Gly Ala Lys Thr Ala Pro Gly Lys Lys Arg

130                 135                 140

Pro Val Glu Gln Ser Pro Gln Glu Pro Asp Ser Ser Ser Gly Ile Gly
145                 150                 155                 160

Lys Thr Gly Gln Gln Pro Ala Lys Lys Arg Leu Asn Phe Gly Gln Thr
                165                 170                 175

Gly Asp Ser Glu Ser Val Pro Asp Pro Gln Pro Leu Gly Glu Pro Pro
            180                 185                 190

Ala Thr Pro Ala Ala Val Gly Pro Thr Thr Met Ala Ser Gly Gly Gly
        195                 200                 205

Ala Pro Met Ala Asp Asn Asn Glu Gly Ala Asp Gly Val Gly Asn Ala
    210                 215                 220

Ser Gly Asn Trp His Cys Asp Ser Thr Trp Leu Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Ser Ala Ser Thr Gly Ala Ser Asn Asp Asn His
            260                 265                 270

Tyr Phe Gly Tyr Ser Thr Pro Trp Gly Tyr Phe Asp Phe Asn Arg Phe
        275                 280                 285

His Cys His Phe Ser Pro Arg Asp Trp Gln Arg Leu Ile Asn Asn Asn
    290                 295                 300

Trp Gly Phe Arg Pro Lys Arg Leu Asn Phe Lys Leu Phe Asn Ile Gln
305                 310                 315                 320

Val Lys Glu Val Thr Thr Asn Asp Gly Val Thr Thr Ile Ala Asn Asn
                325                 330                 335

Leu Thr Ser Thr Val Gln Val Phe Ser Asp Ser Glu Tyr Gln Leu Pro
            340                 345                 350

Tyr Val Leu Gly Ser Ala His Gln Gly Cys Leu Pro Pro Phe Pro Ala
        355                 360                 365

Asp Val Phe Met Ile Pro Gln Tyr Gly Tyr Leu Thr Leu Asn Asn Gly
    370                 375                 380

Ser Gln Ala Val Gly Arg Ser Ser Phe Tyr Cys Leu Glu Tyr Phe Pro
385                 390                 395                 400

Ser Gln Met Leu Arg Thr Gly Asn Asn Phe Thr Phe Ser Tyr Thr Phe
                405                 410                 415

Glu Asp Val Pro Phe His Ser Ser Tyr Ala His Ser Gln Ser Leu Asp
            420                 425                 430

Arg Leu Met Asn Pro Leu Ile Asp Gln Tyr Leu Tyr Tyr Leu Asn Arg
        435                 440                 445

Thr Gln Asn Gln Ser Gly Ser Ala Gln Asn Lys Asp Leu Leu Phe Ser
    450                 455                 460

Arg Gly Ser Pro Ala Gly Met Ser Val Gln Pro Lys Asn Trp Leu Pro
465                 470                 475                 480

Gly Pro Cys Tyr Arg Gln Gln Arg Val Ser Lys Thr Lys Thr Asp Asn
                485                 490                 495

Asn Asn Ser Asn Phe Thr Trp Thr Gly Ala Ser Lys Tyr Asn Leu Asn
            500                 505                 510

Gly Arg Glu Ser Ile Ile Asn Pro Gly Thr Ala Met Ala Ser His Lys
        515                 520                 525

Asp Asp Lys Asp Lys Phe Phe Pro Met Ser Gly Val Met Ile Phe Gly
    530                 535                 540

Lys Glu Ser Ala Gly Ala Ser Asn Thr Ala Leu Asp Asn Val Met Ile
545                 550                 555                 560

Thr Asp Glu Glu Glu Ile Lys Ala Thr Asn Pro Val Ala Thr Glu Arg
                565                 570                 575

Phe Gly Thr Val Ala Val Asn Leu Gln Ser Ser Ser Thr Asp Pro Ala
            580                 585                 590

Thr Gly Asp Val His Val Met Gly Ala Leu Pro Gly Met Val Trp Gln
        595                 600                 605

Asp Arg Asp Val Tyr Leu Gln Gly Pro Ile Trp Ala Lys Ile Pro His
    610                 615                 620

Thr Asp Gly His Phe His Pro Ser Pro Leu Met Gly Gly Phe Gly Leu
625                 630                 635                 640

Lys His Pro Pro Pro Gln Ile Leu Ile Lys Asn Thr Pro Val Pro Ala
                645                 650                 655

Asn Pro Pro Ala Glu Phe Ser Ala Thr Lys Phe Ala Ser Phe Ile Thr
            660                 665                 670

Gln Tyr Ser Thr Gly Gln Val Ser Val Glu Ile Glu Trp Glu Leu Gln
        675                 680                 685

Lys Glu Asn Ser Lys Arg Trp Asn Pro Glu Val Gln Tyr Thr Ser Asn
    690                 695                 700

Tyr Ala Lys Ser Ala Asn Val Asp Phe Thr Val Asp Asn Asn Gly Leu
705                 710                 715                 720

Tyr Thr Glu Pro Arg Pro Ile Gly Thr Arg Tyr Leu Thr Arg Pro Leu
                725                 730                 735

<210>  7
<211>  737
<212>  PRT
<213>  AAV-7

<400>  7

Met Ala Ala Asp Gly Tyr Leu Pro Asp Trp Leu Glu Asp Asn Leu Ser
1               5                   10                  15

Glu Gly Ile Arg Glu Trp Trp Asp Leu Lys Pro Gly Ala Pro Lys Pro
            20                  25                  30

Lys Ala Asn Gln Gln Lys Gln Asp Asn Gly Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Ala Ala Asp Ala Ala Ala Leu Glu His Asp Lys Ala Tyr Asp
65                  70                  75                  80

Gln Gln Leu Lys Ala Gly Asp Asn Pro Tyr Leu Arg Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Gly Ala Lys Thr Ala Pro Ala Lys Lys Arg
    130                 135                 140

Pro Val Glu Pro Ser Pro Gln Arg Ser Pro Asp Ser Ser Thr Gly Ile
145                 150                 155                 160

Gly Lys Lys Gly Gln Gln Pro Ala Arg Lys Arg Leu Asn Phe Gly Gln
                165                 170                 175

Thr Gly Asp Ser Glu Ser Val Pro Asp Pro Gln Pro Leu Gly Glu Pro
            180                 185                 190

Pro Ala Ala Pro Ser Ser Val Gly Ser Gly Thr Val Ala Ala Gly Gly
        195                 200                 205

Gly Ala Pro Met Ala Asp Asn Asn Glu Gly Ala Asp Gly Val Gly Asn
    210                 215                 220

Ala Ser Gly Asn Trp His Cys Asp Ser Thr Trp Leu Gly Asp Arg Val
225                 230                 235                 240

Ile Thr Thr Ser Thr Arg Thr Trp Ala Leu Pro Thr Tyr Asn Asn His
                245                 250                 255

Leu Tyr Lys Gln Ile Ser Ser Glu Thr Ala Gly Ser Thr Asn Asp Asn
            260                 265                 270

Thr Tyr Phe Gly Tyr Ser Thr Pro Trp Gly Tyr Phe Asp Phe Asn Arg
        275                 280                 285

Phe His Cys His Phe Ser Pro Arg Asp Trp Gln Arg Leu Ile Asn Asn
    290                 295                 300

Asn Trp Gly Phe Arg Pro Lys Lys Leu Arg Phe Lys Leu Phe Asn Ile
305                 310                 315                 320

Gln Val Lys Glu Val Thr Thr Asn Asp Gly Val Thr Thr Ile Ala Asn
                325                 330                 335

Asn Leu Thr Ser Thr Ile Gln Val Phe Ser Asp Ser Glu Tyr Gln Leu
            340                 345                 350

Pro Tyr Val Leu Gly Ser Ala His Gln Gly Cys Leu Pro Pro Phe Pro
        355                 360                 365

Ala Asp Val Phe Met Ile Pro Gln Tyr Gly Tyr Leu Thr Leu Asn Asn
    370                 375                 380

Gly Ser Gln Ser Val Gly Arg Ser Ser Phe Tyr Cys Leu Glu Tyr Phe
385                 390                 395                 400

Pro Ser Gln Met Leu Arg Thr Gly Asn Asn Phe Glu Phe Ser Tyr Ser
                405                 410                 415

Phe Glu Asp Val Pro Phe His Ser Ser Tyr Ala His Ser Gln Ser Leu
            420                 425                 430

Asp Arg Leu Met Asn Pro Leu Ile Asp Gln Tyr Leu Tyr Tyr Leu Ala
        435                 440                 445

Arg Thr Gln Ser Asn Pro Gly Gly Thr Ala Gly Asn Arg Glu Leu Gln
    450                 455                 460

Phe Tyr Gln Gly Gly Pro Ser Thr Met Ala Glu Gln Ala Lys Asn Trp
465                 470                 475                 480

Leu Pro Gly Pro Cys Phe Arg Gln Gln Arg Val Ser Lys Thr Leu Asp
                485                 490                 495

Gln Asn Asn Asn Ser Asn Phe Ala Trp Thr Gly Ala Thr Lys Tyr His
            500                 505                 510

Leu Asn Gly Arg Asn Ser Leu Val Asn Pro Gly Val Ala Met Ala Thr
        515                 520                 525

His Lys Asp Asp Glu Asp Arg Phe Phe Pro Ser Ser Gly Val Leu Ile
    530                 535                 540

Phe Gly Lys Thr Gly Ala Thr Asn Lys Thr Thr Leu Glu Asn Val Leu
545                 550                 555                 560

Met Thr Asn Glu Glu Glu Ile Arg Pro Thr Asn Pro Val Ala Thr Glu
                565                 570                 575

Glu Tyr Gly Ile Val Ser Ser Asn Leu Gln Ala Ala Asn Thr Ala Ala
            580                 585                 590

Gln Thr Gln Val Val Asn Asn Gln Gly Ala Leu Pro Gly Met Val Trp
        595                 600                 605

Gln Asn Arg Asp Val Tyr Leu Gln Gly Pro Ile Trp Ala Lys Ile Pro
    610                 615                 620

His Thr Asp Gly Asn Phe His Pro Ser Pro Leu Met Gly Gly Phe Gly

625                 630                 635                 640

Leu Lys His Pro Pro Pro Gln Ile Leu Ile Lys Asn Thr Pro Val Pro
                645                 650                 655

Ala Asn Pro Pro Glu Val Phe Thr Pro Ala Lys Phe Ala Ser Phe Ile
            660                 665                 670

Thr Gln Tyr Ser Thr Gly Gln Val Ser Val Glu Ile Glu Trp Glu Leu
        675                 680                 685

Gln Lys Glu Asn Ser Lys Arg Trp Asn Pro Glu Ile Gln Tyr Thr Ser
    690                 695                 700

Asn Phe Glu Lys Gln Thr Gly Val Asp Phe Ala Val Asp Ser Gln Gly
705                 710                 715                 720

Val Tyr Ser Glu Pro Arg Pro Ile Gly Thr Arg Tyr Leu Thr Arg Asn
                725                 730                 735

Leu

<210>  8
<211>  738
<212>  PRT
<213>  AAV-8

<400>  8

Met Ala Ala Asp Gly Tyr Leu Pro Asp Trp Leu Glu Asp Asn Leu Ser
1               5                   10                  15

Glu Gly Ile Arg Glu Trp Trp Ala Leu Lys Pro Gly Ala Pro Lys Pro
            20                  25                  30

Lys Ala Asn Gln Gln Lys Gln Asp Asp Gly Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Ala Ala Asp Ala Ala Ala Leu Glu His Asp Lys Ala Tyr Asp
65                  70                  75                  80

Gln Gln Leu Gln Ala Gly Asp Asn Pro Tyr Leu Arg Tyr Asn His Ala
                85                  90                  95

Asp Ala Glu Phe Gln Glu Arg Leu Gln Glu Asp Thr Ser Phe Gly Gly

            100                 105                 110

Asn Leu Gly Arg Ala Val Phe Gln Ala Lys Lys Arg Val Leu Glu Pro
        115                 120                 125

Leu Gly Leu Val Glu Glu Gly Ala Lys Thr Ala Pro Gly Lys Lys Arg
    130                 135                 140

Pro Val Glu Pro Ser Pro Gln Arg Ser Pro Asp Ser Ser Thr Gly Ile
145                 150                 155                 160

Gly Lys Lys Gly Gln Gln Pro Ala Arg Lys Arg Leu Asn Phe Gly Gln
                165                 170                 175

Thr Gly Asp Ser Glu Ser Val Pro Asp Pro Gln Pro Leu Gly Glu Pro
            180                 185                 190

Pro Ala Ala Pro Ser Gly Val Gly Pro Asn Thr Met Ala Ala Gly Gly
        195                 200                 205

Gly Ala Pro Met Ala Asp Asn Asn Glu Gly Ala Asp Gly Val Gly Ser
    210                 215                 220

Ser Ser Gly Asn Trp His Cys Asp Ser Thr Trp Leu Gly Asp Arg Val
225                 230                 235                 240

Ile Thr Thr Ser Thr Arg Thr Trp Ala Leu Pro Thr Tyr Asn Asn His
                245                 250                 255

Leu Tyr Lys Gln Ile Ser Asn Gly Thr Ser Gly Gly Ala Thr Asn Asp
            260                 265                 270

Asn Thr Tyr Phe Gly Tyr Ser Thr Pro Trp Gly Tyr Phe Asp Phe Asn
        275                 280                 285

Arg Phe His Cys His Phe Ser Pro Arg Asp Trp Gln Arg Leu Ile Asn
    290                 295                 300

Asn Asn Trp Gly Phe Arg Pro Lys Arg Leu Ser Phe Lys Leu Phe Asn
305                 310                 315                 320

Ile Gln Val Lys Glu Val Thr Gln Asn Glu Gly Thr Lys Thr Ile Ala
                325                 330                 335

Asn Asn Leu Thr Ser Thr Ile Gln Val Phe Thr Asp Ser Glu Tyr Gln
            340                 345                 350

Leu Pro Tyr Val Leu Gly Ser Ala His Gln Gly Cys Leu Pro Pro Phe
        355                 360                 365

Pro Ala Asp Val Phe Met Ile Pro Gln Tyr Gly Tyr Leu Thr Leu Asn
    370                 375                 380

Asn Gly Ser Gln Ala Val Gly Arg Ser Ser Phe Tyr Cys Leu Glu Tyr
385                 390                 395                 400

Phe Pro Ser Gln Met Leu Arg Thr Gly Asn Asn Phe Gln Phe Thr Tyr
                405                 410                 415

Thr Phe Glu Asp Val Pro Phe His Ser Ser Tyr Ala His Ser Gln Ser
            420                 425                 430

Leu Asp Arg Leu Met Asn Pro Leu Ile Asp Gln Tyr Leu Tyr Tyr Leu
        435                 440                 445

Ser Arg Thr Gln Thr Thr Gly Gly Thr Ala Asn Thr Gln Thr Leu Gly
    450                 455                 460

Phe Ser Gln Gly Gly Pro Asn Thr Met Ala Asn Gln Ala Lys Asn Trp
465                 470                 475                 480

Leu Pro Gly Pro Cys Tyr Arg Gln Gln Arg Val Ser Thr Thr Thr Gly
                485                 490                 495

Gln Asn Asn Asn Ser Asn Phe Ala Trp Thr Ala Gly Thr Lys Tyr His
            500                 505                 510

Leu Asn Gly Arg Asn Ser Leu Ala Asn Pro Gly Ile Ala Met Ala Thr
        515                 520                 525

His Lys Asp Asp Glu Glu Arg Phe Phe Pro Ser Asn Gly Ile Leu Ile
    530                 535                 540

Phe Gly Lys Gln Asn Ala Ala Arg Asp Asn Ala Asp Tyr Ser Asp Val
545                 550                 555                 560

Met Leu Thr Ser Glu Glu Glu Ile Lys Thr Thr Asn Pro Val Ala Thr
                565                 570                 575

Glu Glu Tyr Gly Ile Val Ala Asp Asn Leu Gln Gln Gln Asn Thr Ala
            580                 585                 590

Pro Gln Ile Gly Thr Val Asn Ser Gln Gly Ala Leu Pro Gly Met Val
        595                 600                 605

Trp Gln Asn Arg Asp Val Tyr Leu Gln Gly Pro Ile Trp Ala Lys Ile
    610                 615                 620

Pro His Thr Asp Gly Asn Phe His Pro Ser Pro Leu Met Gly Gly Phe
625                 630                 635                 640

Gly Leu Lys His Pro Pro Pro Gln Ile Leu Ile Lys Asn Thr Pro Val
                645                 650                 655

Pro Ala Asp Pro Pro Thr Thr Phe Asn Gln Ser Lys Leu Asn Ser Phe
            660                 665                 670

Ile Thr Gln Tyr Ser Thr Gly Gln Val Ser Val Glu Ile Glu Trp Glu
        675                 680                 685

Leu Gln Lys Glu Asn Ser Lys Arg Trp Asn Pro Glu Ile Gln Tyr Thr
    690                 695                 700

Ser Asn Tyr Tyr Lys Ser Thr Ser Val Asp Phe Ala Val Asn Thr Glu
705                 710                 715                 720

Gly Val Tyr Ser Glu Pro Arg Pro Ile Gly Thr Arg Tyr Leu Thr Arg
                725                 730                 735

Asn Leu

<210>  9
<211>  736
<212>  PRT
<213>  AAV-9

<400>  9

Met Ala Ala Asp Gly Tyr Leu Pro Asp Trp Leu Glu Asp Asn Leu Ser
1               5                   10                  15

Glu Gly Ile Arg Glu Trp Trp Ala Leu Lys Pro Gly Ala Pro Gln Pro
            20                  25                  30

Lys Ala Asn Gln Gln His Gln Asp Asn Ala Arg Gly Leu Val Leu Pro
        35                  40                  45

Gly Tyr Lys Tyr Leu Gly Pro Gly Asn Gly Leu Asp Lys Gly Glu Pro
    50                  55                  60

Val Asn Ala Ala Asp Ala Ala Ala Leu Glu His Asp Lys Ala Tyr Asp
65                  70                  75                  80

Gln Gln Leu Lys Ala Gly Asp Asn Pro Tyr Leu Lys Tyr Asn His Ala
                85                  90                  95

Asp Ala Glu Phe Gln Glu Arg Leu Lys Glu Asp Thr Ser Phe Gly Gly
            100                 105                 110

Asn Leu Gly Arg Ala Val Phe Gln Ala Lys Lys Arg Leu Leu Glu Pro
        115                 120                 125

Leu Gly Leu Val Glu Glu Ala Ala Lys Thr Ala Pro Gly Lys Lys Arg
    130                 135                 140

Pro Val Glu Gln Ser Pro Gln Glu Pro Asp Ser Ser Ala Gly Ile Gly
145                 150                 155                 160

Lys Ser Gly Ala Gln Pro Ala Lys Lys Arg Leu Asn Phe Gly Gln Thr
                165                 170                 175

Gly Asp Thr Glu Ser Val Pro Asp Pro Gln Pro Ile Gly Glu Pro Pro
            180                 185                 190

Ala Ala Pro Ser Gly Val Gly Ser Leu Thr Met Ala Ser Gly Gly Gly
        195                 200                 205

Ala Pro Val Ala Asp Asn Asn Glu Gly Ala Asp Gly Val Gly Ser Ser
    210                 215                 220

Ser Gly Asn Trp His Cys Asp Ser Gln Trp Leu Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Asn Ser Thr Ser Gly Gly Ser Ser Asn Asp Asn
            260                 265                 270

Ala Tyr Phe Gly Tyr Ser Thr Pro Trp Gly Tyr Phe Asp Phe Asn Arg
        275                 280                 285

Phe His Cys His Phe Ser Pro Arg Asp Trp Gln Arg Leu Ile Asn Asn
    290                 295                 300

Asn Trp Gly Phe Arg Pro Lys Arg Leu Asn Phe Lys Leu Phe Asn Ile
305                 310                 315                 320

Gln Val Lys Glu Val Thr Asp Asn Asn Gly Val Lys Thr Ile Ala Asn
                325                 330                 335

Asn Leu Thr Ser Thr Val Gln Val Phe Thr Asp Ser Asp Tyr Gln Leu
            340                 345                 350

Pro Tyr Val Leu Gly Ser Ala His Glu Gly Cys Leu Pro Pro Phe Pro
        355                 360                 365

Ala Asp Val Phe Met Ile Pro Gln Tyr Gly Tyr Leu Thr Leu Asn Asp
    370                 375                 380

Gly Ser Gln Ala Val Gly Arg Ser Ser Phe Tyr Cys Leu Glu Tyr Phe
385                 390                 395                 400

Pro Ser Gln Met Leu Arg Thr Gly Asn Asn Phe Gln Phe Ser Tyr Glu
                405                 410                 415

Phe Glu Asn Val Pro Phe His Ser Ser Tyr Ala His Ser Gln Ser Leu
            420                 425                 430

Asp Arg Leu Met Asn Pro Leu Ile Asp Gln Tyr Leu Tyr Tyr Leu Ser
        435                 440                 445

Lys Thr Ile Asn Gly Ser Gly Gln Asn Gln Gln Thr Leu Lys Phe Ser
    450                 455                 460

Val Ala Gly Pro Ser Asn Met Ala Val Gln Gly Arg Asn Tyr Ile Pro
465                 470                 475                 480

Gly Pro Ser Tyr Arg Gln Gln Arg Val Ser Thr Thr Val Thr Gln Asn
                485                 490                 495

Asn Asn Ser Glu Phe Ala Trp Pro Gly Ala Ser Ser Trp Ala Leu Asn
            500                 505                 510

Gly Arg Asn Ser Leu Met Asn Pro Gly Pro Ala Met Ala Ser His Lys
        515                 520                 525

Glu Gly Glu Asp Arg Phe Phe Pro Leu Ser Gly Ser Leu Ile Phe Gly
    530                 535                 540

Lys Gln Gly Thr Gly Arg Asp Asn Val Asp Ala Asp Lys Val Met Ile
545                 550                 555                 560

Thr Asn Glu Glu Glu Ile Lys Thr Thr Asn Pro Val Ala Thr Glu Ser
                565                 570                 575

Tyr Gly Gln Val Ala Thr Asn His Gln Ser Ala Gln Ala Gln Ala Gln

580                 585                 590

Thr Gly Trp Val Gln Asn Gln Gly Ile Leu Pro Gly Met Val Trp Gln
        595                 600                 605

Asp Arg Asp Val Tyr Leu Gln Gly Pro Ile Trp Ala Lys Ile Pro His
    610                 615                 620

Thr Asp Gly Asn Phe His Pro Ser Pro Leu Met Gly Gly Phe Gly Met
625                 630                 635                 640

Lys His Pro Pro Pro Gln Ile Leu Ile Lys Asn Thr Pro Val Pro Ala
                645                 650                 655

Asp Pro Pro Thr Ala Phe Asn Lys Asp Lys Leu Asn Ser Phe Ile Thr
            660                 665                 670

Gln Tyr Ser Thr Gly Gln Val Ser Val Glu Ile Glu Trp Glu Leu Gln
        675                 680                 685

Lys Glu Asn Ser Lys Arg Trp Asn Pro Glu Ile Gln Tyr Thr Ser Asn
    690                 695                 700

Tyr Tyr Lys Ser Asn Asn Val Glu Phe Ala Val Asn Thr Glu Gly Val
705                 710                 715                 720

Tyr Ser Glu Pro Arg Pro Ile Gly Thr Arg Tyr Leu Thr Arg Asn Leu
                725                 730                 735

<210>  10
<211>  738
<212>  PRT
<213>  AAV-10

<400>  10

Met Ala Ala Asp Gly Tyr Leu Pro Asp Trp Leu Glu Asp Asn Leu Ser
1               5                   10                  15

Glu Gly Ile Arg Glu Trp Trp Asp Leu Lys Pro Gly Ala Pro Lys Pro
            20                  25                  30

Lys Ala Asn Gln Gln Lys Gln Asp Asp Gly Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Ala Ala Asp Ala Ala Ala Leu Glu His Asp Lys Ala Tyr Asp

65                  70                  75                  80

Gln Gln Leu Lys Ala Gly Asp Asn Pro Tyr Leu Arg Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Ala Ala Lys Thr Ala Pro Gly Lys Lys Arg
    130                 135                 140

Pro Val Glu Pro Ser Pro Gln Arg Ser Pro Asp Ser Ser Thr Gly Ile
145                 150                 155                 160

Gly Lys Lys Gly Gln Gln Pro Ala Lys Lys Arg Leu Asn Phe Gly Gln
                165                 170                 175

Thr Gly Glu Ser Glu Ser Val Pro Asp Pro Gln Pro Ile Gly Glu Pro
            180                 185                 190

Pro Ala Gly Pro Ser Gly Leu Gly Ser Gly Thr Met Ala Ala Gly Gly
        195                 200                 205

Gly Ala Pro Met Ala Asp Asn Asn Glu Gly Ala Asp Gly Val Gly Ser
    210                 215                 220

Ser Ser Gly Asn Trp His Cys Asp Ser Thr Trp Leu Gly Asp Arg Val
225                 230                 235                 240

Ile Thr Thr Ser Thr Arg Thr Trp Ala Leu Pro Thr Tyr Asn Asn His
                245                 250                 255

Leu Tyr Lys Gln Ile Ser Asn Gly Thr Ser Gly Gly Ser Thr Asn Asp
            260                 265                 270

Asn Thr Tyr Phe Gly Tyr Ser Thr Pro Trp Gly Tyr Phe Asp Phe Asn
        275                 280                 285

Arg Phe His Cys His Phe Ser Pro Arg Asp Trp Gln Arg Leu Ile Asn
    290                 295                 300

Asn Asn Trp Gly Phe Arg Pro Lys Arg Leu Ser Phe Lys Leu Phe Asn
305                 310                 315                 320

Ile Gln Val Lys Glu Val Thr Gln Asn Glu Gly Thr Lys Thr Ile Ala
                325                 330                 335

Asn Asn Leu Thr Ser Thr Ile Gln Val Phe Thr Asp Ser Glu Tyr Gln
            340                 345                 350

Leu Pro Tyr Val Leu Gly Ser Ala His Gln Gly Cys Leu Pro Pro Phe
        355                 360                 365

Pro Ala Asp Val Phe Met Ile Pro Gln Tyr Gly Tyr Leu Thr Leu Asn
    370                 375                 380

Asn Gly Ser Gln Ala Val Gly Arg Ser Ser Phe Tyr Cys Leu Glu Tyr
385                 390                 395                 400

Phe Pro Ser Gln Met Leu Arg Thr Gly Asn Asn Phe Glu Phe Ser Tyr
                405                 410                 415

Thr Phe Glu Asp Val Pro Phe His Ser Ser Tyr Ala His Ser Gln Ser
            420                 425                 430

Leu Asp Arg Leu Met Asn Pro Leu Ile Asp Gln Tyr Leu Tyr Tyr Leu
        435                 440                 445

Ser Arg Thr Gln Ser Thr Gly Gly Thr Gln Gly Thr Gln Gln Leu Leu
    450                 455                 460

Phe Ser Gln Ala Gly Pro Ala Asn Met Ser Ala Gln Ala Lys Asn Trp
465                 470                 475                 480

Leu Pro Gly Pro Cys Tyr Arg Gln Gln Arg Val Ser Thr Thr Leu Ser
                485                 490                 495

Gln Asn Asn Asn Ser Asn Phe Ala Trp Thr Gly Ala Thr Lys Tyr His
            500                 505                 510

Leu Asn Gly Arg Asp Ser Leu Val Asn Pro Gly Val Ala Met Ala Thr
        515                 520                 525

His Lys Asp Asp Glu Glu Arg Phe Phe Pro Ser Ser Gly Val Leu Met
    530                 535                 540

Phe Gly Lys Gln Gly Ala Gly Arg Asp Asn Val Asp Tyr Ser Ser Val
545                 550                 555                 560

Met Leu Thr Ser Glu Glu Glu Ile Lys Thr Thr Asn Pro Val Ala Thr
                565                 570                 575

Glu Gln Tyr Gly Val Val Ala Asp Asn Leu Gln Gln Ala Asn Thr Gly
                580                 585                 590

Pro Ile Val Gly Asn Val Asn Ser Gln Gly Ala Leu Pro Gly Met Val
            595                 600                 605

Trp Gln Asn Arg Asp Val Tyr Leu Gln Gly Pro Ile Trp Ala Lys Ile
    610                 615                 620

Pro His Thr Asp Gly Asn Phe His Pro Ser Pro Leu Met Gly Gly Phe
625                 630                 635                 640

Gly Leu Lys His Pro Pro Pro Gln Ile Leu Ile Lys Asn Thr Pro Val
                645                 650                 655

Pro Ala Asp Pro Pro Thr Thr Phe Ser Gln Ala Lys Leu Ala Ser Phe
                660                 665                 670

Ile Thr Gln Tyr Ser Thr Gly Gln Val Ser Val Glu Ile Glu Trp Glu
            675                 680                 685

Leu Gln Lys Glu Asn Ser Lys Arg Trp Asn Pro Glu Ile Gln Tyr Thr
    690                 695                 700

Ser Asn Tyr Tyr Lys Ser Thr Asn Val Asp Phe Ala Val Asn Thr Glu
705                 710                 715                 720

Gly Thr Tyr Ser Glu Pro Arg Pro Ile Gly Thr Arg Tyr Leu Thr Arg
                725                 730                 735

Asn Leu

<210>  11
<211>  700
<212>  PRT
<213>  AAV-11

<400>  11

Met Ala Ala Asp Gly Tyr Leu Pro Asp Trp Leu Glu Asp Asn Leu Ser
1               5                   10                  15

Glu Gly Ile Arg Glu Trp Trp Asp Leu Lys Pro Gly Ala Pro Lys Pro
            20                  25                  30

Lys Ala Asn Gln Gln Lys Gln Asp Asp Gly Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Ala Ala Asp Ala Ala Ala Leu Glu His Asp Lys Ala Tyr Asp
65                  70                  75                  80

Gln Gln Leu Lys Ala Gly Asp Asn Pro Tyr Leu Arg Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Gly Ala Lys Thr Ala Pro Gly Lys Lys Arg
    130                 135                 140

Pro Leu Glu Ser Pro Gln Glu Pro Asp Ser Ser Ser Gly Ile Gly Lys
145                 150                 155                 160

Lys Gly Lys Gln Pro Ala Arg Lys Arg Leu Asn Phe Glu Glu Asp Thr
                165                 170                 175

Gly Ala Gly Asp Gly Pro Pro Glu Gly Ser Asp Thr Ser Ala Met Ser
            180                 185                 190

Ser Asp Ile Glu Met Arg Ala Ala Pro Gly Gly Asn Ala Val Asp Ala
        195                 200                 205

Gly Gln Gly Ser Asp Gly Val Gly Asn Ala Ser Gly Asp Trp His Cys
    210                 215                 220

Asp Ser Thr Trp Ser Glu Gly Lys Val Thr Thr Thr Ser Thr Arg Thr
225                 230                 235                 240

Trp Val Leu Pro Thr Tyr Asn Asn His Leu Tyr Leu Arg Leu Gly Thr
                245                 250                 255

Thr Ser Ser Ser Asn Thr Tyr Asn Gly Phe Ser Thr Pro Trp Gly Tyr
            260                 265                 270

Phe Asp Phe Asn Arg Phe His Cys His Phe Ser Pro Arg Asp Trp Gln
        275                 280                 285

Arg Leu Ile Asn Asn Asn Trp Gly Leu Arg Pro Lys Ala Met Arg Val
    290                 295                 300

Lys Ile Phe Asn Ile Gln Val Lys Glu Val Thr Thr Ser Asn Gly Glu
305                 310                 315                 320

Thr Thr Val Ala Asn Asn Leu Thr Ser Thr Val Gln Ile Phe Ala Asp
                325                 330                 335

Ser Ser Tyr Glu Leu Pro Tyr Val Met Asp Ala Gly Gln Glu Gly Ser
            340                 345                 350

Leu Pro Pro Phe Pro Asn Asp Val Phe Met Val Pro Gln Tyr Gly Tyr
        355                 360                 365

Cys Gly Ile Val Thr Gly Glu Asn Gln Asn Gln Thr Asp Arg Asn Ala
    370                 375                 380

Phe Tyr Cys Leu Glu Tyr Phe Pro Ser Gln Met Leu Arg Thr Gly Asn
385                 390                 395                 400

Asn Phe Glu Met Ala Tyr Asn Phe Glu Lys Val Pro Phe His Ser Met
                405                 410                 415

Tyr Ala His Ser Gln Ser Leu Asp Arg Leu Met Asn Pro Leu Leu Asp
            420                 425                 430

Gln Tyr Leu Trp His Leu Gln Ser Thr Thr Ser Gly Glu Thr Leu Asn
        435                 440                 445

Gln Gly Asn Ala Ala Thr Thr Phe Gly Lys Ile Arg Ser Gly Asp Phe
    450                 455                 460

Ala Phe Tyr Arg Lys Asn Trp Leu Pro Gly Pro Cys Val Lys Gln Gln
465                 470                 475                 480

Arg Phe Ser Lys Thr Ala Ser Gln Asn Tyr Lys Ile Pro Ala Ser Gly
                485                 490                 495

Gly Asn Ala Leu Leu Lys Tyr Asp Thr His Tyr Thr Leu Asn Asn Arg
            500                 505                 510

Trp Ser Asn Ile Ala Pro Gly Pro Pro Met Ala Thr Ala Gly Pro Ser
        515                 520                 525

Asp Gly Asp Phe Ser Asn Ala Gln Leu Ile Phe Pro Gly Pro Ser Val
    530                 535                 540

Thr Gly Asn Thr Thr Thr Ser Ala Asn Asn Leu Leu Phe Thr Ser Glu

545                 550                 555                 560

Glu Glu Ile Ala Ala Thr Asn Pro Arg Asp Thr Asp Met Phe Gly Gln
                565                 570                 575

Ile Ala Asp Asn Asn Gln Asn Ala Thr Thr Ala Pro Ile Thr Gly Asn
            580                 585                 590

Val Thr Ala Met Gly Val Leu Pro Gly Met Val Trp Gln Asn Arg Asp
        595                 600                 605

Ile Tyr Tyr Gln Gly Pro Ile Trp Ala Lys Ile Pro His Ala Asp Gly
    610                 615                 620

His Phe His Pro Ser Pro Leu Ile Gly Gly Phe Gly Leu Lys His Pro
625                 630                 635                 640

Pro Pro Gln Ile Phe Ile Lys Asn Thr Pro Val Pro Ala Asn Pro Ala
                645                 650                 655

Thr Thr Phe Thr Ala Ala Arg Val Asp Ser Phe Ile Thr Gln Tyr Ser
            660                 665                 670

Thr Gly Gln Val Ala Val Gln Ile Glu Trp Glu Ile Glu Lys Glu Arg
        675                 680                 685

Ser Lys Arg Trp Asn Pro Glu Val Gln Phe Thr Ser
    690                 695                 700

<210>  12
<211>  709
<212>  PRT
<213>  AAVbovine

<400>  12

Gly Asp Pro Val Asn Phe Ala Asp Glu Val Ala Arg Glu His Asp Leu
1               5                   10                  15

Ser Tyr Gln Lys Gln Leu Glu Ala Gly Asp Asn Pro Tyr Leu Lys Tyr
            20                  25                  30

Asn His Ala Asp Ala Glu Phe Gln Glu Lys Leu Ala Ser Asp Thr Ser
        35                  40                  45

Phe Gly Gly Asn Leu Gly Lys Ala Val Phe Gln Ala Lys Lys Arg Ile
    50                  55                  60

Leu Glu Pro Leu Gly Leu Val Glu Thr Pro Asp Lys Thr Ala Pro Ala

65                  70                  75                  80

Ala Lys Lys Arg Pro Leu Glu Gln Ser Pro Gln Glu Pro Asp Ser Ser
                85                  90                  95

Ser Gly Val Gly Lys Lys Gly Lys Gln Pro Ala Arg Lys Arg Leu Asn
            100                 105                 110

Phe Asp Asp Glu Pro Gly Ala Gly Asp Gly Pro Pro Pro Glu Gly Pro
        115                 120                 125

Ser Ser Gly Ala Met Ser Thr Glu Thr Glu Met Arg Ala Ala Ala Gly
    130                 135                 140

Gly Asn Gly Gly Asp Ala Gly Gln Gly Ala Glu Gly Val Gly Asn Ala
145                 150                 155                 160

Ser Gly Asp Trp His Cys Asp Ser Thr Trp Ser Glu Ser His Val Thr
                165                 170                 175

Thr Thr Ser Thr Arg Thr Trp Val Leu Pro Thr Tyr Asn Asn His Leu
            180                 185                 190

Tyr Leu Arg Leu Gly Ser Ser Asn Ala Ser Asp Thr Phe Asn Gly Phe
        195                 200                 205

Ser Thr Pro Trp Gly Tyr Phe Asp Phe Asn Arg Phe His Cys His Phe
    210                 215                 220

Ser Pro Arg Asp Trp Gln Arg Leu Ile Asn Asn His Trp Gly Leu Arg
225                 230                 235                 240

Pro Lys Ser Met Gln Val Arg Ile Phe Asn Ile Gln Val Lys Glu Val
                245                 250                 255

Thr Thr Ser Asn Gly Glu Thr Thr Val Ser Asn Asn Leu Thr Ser Thr
            260                 265                 270

Val Gln Ile Phe Ala Asp Ser Thr Tyr Glu Leu Pro Tyr Val Met Asp
        275                 280                 285

Ala Gly Gln Glu Gly Ser Leu Pro Pro Phe Pro Asn Asp Val Phe Met
    290                 295                 300

Val Pro Gln Tyr Gly Tyr Cys Gly Leu Val Thr Gly Gly Ser Ser Gln
305                 310                 315                 320

Asn Gln Thr Asp Arg Asn Ala Phe Tyr Cys Leu Glu Tyr Phe Pro Ser
                    325                 330                 335

Gln Met Leu Arg Thr Gly Asn Asn Phe Glu Met Val Tyr Lys Phe Glu
            340                 345                 350

Asn Val Pro Phe His Ser Met Tyr Ala His Ser Gln Ser Leu Asp Arg
        355                 360                 365

Leu Met Asn Pro Leu Leu Asp Gln Tyr Leu Trp Glu Leu Gln Ser Thr
    370                 375                 380

Thr Ser Gly Gly Thr Leu Asn Gln Gly Asn Ser Ala Thr Asn Phe Ala
385                 390                 395                 400

Lys Leu Thr Lys Thr Asn Phe Ser Gly Tyr Arg Lys Asn Trp Leu Pro
                405                 410                 415

Gly Pro Met Met Lys Gln Gln Arg Phe Ser Lys Thr Ala Ser Gln Asn
            420                 425                 430

Tyr Lys Ile Pro Gln Gly Arg Asn Asn Ser Leu Leu His Tyr Glu Thr
        435                 440                 445

Arg Thr Thr Leu Asp Gly Arg Trp Ser Asn Phe Ala Pro Gly Thr Ala
    450                 455                 460

Met Ala Thr Ala Ala Asn Asp Ala Thr Asp Phe Ser Gln Ala Gln Leu
465                 470                 475                 480

Ile Phe Ala Gly Pro Asn Ile Thr Gly Asn Thr Thr Thr Asp Ala Asn
                485                 490                 495

Asn Leu Met Phe Thr Ser Glu Asp Glu Leu Arg Ala Thr Asn Pro Arg
            500                 505                 510

Asp Thr Asp Leu Phe Gly His Leu Ala Thr Asn Gln Gln Asn Ala Thr
        515                 520                 525

Thr Val Pro Thr Val Asp Asp Val Asp Gly Val Gly Val Tyr Pro Gly
    530                 535                 540

Met Val Trp Gln Asp Arg Asp Ile Tyr Tyr Gln Gly Pro Ile Trp Ala
545                 550                 555                 560

Lys Ile Pro His Thr Asp Gly His Phe His Pro Ser Pro Leu Ile Gly
                565                 570                 575

Gly Phe Gly Leu Lys Ser Pro Pro Pro Gln Ile Phe Ile Lys Asn Thr
                580                 585                 590

Pro Val Pro Ala Asn Pro Ala Thr Thr Phe Ser Pro Ala Arg Ile Asn
        595                 600                 605

Ser Phe Ile Thr Gln Tyr Ser Thr Gly Gln Val Ala Val Lys Ile Glu
    610                 615                 620

Trp Glu Ile Gln Lys Glu Arg Ser Lys Arg Trp Asn Pro Glu Val Gln
625                 630                 635                 640

Phe Thr Ser Asn Tyr Gly Ala Gln Asp Ser Leu Leu Trp Ala Pro Asp
                645                 650                 655

Asn Ala Gly Ala Tyr Lys Glu Pro Arg Ala Ile Gly Ser Arg Tyr Leu
            660                 665                 670

Thr Asn His Leu Asn Tyr Gly Asn Gln Ser Ser Met Leu Trp Ala Pro
        675                 680                 685

Asp Thr Thr Gly Lys Tyr Thr Glu Pro Arg Val Ile Gly Ser Arg Tyr
    690                 695                 700

Leu Thr Asn His Leu
705

<210>  13
<211>  7
<212>  PRT
<213>  artificial

<220>
<223>  peptide

<400>  13

Lys Gly Gly Ala Leu Gly Gly
1               5

<210>  14
<211>  7
<212>  PRT
<213>  artificial

<220>
<223>  peptide

<400>  14

Ala Ser Thr Gly Pro His Thr
1               5

<210>  15
<211>  7
<212>  PRT
<213>  artificial

<220>
<223>  peptide

<400>  15

Thr Ser Pro Ser Pro Ser Arg
1               5


<210>  16
<211>  7
<212>  PRT
<213>  artificial

<220>
<223>  peptide

<400>  16

Asn Asn Pro Gly Ala Pro Arg
1               5


<210>  17
<211>  7
<212>  PRT
<213>  artificial

<220>
<223>  peptide

<400>  17

Gly Val Ser Pro Glu Arg Arg
1               5


<210>  18
<211>  7
<212>  PRT
<213>  artificial

<220>
<223>  peptide

<400>  18

Trp His Ala Pro Thr His Gly
1               5


<210>  19
<211>  7
<212>  PRT
<213>  artificial

<220>
<223>  peptide

<400>  19

Asn Ala Gln Pro Pro Ser Arg
1               5


<210>  20
<211>  7
<212>  PRT
<213>  artificial

<220>
<223>  peptide

<400>  20

His Ala Ala Gly Leu Pro Lys
1               5


<210>  21
<211>  7
<212>  PRT
<213>  artificial

<220>
<223>  peptide

<400>  21

Pro Ala Asn Gln Ser Val Arg
1               5


<210>  22
<211>  7
<212>  PRT
<213>  artificial

<220>
<223>  peptide

<400>  22

Gly Ser Asn Ala Thr Ala Ser
1               5


<210>  23
<211>  7
<212>  PRT
<213>  artificial

<220>
<223>  peptide

<400>  23

Gly Ala His Arg Ser Asp Ser
1               5

<210>  24
<211>  7
<212>  PRT
<213>  artificial

<220>
<223>  peptide

<400>  24

Ala Asn Tyr Gln Gly Pro Ala
1               5


<210>  25
<211>  7
<212>  PRT
<213>  artificial

<220>
<223>  peptide

<400>  25

Asn His Thr Ala Ala Thr Trp
1               5


<210>  26
<211>  7
<212>  PRT
<213>  artificial

<220>
<223>  peptide

<400>  26

Thr Asn His Ala Ser Ala Ala
1               5


<210>  27
<211>  7
<212>  PRT
<213>  artificial

<220>
<223>  peptide

<400>  27

Asn Tyr Pro Gly Ala His Ser
1               5


<210>  28
<211>  7
<212>  PRT
<213>  artificial

<220>
<223>  peptide

<400>  28

Ile Gly Thr Asn Ser Pro Val
1               5


<210>  29
<211>  7
<212>  PRT
<213>  artificial

<220>
<223>  peptide

<400>  29

Ala Gln Tyr Ser Ala Ala Val
1               5


<210>  30
<211>  7
<212>  PRT
<213>  artificial

<220>
<223>  peptide

<400>  30

Ser Tyr Gly Gln Ser Ala Gly
1               5


<210>  31
<211>  7
<212>  PRT
<213>  artificial

<220>
<223>  peptide

<400>  31

Ala His Gly Gly Ser Ile Pro
1               5


<210>  32
<211>  7
<212>  PRT
<213>  artificial

<220>
<223>  peptide

<400>  32

Tyr Gln Ser Gly Ser Leu Pro
1               5

<210>  33
<211>  7
<212>  PRT
<213>  artificial

<220>
<223>  peptide

<400>  33

Asp Ser Gln His Thr Gln His
1               5


<210>  34
<211>  7
<212>  PRT
<213>  artificial

<220>
<223>  peptide

<400>  34

Gly Ser Asp Arg Val Ser Thr
1               5


<210>  35
<211>  7
<212>  PRT
<213>  artificial

<220>
<223>  peptide

<400>  35

Thr Ser Asn Arg Ala Ala Thr
1               5


<210>  36
<211>  7
<212>  PRT
<213>  artificial

<220>
<223>  peptide

<400>  36

Gly Gly Thr Thr Leu Ala His
1               5


<210>  37
<211>  7
<212>  PRT
<213>  artificial

<220>
<223>  peptide

<400>  37

Ser Ser Pro Asn His Val Ala
1               5


<210>  38
<211>  7
<212>  PRT
<213>  artificial

<220>
<223>  peptide

<400>  38

Gly Asn Ala Arg Pro Pro Asp
1               5


<210>  39
<211>  7
<212>  PRT
<213>  artificial

<220>
<223>  peptide

<400>  39

Pro Gly Gln Thr Arg Asp Ser
1               5


<210>  40
<211>  7
<212>  PRT
<213>  artificial

<220>
<223>  peptide

<400>  40

Glu Asn Ala Thr Leu Arg Thr
1               5


<210>  41
<211>  7
<212>  PRT
<213>  artificial

<220>
<223>  peptide

<400>  41

His Thr Arg Thr Ser Gln Glu
1               5

<210>  42
<211>  7
<212>  PRT
<213>  artificial

<220>
<223>  peptide

<400>  42

Pro Val Gly Ser Pro Ser Arg
1               5


<210>  43
<211>  7
<212>  PRT
<213>  artificial

<220>
<223>  peptide

<400>  43

Ser Ser Gly Thr Thr Arg Asp
1               5


<210>  44
<211>  7
<212>  PRT
<213>  artificial

<220>
<223>  peptide

<400>  44

Asp Asp Gly Arg Pro Ser Arg
1               5


<210>  45
<211>  7
<212>  PRT
<213>  artificial

<220>
<223>  peptide

<400>  45

Ala Ala Tyr Gly Ala Gln Ser
1               5


<210>  46
<211>  7
<212>  PRT
<213>  artificial

<220>
<223>  peptide

<400>  46

Arg Glu Gly Ser Pro Gly Arg
1               5


<210>  47
<211>  7
<212>  PRT
<213>  artificial

<220>
<223>  peptide

<400>  47

Asn Lys Thr Gly Ala Ser Pro
1               5


<210>  48
<211>  7
<212>  PRT
<213>  artificial

<220>
<223>  peptide

<400>  48

Lys Ala Ser Gly Gln Thr Val
1               5


<210>  49
<211>  7
<212>  PRT
<213>  artificial

<220>
<223>  peptide

<400>  49

Arg Asp Thr Thr Thr Gly Ala
1               5


<210>  50
<211>  7
<212>  PRT
<213>  artificial

<220>
<223>  peptide

<400>  50

Ala Tyr Gly Pro Asn Ala Gly
1               5

<210>  51
<211>  7
<212>  PRT
<213>  artificial

<220>
<223>  peptide

<400>  51

Asp Ser Asp Gly Pro Arg Pro
1               5


<210>  52
<211>  7
<212>  PRT
<213>  artificial

<220>
<223>  peptide

<400>  52

Gly Ser Ser Ala Thr Ala Ser
1               5


<210>  53
<211>  7
<212>  PRT
<213>  artificial

<220>
<223>  peptide

<400>  53

Thr Ser Ala Gly Ser Pro Arg
1               5


<210>  54
<211>  7
<212>  PRT
<213>  artificial

<220>
<223>  peptide

<400>  54

Gly Pro Ala His Thr Ser Ser
1               5


<210>  55
<211>  7
<212>  PRT
<213>  artificial

<220>
<223>  peptide

<400>  55

Asn Ala Ala Gly Thr Pro Arg
1               5


<210>  56
<211>  7
<212>  PRT
<213>  artificial

<220>
<223>  peptide

<400>  56

Asn Arg Thr Ala Leu Gly Ser
1               5


<210>  57
<211>  7
<212>  PRT
<213>  artificial

<220>
<223>  peptide

<400>  57

Lys Ser His Ser Ala Ser Pro
1               5


<210>  58
<211>  7
<212>  PRT
<213>  artificial

<220>
<223>  peptide

<400>  58

Arg Gly His Thr Ala Pro Ile
1               5


<210>  59
<211>  7
<212>  PRT
<213>  artificial

<220>
<223>  peptide

<400>  59

Pro Thr Ser Gly Ala Pro Lys
1               5

<210>  60
<211>  7
<212>  PRT
<213>  artificial

<220>
<223>  peptide

<400>  60

Gly Asn Arg Val Gln Ser Thr
1               5


<210>  61
<211>  7
<212>  PRT
<213>  artificial

<220>
<223>  peptide

<400>  61

Asn Thr Ser Leu Thr Ala Arg
1               5


<210>  62
<211>  7
<212>  PRT
<213>  artificial

<220>
<223>  peptide

<400>  62

Arg Asp Arg Ala Thr Ser Pro
1               5


<210>  63
<211>  7
<212>  PRT
<213>  artificial

<220>
<223>  peptide

<400>  63

Gly Ala Glu Arg Thr Tyr Ser
1               5


<210>  64
<211>  7
<212>  PRT
<213>  artificial

<220>
<223>  peptide

<400>  64

Ala Arg Gly Ser Val Ser Pro
1               5


<210>  65
<211>  7
<212>  PRT
<213>  artificial

<220>
<223>  peprtide

<400>  65

Thr Gly Arg Ser Ser Gly Asp
1               5


<210>  66
<211>  7
<212>  PRT
<213>  artificial

<220>
<223>  peptide

<400>  66

His Pro Ala Arg Pro Asn Ala
1               5


<210>  67
<211>  7
<212>  PRT
<213>  artificial

<220>
<223>  peptide

<400>  67

Ser Asp His Leu His Pro Arg
1               5


<210>  68
<211>  7
<212>  PRT
<213>  artificial

<220>
<223>  peptide

<400>  68

Thr Val Leu Ser Ser Gln Arg
1               5

<210>  69
<211>  7
<212>  PRT
<213>  artificial

<220>
<223>  peptide

<400>  69

Val Ser Ser Ser Ser Arg Pro
1               5


<210>  70
<211>  7
<212>  PRT
<213>  artificial

<220>
<223>  peptide

<400>  70

Thr Gly Ser Ser Lys Gly Val
1               5


<210>  71
<211>  7
<212>  PRT
<213>  artificial

<220>
<223>  peptide

<400>  71

Ser Pro Gly Gly Pro Ala Arg
1               5


<210>  72
<211>  7
<212>  PRT
<213>  artificial

<220>
<223>  peptide

<400>  72

Gly Arg Ser Glu Gln Ile Arg
1               5


<210>  73
<211>  7
<212>  PRT
<213>  artificial

<220>
<223>  peptide

<400>  73

Val Thr Ala Arg Pro Glu Gly
1               5


<210>  74
<211>  7
<212>  PRT
<213>  artificial

<220>
<223>  peptide

<400>  74

Arg Asp Pro Ser Pro Gly Arg
1               5


<210>  75
<211>  7
<212>  PRT
<213>  artificial

<220>
<223>  peptide

<400>  75

Ala Ala Ser Gly Val Ala Arg
1               5


<210>  76
<211>  7
<212>  PRT
<213>  artificial

<220>
<223>  peptide

<400>  76

Thr Ser Ala Arg Ser Val Pro
1               5


<210>  77
<211>  7
<212>  PRT
<213>  artificial

<220>
<223>  peptide

<400>  77

Ala Arg Gly Gly Ser Ile Pro
1               5

<210>  78
<211>  7
<212>  PRT
<213>  artificial

<220>
<223>  peptide

<400>  78

Arg Ala Ala Gly Gln Val Ala
1               5


<210>  79
<211>  7
<212>  PRT
<213>  artificial

<220>
<223>  peptide

<400>  79

Thr Ser Gln Gln Arg Ile Pro
1               5


<210>  80
<211>  7
<212>  PRT
<213>  artificial

<220>
<223>  peptide

<400>  80

Asn Ser Ala Arg Pro Asn Ser
1               5


<210>  81
<211>  7
<212>  PRT
<213>  artificial

<220>
<223>  peptide

<400>  81

Pro Val Gly Ser Pro Gly Arg
1               5


<210>  82
<211>  7
<212>  PRT
<213>  artificial

<220>
<223>  peptide

<400>  82

Thr Gln Ser Pro Pro Gly Arg
1               5


<210>  83
<211>  7
<212>  PRT
<213>  artificial

<220>
<223>  peptride

<400>  83

Gly Arg Arg Asp Ser Pro Val
1               5


<210>  84
<211>  7
<212>  PRT
<213>  artificial

<220>
<223>  peptide

<400>  84

Asn Gly Ser Pro Val Gly Arg
1               5


<210>  85
<211>  7
<212>  PRT
<213>  artificial

<220>
<223>  peptide

<400>  85

Lys Val Ser Gly His Gln Ala
1               5


<210>  86
<211>  7
<212>  PRT
<213>  artificial

<220>
<223>  peptide

<400>  86

Ala Arg Ser Gln Val His Ala
1               5

<210>  87
<211>  7
<212>  PRT
<213>  artificial

<220>
<223>  peptide

<400>  87

Ala Arg Thr Thr His Ser Pro
1               5


<210>  88
<211>  7
<212>  PRT
<213>  artificial

<220>
<223>  peprtide

<400>  88

Asn Leu Ser Arg Ala Ser Pro
1               5


<210>  89
<211>  7
<212>  PRT
<213>  artificial

<220>
<223>  peptide

<400>  89

Asn Thr Pro Gln Ser Ala Arg
1               5


<210>  90
<211>  7
<212>  PRT
<213>  artificial

<220>
<223>  peptide

<400>  90

Gly Arg Pro Gly Ser Gly Tyr
1               5


<210>  91
<211>  7
<212>  PRT
<213>  artificial

<220>
<223>  peptide

<400>  91

Val Pro Ser Ala Pro Gln Arg
1               5


<210>  92
<211>  7
<212>  PRT
<213>  artificial

<220>
<223>  peptide

<400>  92

Ala Pro Ala Asn Leu Pro Arg
1               5


<210>  93
<211>  7
<212>  PRT
<213>  artificial

<220>
<223>  peptide

<400>  93

Leu Gly Arg Pro Ala Gly Pro
1               5


<210>  94
<211>  7
<212>  PRT
<213>  artificial

<220>
<223>  peptide

<400>  94

Val Leu Gly Thr Lys Gly Ala
1               5


<210>  95
<211>  7
<212>  PRT
<213>  artificial

<220>
<223>  peptide

<400>  95

Gly Arg Pro Glu Gln Ile Arg
1               5

<210>  96
<211>  7
<212>  PRT
<213>  artificial

<220>
<223>  peptide

<400>  96

Gly Leu Ala Gly Val Val Arg
1               5


<210>  97
<211>  7
<212>  PRT
<213>  artificial

<220>
<223>  peptide

<400>  97

Ala His Arg Ser Thr Gly Thr
1               5


<210>  98
<211>  7
<212>  PRT
<213>  artificial

<220>
<223>  peptide

<400>  98

Gly Thr Ser Pro Ala Asn Arg
1               5


<210>  99
<211>  7
<212>  PRT
<213>  artificial

<220>
<223>  peptide

<400>  99

Asn Arg Pro Asn Ala Ala Ser
1               5


<210>  100
<211>  7
<212>  PRT
<213>  artificial

<220>
<223>  peptide

<400>  100

Ala Arg Ser Gly Ser Gly Ala
1               5


<210>  101
<211>  7
<212>  PRT
<213>  artificial

<220>
<223>  peptide#

<400>  101

Arg His Ser Ser Thr Pro Val
1               5


<210>  102
<211>  7
<212>  PRT
<213>  artificial

<220>
<223>  peptide

<400>  102

Asn Ser Pro Leu Gly Thr Arg
1               5


<210>  103
<211>  7
<212>  PRT
<213>  artificial

<220>
<223>  peptide

<400>  103

Asn Thr Pro Ser Pro Ala Arg
1               5


<210>  104
<211>  7
<212>  PRT
<213>  artificial

<220>
<223>  peptide

<400>  104

Asn Ser Pro Ala Ala Gly Arg
1               5

<210>  105
<211>  7
<212>  PRT
<213>  artificial

<220>
<223>  peptide

<400>  105

Ser Asn Arg Thr Ser Ser Val
1               5


<210>  106
<211>  7
<212>  PRT
<213>  artificial

<220>
<223>  peptide

<400>  106

Ser Val Arg Gly Gly Thr Pro
1               5


<210>  107
<211>  7
<212>  PRT
<213>  artificial

<220>
<223>  peptide

<400>  107

Ser Pro Ala Asp Ser Arg Val
1               5


<210>  108
<211>  7
<212>  PRT
<213>  artificial

<220>
<223>  peptide

<400>  108

Ser Thr Arg Gly Gln Gly His
1               5


<210>  109
<211>  7
<212>  PRT
<213>  artificial

<220>
<223>  peptide

<400>  109

Val Asp Ser Gly Leu Ser Gly
1               5


<210>  110
<211>  7
<212>  PRT
<213>  artificial

<220>
<223>  peptide

<400>  110

Gly Asp Gly Ser Phe Ser Gly
1               5


<210>  111
<211>  7
<212>  PRT
<213>  artificial

<220>
<223>  peptide

<400>  111

Val Ser Gly Pro Pro His Thr
1               5


<210>  112
<211>  7
<212>  PRT
<213>  artificial

<220>
<223>  peptide

<400>  112

Arg His Gly Pro Gln His Pro
1               5


<210>  113
<211>  7
<212>  PRT
<213>  artificial

<220>
<223>  peptide

<400>  113

Arg Asp Arg Ala Ala Ser Pro
1               5

<210>  114
<211>  7
<212>  PRT
<213>  artificial

<220>
<223>  peptide

<400>  114

Gly Asp Arg Ala Thr Ser Pro
1               5


<210>  115
<211>  7
<212>  PRT
<213>  artificial

<220>
<223>  peptide

<400>  115

Arg Gly Arg Ala Thr Ser Pro
1               5


<210>  116
<211>  7
<212>  PRT
<213>  artificial

<220>
<223>  peptide

<400>  116

Arg Asp Arg Val Thr Ser Pro
1               5


<210>  117
<211>  7
<212>  PRT
<213>  artificial

<220>
<223>  peptide

<400>  117

Pro Val Leu Asn Pro Pro Pro
1               5


<210>  118
<211>  7
<212>  PRT
<213>  artificial

<220>
<223>  peptride

<400>  118

Ser Phe Gln Arg Ala Ser Pro
1               5


<210>  119
<211>  7
<212>  PRT
<213>  artificial

<220>
<223>  peptide

<400>  119

Arg Asp Arg Ala Thr Thr Pro
1               5


<210>  120
<211>  7
<212>  PRT
<213>  artificial

<220>
<223>  peptide

<400>  120

Gln Ser Arg Leu Ser Thr Gln
1               5


<210>  121
<211>  7
<212>  PRT
<213>  artificial

<220>
<223>  peptide

<400>  121

Ser Ser Gly Gly Gly Thr Pro
1               5


<210>  122
<211>  7
<212>  PRT
<213>  artificial

<220>
<223>  peptide

<400>  122

Lys Gly Glu Ala Leu Gly Gly
1               5

<210>  123
<211>  7
<212>  PRT
<213>  artificial

<220>
<223>  peptide

<400>  123

Ser Val Arg Gly Ala Pro Pro
1               5


<210>  124
<211>  7
<212>  PRT
<213>  artificial

<220>
<223>  peptide

<400>  124

Asp His Asp Ser Pro Ser Val
1               5


<210>  125
<211>  7
<212>  PRT
<213>  artificial

<220>
<223>  peptide

<400>  125

Arg Asp Gln Ala Thr Ser Pro
1               5


<210>  126
<211>  7
<212>  PRT
<213>  artificial

<220>
<223>  peptide

<400>  126

Arg Asp Arg Ala Thr Ser Ser
1               5


<210>  127
<211>  7
<212>  PRT
<213>  artificial

<220>
<223>  peptide

<400>  127

Gly Lys Pro Pro Arg Thr Glu
1               5


<210>  128
<211>  7
<212>  PRT
<213>  artificial

<220>
<223>  peptide

<400>  128

Ala His Arg Gly Leu Gln Val
1               5


<210>  129
<211>  7
<212>  PRT
<213>  artificial

<220>
<223>  peptide

<400>  129

Val Ala Ser Leu Ala Ala Arg
1               5


<210>  130
<211>  7
<212>  PRT
<213>  artificial

<220>
<223>  peptide

<400>  130

Arg Asp Arg Thr Thr Ser Pro
1               5


<210>  131
<211>  7
<212>  PRT
<213>  artificial

<220>
<223>  peptide

<400>  131

Arg Asp Trp Ala Thr Ser Pro
1               5

<210>  132
<211>  7
<212>  PRT
<213>  artificial

<220>
<223>  peprtide

<400>  132

Arg Asn Arg Ala Thr Ser Pro
1               5


<210>  133
<211>  7
<212>  PRT
<213>  artificial

<220>
<223>  peptide

<400>  133

Arg Asp Arg Ala Thr Ser Leu
1               5


<210>  134
<211>  7
<212>  PRT
<213>  artificial

<220>
<223>  peptide

<400>  134

Lys Asp Arg Ala Thr Ser Pro
1               5


<210>  135
<211>  7
<212>  PRT
<213>  artificial

<220>
<223>  peptide

<400>  135

Ala Arg Tyr Ser Ala Ala Val
1               5


<210>  136
<211>  7
<212>  PRT
<213>  artificial

<220>
<223>  peptide

<400>  136

Gly Ala His Arg Ser Val Ser
1               5


<210>  137
<211>  7
<212>  PRT
<213>  artificial

<220>
<223>  peptide

<400>  137

Trp Asp Arg Ala Thr Ser Pro
1               5


<210>  138
<211>  7
<212>  PRT
<213>  artificial

<220>
<223>  peptide

<400>  138

Glu Arg Pro Tyr Gln Ala Gly
1               5


<210>  139
<211>  7
<212>  PRT
<213>  artificial

<220>
<223>  peptide

<400>  139

Asn Ala Pro Gly Asn Leu Arg
1               5


<210>  140
<211>  7
<212>  PRT
<213>  artificial

<220>
<223>  peptide

<400>  140

Asn Asn Pro Leu Pro Gln Arg
1               5

<210>  141
<211>  7
<212>  PRT
<213>  artificial

<220>
<223>  peptide

<400>  141

Ala Arg Pro Thr Val Pro Tyr
1               5


<210>  142
<211>  7
<212>  PRT
<213>  artificial

<220>
<223>  peptide

<400>  142

Arg Ser Gly Ala Ala Val Ala
1               5


<210>  143
<211>  7
<212>  PRT
<213>  artificial

<220>
<223>  peptide

<400>  143

Asn Thr Pro Asn Pro Leu Arg
1               5


<210>  144
<211>  7
<212>  PRT
<213>  artificial

<220>
<223>  peptide

<400>  144

Gly Gly Thr Thr Leu Gly Arg
1               5


<210>  145
<211>  7
<212>  PRT
<213>  artificial

<220>
<223>  peptide

<400>  145

Ala Ser Thr Gly Leu Pro Arg
1               5


<210>  146
<211>  7
<212>  PRT
<213>  artificial

<220>
<223>  peptide

<400>  146

Ala Thr Gly Val Ile Gln Arg
1               5


<210>  147
<211>  7
<212>  PRT
<213>  artificial

<220>
<223>  peptide

<400>  147

Asn Ser Ala Ala Leu Gly Arg
1               5


<210>  148
<211>  7
<212>  PRT
<213>  artificial

<220>
<223>  peptide

<400>  148

Gly Glu Pro Gln Pro Arg Phe
1               5


<210>  149
<211>  7
<212>  PRT
<213>  artificial

<220>
<223>  peptide

<400>  149

Gly His Ser Ala Arg Ser Ile
1               5

<210>  150
<211>  7
<212>  PRT
<213>  artificial

<220>
<223>  peptide

<400>  150

Val Ser Ser Ser Thr Pro Arg
1               5


<210>  151
<211>  7
<212>  PRT
<213>  artificial

<220>
<223>  peptide

<400>  151

Asn Gln Pro Asn Pro Val Arg
1               5


<210>  152
<211>  7
<212>  PRT
<213>  artificial

<220>
<223>  peptide

<400>  152

Pro Ser Gly Gly Pro Pro Arg
1               5


<210>  153
<211>  7
<212>  PRT
<213>  artificial

<220>
<223>  peptide

<400>  153

Ala Arg Asp Gln Leu Pro Arg
1               5


<210>  154
<211>  7
<212>  PRT
<213>  artificial

<220>
<223>  peptide

<400>  154

Arg Gly Ser His Pro Asn Ile
1               5


<210>  155
<211>  7
<212>  PRT
<213>  artificial

<220>
<223>  peptide

<400>  155

Asn Arg Ala Ser Asn Thr Ser
1               5


<210>  156
<211>  7
<212>  PRT
<213>  artificial

<220>
<223>  peptide

<400>  156

Val Asn Ala Val Pro Gly Arg
1               5


<210>  157
<211>  7
<212>  PRT
<213>  artificial

<220>
<223>  peptide

<400>  157

Pro Ala Asn Thr Pro Gly Arg
1               5


<210>  158
<211>  7
<212>  PRT
<213>  artificial

<220>
<223>  peptide

<400>  158

Ile Ser Ser Ser Thr Ala Arg
1               5

<210>  159
<211>  7
<212>  PRT
<213>  artificial

<220>
<223>  peptide

<400>  159

Ala Arg Ala Gly Arg Asp Leu
1               5


<210>  160
<211>  7
<212>  PRT
<213>  artificial

<220>
<223>  peptide

<400>  160

Arg Gly Gly Glu Val Pro Arg
1               5


<210>  161
<211>  7
<212>  PRT
<213>  artificial

<220>
<223>  peptide

<400>  161

Val Asn Leu Val Pro Ser Arg
1               5


<210>  162
<211>  7
<212>  PRT
<213>  artificial

<220>
<223>  peptide

<400>  162

Leu Ala Arg Asn Gln Ser Gly
1               5


<210>  163
<211>  7
<212>  PRT
<213>  artificial

<220>
<223>  peptide

<400>  163

Ser Ser Ser Gly Ser Pro Arg
1               5


<210>  164
<211>  7
<212>  PRT
<213>  artificial

<220>
<223>  peptide

<400>  164

Ser Pro Gly Ile Asn Leu Arg
1               5


<210>  165
<211>  7
<212>  PRT
<213>  artificial

<220>
<223>  peptide

<400>  165

Ala Val Ala Gly Ser Pro Arg
1               5


<210>  166
<211>  7
<212>  PRT
<213>  artificial

<220>
<223>  peptide

<400>  166

Arg Ser Pro Asp Ser Ala Arg
1               5


<210>  167
<211>  7
<212>  PRT
<213>  artificial

<220>
<223>  peptide

<400>  167

Asn Gln Pro Val Pro Asn Arg
1               5

<210>  168
<211>  7
<212>  PRT
<213>  artificial

<220>
<223>  peptide

<400>  168

Pro Ser Ser Pro Val Ala Arg
1               5


<210>  169
<211>  7
<212>  PRT
<213>  artificial

<220>
<223>  peptide

<400>  169

Lys Asp Ala Gln Leu Gly Arg
1               5


<210>  170
<211>  7
<212>  PRT
<213>  artificial

<220>
<223>  peptide

<400>  170

Val Thr Ser Ser Ser Gly Lys
1               5


<210>  171
<211>  7
<212>  PRT
<213>  artificial

<220>
<223>  peptide

<400>  171

Ala Ala Asn Met Thr Ala Arg
1               5


<210>  172
<211>  7
<212>  PRT
<213>  artificial

<220>
<223>  peptide

<400>  172

Ser Ser Ile Asn Pro Pro Arg
1               5


<210>  173
<211>  7
<212>  PRT
<213>  artificial

<220>
<223>  peptide

<400>  173

Ala Tyr Ala Gly Thr Pro Lys
1               5


<210>  174
<211>  7
<212>  PRT
<213>  artificial

<220>
<223>  peptide

<400>  174

Arg Gly Ser Ser Pro Val Arg
1               5


<210>  175
<211>  7
<212>  PRT
<213>  artificial

<220>
<223>  peptide

<400>  175

Gly Gly Gly Leu Thr Pro Arg
1               5


<210>  176
<211>  7
<212>  PRT
<213>  artificial

<220>
<223>  peptide

<400>  176

Gly Ala Ser Ser Val Ser Arg
1               5

<210>  177
<211>  7
<212>  PRT
<213>  artificial

<220>
<223>  peptide

<400>  177

Val His Leu Ser Pro Gly Arg
1               5


<210>  178
<211>  7
<212>  PRT
<213>  artificial

<220>
<223>  peptide

<400>  178

Asn Ser Pro Asn Val Ala Arg
1               5


<210>  179
<211>  7
<212>  PRT
<213>  artificial

<220>
<223>  peptide

<400>  179

Gly Gly Arg Asp Gln Leu Arg
1               5


<210>  180
<211>  7
<212>  PRT
<213>  artificial

<220>
<223>  peptide

<400>  180

Ala Ala Gln Gly Met Pro Lys
1               5


<210>  181
<211>  7
<212>  PRT
<213>  artificial

<220>
<223>  peptide

<400>  181

Arg Arg Asp Leu Asp Val Arg
1               5


<210>  182
<211>  7
<212>  PRT
<213>  artificial

<220>
<223>  peptide

<400>  182

Val Thr Ser Thr Ser Ala Arg
1               5


<210>  183
<211>  7
<212>  PRT
<213>  artificial

<220>
<223>  peptide

<400>  183

Arg His Gly Ser Pro Gly Ser
1               5


<210>  184
<211>  7
<212>  PRT
<213>  artificial

<220>
<223>  peptide

<400>  184

Asn Ser Pro Ser Gly Pro Arg
1               5


<210>  185
<211>  7
<212>  PRT
<213>  artificial

<220>
<223>  peptide

<400>  185

Arg Asp Gly Ile Thr Ser Arg
1               5

<210>  186
<211>  7
<212>  PRT
<213>  artificial

<220>
<223>  peptide

<400>  186

Ser Asn Ala Pro Ser Ala Arg
1               5


<210>  187
<211>  7
<212>  PRT
<213>  artificial

<220>
<223>  peptide

<400>  187

Val Met Asn Gly Gln Leu Arg
1               5


<210>  188
<211>  7
<212>  PRT
<213>  artificial

<220>
<223>  peptide

<400>  188

Tyr Ser Ala Gly Ser Pro Lys
1               5


<210>  189
<211>  7
<212>  PRT
<213>  artificial

<220>
<223>  peptide

<400>  189

Ser Asp Asn His Thr Ala Arg
1               5


<210>  190
<211>  7
<212>  PRT
<213>  artificial

<220>
<223>  peptide

<400>  190

Arg Asp Arg Ala Gly Pro Pro
1               5


<210>  191
<211>  7
<212>  PRT
<213>  artificial

<220>
<223>  peptide

<400>  191

Arg Val Arg Ala Thr Ser Pro
1               5


<210>  192
<211>  7
<212>  PRT
<213>  artificial

<220>
<223>  peptide

<400>  192

Ser Thr Arg Gly His Gly Ala
1               5


<210>  193
<211>  7
<212>  PRT
<213>  artificial

<220>
<223>  peptide

<400>  193

Ser Thr Arg Gly Arg Gly Ala
1               5


<210>  194
<211>  7
<212>  PRT
<213>  artificial

<220>
<223>  peptide

<400>  194

Asn Asp Val Arg Ala Pro Pro
1               5

<210>  195
<211>  7
<212>  PRT
<213>  artificial

<220>
<223>  peptide

<400>  195

Ser Asn Gly Gly Ser Pro Val
1               5


<210>  196
<211>  7
<212>  PRT
<213>  artificial

<220>
<223>  peptide

<400>  196

Met His Ala Ser Pro Ser Gly
1               5


<210>  197
<211>  7
<212>  PRT
<213>  artificial

<220>
<223>  peptide

<400>  197

Ala Ala Ser His Pro Asn Gln
1               5


<210>  198
<211>  7
<212>  PRT
<213>  artificial

<220>
<223>  peptide

<400>  198

Asp Thr Gly Ser His Ala Leu
1               5


<210>  199
<211>  7
<212>  PRT
<213>  artificial

<220>
<223>  peptide

<400>  199

Ser Pro Ser Asp Arg Phe Ser
1               5


<210>  200
<211>  7
<212>  PRT
<213>  artificial

<220>
<223>  peptide

<400>  200

Gly Pro Asn Ala Thr Ala Ser
1               5


<210>  201
<211>  7
<212>  PRT
<213>  artificial

<220>
<223>  peptide

<400>  201

Gly Gly Pro Gly Tyr Thr Ala
1               5


<210>  202
<211>  7
<212>  PRT
<213>  artificial

<220>
<223>  peptide

<400>  202

Gly Ser Asp Ala Thr Ala Ser
1               5


<210>  203
<211>  7
<212>  PRT
<213>  artificial

<220>
<223>  peptide

<400>  203

Ala Gln Tyr Ser Ala Ala Ala
1               5

<210>  204
<211>  7
<212>  PRT
<213>  artificial

<220>
<223>  peptide

<400>  204

Val Pro Thr Leu Arg Pro Ala
1               5


<210>  205
<211>  7
<212>  PRT
<213>  artificial

<220>
<223>  peptide

<400>  205

Ala Gly Thr Gly Ile Pro Val
1               5


<210>  206
<211>  7
<212>  PRT
<213>  artificial

<220>
<223>  peptide

<400>  206

Gly Ser Asn Ala Thr Ala Gly
1               5


<210>  207
<211>  7
<212>  PRT
<213>  artificial

<220>
<223>  peptide

<400>  207

Gly Pro Ala Asn Ser Ser His
1               5


<210>  208
<211>  7
<212>  PRT
<213>  artificial

<220>
<223>  peptide

<400>  208

Ala Ala Arg Ser Thr Asp Leu
1               5


<210>  209
<211>  7
<212>  PRT
<213>  artificial

<220>
<223>  peptide

<400>  209

Ser His Gly Gly Leu Ser Glu
1               5


<210>  210
<211>  7
<212>  PRT
<213>  artificial

<220>
<223>  peptide

<400>  210

Gly Gln Ala Met Ser Gly Gly
1               5


<210>  211
<211>  7
<212>  PRT
<213>  artificial

<220>
<223>  peptide

<400>  211

Gly Thr Asn Ala Thr Ala Ser
1               5


<210>  212
<211>  7
<212>  PRT
<213>  artificial

<220>
<223>  peptide

<400>  212

Phe Thr Ala Ala Asn Asn Gln
1               5

<210>  213
<211>  7
<212>  PRT
<213>  artificial

<220>
<223>  peptide

<400>  213

Ser Ser Asn Ala Thr Ala Ser
1               5


<210>  214
<211>  7
<212>  PRT
<213>  artificial

<220>
<223>  peptide

<400>  214

Gly Ser Asn Thr Thr Ala Ser
1               5


<210>  215
<211>  7
<212>  PRT
<213>  artificial

<220>
<223>  peptide

<400>  215

Val Ser Pro Asn His Ala Ala
1               5


<210>  216
<211>  7
<212>  PRT
<213>  artificial

<220>
<223>  peptide

<400>  216

Gly Ser Asn Ala Ala Ala Ser
1               5


<210>  217
<211>  7
<212>  PRT
<213>  artificial

<220>
<223>  peptide

<400>  217

Asn Val Arg Pro His Pro Ala
1               5


<210>  218
<211>  7
<212>  PRT
<213>  artificial

<220>
<223>  peptide

<400>  218

Asn Ser Glu Arg Ser Val Gln
1               5


<210>  219
<211>  7
<212>  PRT
<213>  artificial

<220>
<223>  peptide

<400>  219

Thr Val Gly Arg Asp Ser Val
1               5


<210>  220
<211>  7
<212>  PRT
<213>  artificial

<220>
<223>  peptide

<400>  220

Pro Ser Asp Ser Thr Asp Arg
1               5


<210>  221
<211>  7
<212>  PRT
<213>  artificial

<220>
<223>  peptide

<400>  221

Pro Ala Asn Thr Pro Pro Arg
1               5

<210>  222
<211>  7
<212>  PRT
<213>  artificial

<220>
<223>  peptide

<400>  222

Gly Ala Asn Arg Pro Gly Leu
1               5


<210>  223
<211>  7
<212>  PRT
<213>  artificial

<220>
<223>  peptide

<400>  223

Thr Asn His Ala Gly Ala Ala
1               5


<210>  224
<211>  7
<212>  PRT
<213>  artificial

<220>
<223>  peptide

<400>  224

Ala Ser Thr Gly Pro Arg Thr
1               5


<210>  225
<211>  7
<212>  PRT
<213>  artificial

<220>
<223>  peptide

<400>  225

Ser Ser Tyr Ala Pro Ser Ser
1               5


<210>  226
<211>  7
<212>  PRT
<213>  artificial

<220>
<223>  peptide

<400>  226

Ala Gln Pro Pro Gln Pro Pro
1               5


<210>  227
<211>  7
<212>  PRT
<213>  artificial

<220>
<223>  peptide

<400>  227

Ala Pro Thr Gly Pro His Thr
1               5


<210>  228
<211>  7
<212>  PRT
<213>  artificial

<220>
<223>  peptide

<400>  228

Ala His Leu Ser Gly Thr Ser
1               5


<210>  229
<211>  7
<212>  PRT
<213>  artificial

<220>
<223>  peptide

<400>  229

Thr Arg Ser Gly Asp Ser Ser
1               5


<210>  230
<211>  7
<212>  PRT
<213>  artificial

<220>
<223>  peptide

<400>  230

Arg Thr Gly Arg Thr Ser Pro
1               5

<210>  231
<211>  7
<212>  PRT
<213>  artificial

<220>
<223>  peptide

<400>  231

Arg Thr Gly Arg Asp Ser Pro
1               5


<210>  232
<211>  7
<212>  PRT
<213>  artificial

<220>
<223>  peptide

<400>  232

Asp Asp Phe Gln Arg Ala Arg
1               5


<210>  233
<211>  7
<212>  PRT
<213>  artificial

<220>
<223>  peptide

<400>  233

Arg Asp Arg Ala Asp Ser Pro
1               5


<210>  234
<211>  7
<212>  PRT
<213>  artificial

<220>
<223>  peptide

<400>  234

Arg Asp Arg Ala Ile Ser Pro
1               5


<210>  235
<211>  7
<212>  PRT
<213>  artificial

<220>
<223>  peptide

<400>  235

Arg Asp Arg Arg Thr Ser Pro
1               5


<210>  236
<211>  7
<212>  PRT
<213>  artificial

<220>
<223>  peptide

<400>  236

Gly Ala Arg Arg Ser Asp Ser
1               5


<210>  237
<211>  7
<212>  PRT
<213>  artificial

<220>
<223>  peptide

<400>  237

Arg Thr Gly Gly Thr Ser Pro
1               5


<210>  238
<211>  7
<212>  PRT
<213>  artificial

<220>
<223>  peptide

<400>  238

Arg Thr Gly Ala Thr Ser Pro
1               5


<210>  239
<211>  7
<212>  PRT
<213>  artificial

<220>
<223>  peptide

<400>  239

Gly Ala His Arg Pro Asp Ser
1               5

<210>  240
<211>  7
<212>  PRT
<213>  artificial

<220>
<223>  peptide

<400>  240

Arg Arg Ser Gly Gly Thr Pro
1               5


<210>  241
<211>  7
<212>  PRT
<213>  artificial

<220>
<223>  peptide

<400>  241

Pro Pro Ser Ala Ala Ala His
1               5


<210>  242
<211>  7
<212>  PRT
<213>  artificial

<220>
<223>  peptide

<400>  242

Ser Ala His Arg Ser Asp Ser
1               5


<210>  243
<211>  7
<212>  PRT
<213>  artificial

<220>
<223>  peptide

<400>  243

Gly Ala His Arg Ser Asp Thr
1               5


<210>  244
<211>  7
<212>  PRT
<213>  artificial

<220>
<223>  peptide

<400>  244

Ala Gln Tyr Gly Ala Ala Val
1               5


<210>  245
<211>  7
<212>  PRT
<213>  artificial

<220>
<223>  peptide

<400>  245

Gly Ala His Arg Ser Asp Pro
1               5


<210>  246
<211>  7
<212>  PRT
<213>  artificial

<220>
<223>  peptide

<400>  246

Lys Gly Gly Arg Ser Ala Val
1               5


<210>  247
<211>  7
<212>  PRT
<213>  artificial

<220>
<223>  peptide

<400>  247

Pro Pro Ala Asp Ser Arg Val
1               5


<210>  248
<211>  7
<212>  PRT
<213>  artificial

<220>
<223>  peptide

<400>  248

Gly Ala Asn Arg Ser Asp Ser
1               5

<210> 249
<211> 7
<212> PRT
<213> artificial

<220>
<223> peptide

<400> 249

Arg Asp Arg Ala Thr Leu Pro
1               5


<210> 250
<211> 7
<212> PRT
<213> artificial

<220>
<223> peptide

<400> 250

Ser Ile Ser Ala Thr Ala Arg
1               5


<210> 251
<211> 7
<212> PRT
<213> artificial

<220>
<223> peptide

<400> 251

Thr Ala Asn Pro Gly Leu Arg
1               5


<210> 252
<211> 7
<212> PRT
<213> artificial

<220>
<223> peptide

<400> 252

Arg Gly Gly Ala Leu Gly Gly
1               5


<210> 253
<211> 7
<212> PRT
<213> artificial

<220>
<223>  peptide

<400>  253

Pro Pro Asp Ala Tyr Arg Met
1               5


<210>  254
<211>  7
<212>  PRT
<213>  artificial

<220>
<223>  peptide

<400>  254

Ser Arg Ala Ser Pro Val Ala
1               5


<210>  255
<211>  7
<212>  PRT
<213>  artificial

<220>
<223>  peptide

<400>  255

Arg Asp Arg Ala Thr Phe Pro
1               5


<210>  256
<211>  7
<212>  PRT
<213>  artificial

<220>
<223>  peptide

<400>  256

Arg Asp Arg Arg Asp Ser Pro
1               5


<210>  257
<211>  7
<212>  PRT
<213>  artificial

<220>
<223>  peptide

<400>  257

Ser Gly Pro Gly Gly Thr Pro
1               5

<210>  258
<211>  7
<212>  PRT
<213>  artificial

<220>
<223>  peptide

<400>  258

Arg Arg Pro Gly Gly Thr Pro
1               5


<210>  259
<211>  7
<212>  PRT
<213>  artificial

<220>
<223>  peptide

<400>  259

Met Arg Pro Gly Asn Ser Ile
1               5


<210>  260
<211>  7
<212>  PRT
<213>  artificial

<220>
<223>  peptide

<400>  260

Ala Gly Arg Ser Pro Val Ser
1               5


<210>  261
<211>  7
<212>  PRT
<213>  artificial

<220>
<223>  peptide

<400>  261

Ala Ala Arg Ser Tyr Ser Ser
1               5


<210>  262
<211>  7
<212>  PRT
<213>  artificial

<220>
<223>  peptide

<400>  262

Gln Thr Asn Pro Val Arg His
1               5


<210>  263
<211>  7
<212>  PRT
<213>  artificial

<220>
<223>  peptide

<400>  263

Glu Gly Gly Ala Leu Gly Gly
1               5


<210>  264
<211>  7
<212>  PRT
<213>  artificial

<220>
<223>  peptide

<400>  264

Leu Ala Ala Arg Asp Ala Arg
1               5


<210>  265
<211>  7
<212>  PRT
<213>  artificial

<220>
<223>  peptide

<400>  265

Met Pro Ser Gly Thr Thr Arg
1               5


<210>  266
<211>  7
<212>  PRT
<213>  artificial

<220>
<223>  peptide

<400>  266

Gly Ala His Arg Ser Gly Ser
1               5

<210>  267
<211>  7
<212>  PRT
<213>  artificial

<220>
<223>  peptide

<400>  267

Gly Thr His Arg Ser Asp Ser
1               5


<210>  268
<211>  7
<212>  PRT
<213>  artificial

<220>
<223>  peptide

<400>  268

Glu Gly Pro Gly Asp Ser Pro
1               5


<210>  269
<211>  7
<212>  PRT
<213>  artificial

<220>
<223>  peptide

<400>  269

Arg Asp Arg Ala Ser Ser Pro
1               5


<210>  270
<211>  7
<212>  PRT
<213>  artificial

<220>
<223>  peptide

<400>  270

Glu Gly Pro Gly Thr Ser Pro
1               5


<210>  271
<211>  7
<212>  PRT
<213>  artificial

<220>
<223>  peptide

<400>  271

Lys Gly Gly Ala Thr Ser Pro
1               5


<210>  272
<211>  7
<212>  PRT
<213>  artificial

<220>
<223>  peptide

<400>  272

Glu Asp Arg Ala Thr Ser Pro
1               5


<210>  273
<211>  7
<212>  PRT
<213>  artificial

<220>
<223>  peptide

<400>  273

Ser Arg Gly Ala Pro Thr Thr
1               5


<210>  274
<211>  7
<212>  PRT
<213>  artificial

<220>
<223>  peptide

<400>  274

Asp Ser Arg Leu Lys Gln Ser
1               5


<210>  275
<211>  7
<212>  PRT
<213>  artificial

<220>
<223>  peptide

<400>  275

Ala Asn Thr Val Leu Arg Pro
1               5

<210>  276
<211>  12
<212>  PRT
<213>  artificial

<220>
<223>  peptide

<400>  276

Ala Ala Ala Lys Gly Gly Ala Leu Gly Gly Ala Ala
1               5                   10


<210>  277
<211>  12
<212>  PRT
<213>  artificial

<220>
<223>  peptide

<400>  277

Ala Ala Ala Ala Ser Thr Gly Pro His Thr Ala Ala
1               5                   10


<210>  278
<211>  12
<212>  PRT
<213>  artificial

<220>
<223>  peptide

<400>  278

Ala Ser Ala Thr Ser Pro Ser Pro Ser Arg Ala Ala
1               5                   10


<210>  279
<211>  12
<212>  PRT
<213>  artificial

<220>
<223>  peptide

<400>  279

Ala Ser Ala Asn Asn Pro Gly Ala Pro Arg Ala Ala
1               5                   10


<210>  280
<211>  12
<212>  PRT
<213>  artificial

<220>
<223>  peptide

<400>  280

Ala Ser Ala Gly Val Ser Pro Glu Arg Arg Ala Ala
1               5                   10


<210>  281
<211>  12
<212>  PRT
<213>  artificial

<220>
<223>  peptide

<400>  281

Ala Ser Ala Trp His Ala Pro Thr His Gly Ala Ala
1               5                   10


<210>  282
<211>  12
<212>  PRT
<213>  artificial

<220>
<223>  peptide

<400>  282

Ala Ser Ala Asn Ala Gln Pro Pro Ser Arg Ala Ala
1               5                   10


<210>  283
<211>  12
<212>  PRT
<213>  artificial

<220>
<223>  peptide

<400>  283

Ala Ser Ala His Ala Ala Gly Leu Pro Lys Ala Ala
1               5                   10


<210>  284
<211>  12
<212>  PRT
<213>  artificial

<220>
<223>  peptide

<400>  284

Ala Ala Ala Pro Ala Asn Gln Ser Val Arg Ala Ala
1               5                   10

<210>  285
<211>  12
<212>  PRT
<213>  artificial

<220>
<223>  peptide

<400>  285

Ala Ala Ala Gly Ser Asn Ala Thr Ala Ser Ala Ala
1               5                   10


<210>  286
<211>  12
<212>  PRT
<213>  artificial

<220>
<223>  peptide

<400>  286

Ala Ala Ala Gly Ala His Arg Ser Asp Ser Ala Ala
1               5                   10


<210>  287
<211>  12
<212>  PRT
<213>  artificial

<220>
<223>  peptide

<400>  287

Ala Ala Ala Ala Asn Tyr Gln Gly Pro Ala Ala Ala
1               5                   10


<210>  288
<211>  12
<212>  PRT
<213>  artificial

<220>
<223>  peptide

<400>  288

Ala Ala Ala Asn His Thr Ala Ala Thr Trp Ala Ala
1               5                   10


<210>  289
<211>  12
<212>  PRT
<213>  artificial

<220>
<223>  peptide

<400>  289

Ala Ala Ala Thr Asn His Ala Ser Ala Ala Ala Ala
1               5                   10


<210>  290
<211>  12
<212>  PRT
<213>  artificial

<220>
<223>  peptide

<400>  290

Ala Ala Ala Asn Tyr Pro Gly Ala His Ser Ala Ala
1               5                   10


<210>  291
<211>  12
<212>  PRT
<213>  artificial

<220>
<223>  peptide

<400>  291

Ala Ala Ala Ile Gly Thr Asn Ser Pro Val Ala Ala
1               5                   10


<210>  292
<211>  12
<212>  PRT
<213>  artificial

<220>
<223>  peptide

<400>  292

Ala Ala Ala Ala Gln Tyr Ser Ala Ala Val Ala Ala
1               5                   10


<210>  293
<211>  12
<212>  PRT
<213>  artificial

<220>
<223>  peptide

<400>  293

Ala Ala Ala Ser Tyr Gly Gln Ser Ala Gly Ala Ala
1               5                   10

<210>  294
<211>  12
<212>  PRT
<213>  artificial

<220>
<223>  peptide

<400>  294

Ala Ala Ala Ala His Gly Gly Ser Ile Pro Ala Ala
1               5                   10


<210>  295
<211>  12
<212>  PRT
<213>  artificial

<220>
<223>  peptide

<400>  295

Ala Ala Ala Tyr Gln Ser Gly Ser Leu Pro Ala Ala
1               5                   10


<210>  296
<211>  12
<212>  PRT
<213>  artificial

<220>
<223>  peptide

<400>  296

Ala Ala Ala Asp Ser Gln His Thr Gln His Ala Ala
1               5                   10


<210>  297
<211>  12
<212>  PRT
<213>  artificial

<220>
<223>  peptide

<400>  297

Ala Ala Ala Gly Ser Asp Arg Val Ser Thr Ala Ala
1               5                   10


<210>  298
<211>  12
<212>  PRT
<213>  artificial

<220>
<223>  peptide

<400>  298

Ala Ala Ala Thr Ser Asn Arg Ala Ala Thr Ala Ala
1               5                   10


<210>  299
<211>  12
<212>  PRT
<213>  artificial

<220>
<223>  peptide

<400>  299

Ala Ala Ala Gly Gly Thr Thr Leu Ala His Ala Ala
1               5                   10


<210>  300
<211>  12
<212>  PRT
<213>  artificial

<220>
<223>  peptide

<400>  300

Ala Ala Ala Ser Ser Pro Asn His Val Ala Ala Ala
1               5                   10


<210>  301
<211>  12
<212>  PRT
<213>  artificial

<220>
<223>  peptide

<400>  301

Ala Ala Ala Gly Asn Ala Arg Pro Pro Asp Ala Ala
1               5                   10


<210>  302
<211>  12
<212>  PRT
<213>  artificial

<220>
<223>  peptide

<400>  302

Ala Ala Ala Pro Gly Gln Thr Arg Asp Ser Ala Ala
1               5                   10

<210>  303
<211>  12
<212>  PRT
<213>  artificial

<220>
<223>  peptide

<400>  303

Ala Ala Ala Glu Asn Ala Thr Leu Arg Thr Ala Ala
1               5                   10


<210>  304
<211>  12
<212>  PRT
<213>  artificial

<220>
<223>  peptide

<400>  304

Ala Ala Ala His Thr Arg Thr Ser Gln Glu Ala Ala
1               5                   10


<210>  305
<211>  12
<212>  PRT
<213>  artificial

<220>
<223>  peptide

<400>  305

Ala Ala Ala Pro Val Gly Ser Pro Ser Arg Ala Ala
1               5                   10


<210>  306
<211>  12
<212>  PRT
<213>  artificial

<220>
<223>  peptide

<400>  306

Ala Ala Ala Ser Ser Gly Thr Thr Arg Asp Ala Ala
1               5                   10


<210>  307
<211>  12
<212>  PRT
<213>  artificial

<220>
<223>  peptide

<400>  307

Ala Ala Ala Asp Asp Gly Arg Pro Ser Arg Ala Ala
1               5                   10


<210>  308
<211>  12
<212>  PRT
<213>  artificial

<220>
<223>  peptide

<400>  308

Ala Ala Ala Ala Ala Tyr Gly Ala Gln Ser Ala Ala
1               5                   10


<210>  309
<211>  12
<212>  PRT
<213>  artificial

<220>
<223>  peptide

<400>  309

Ala Ala Ala Arg Glu Gly Ser Pro Gly Arg Ala Ala
1               5                   10


<210>  310
<211>  12
<212>  PRT
<213>  artificial

<220>
<223>  peptide

<400>  310

Ala Ala Ala Asn Lys Thr Gly Ala Ser Pro Ala Ala
1               5                   10


<210>  311
<211>  12
<212>  PRT
<213>  artificial

<220>
<223>  peptide

<400>  311

Ala Ala Ala Lys Ala Ser Gly Gln Thr Val Ala Ala
1               5                   10

<210>  312
<211>  12
<212>  PRT
<213>  artificial

<220>
<223>  peptide

<400>  312

Ala Ala Ala Arg Asp Thr Thr Thr Gly Ala Ala Ala
1               5                   10


<210>  313
<211>  12
<212>  PRT
<213>  artificial

<220>
<223>  peptide

<400>  313

Ala Ala Ala Ala Tyr Gly Pro Asn Ala Gly Ala Ala
1               5                   10


<210>  314
<211>  12
<212>  PRT
<213>  artificial

<220>
<223>  peptide

<400>  314

Ala Ala Ala Asp Ser Asp Gly Pro Arg Pro Ala Ala
1               5                   10


<210>  315
<211>  12
<212>  PRT
<213>  artificial

<220>
<223>  peptide

<400>  315

Ala Ala Ala Gly Ser Ser Ala Thr Ala Ser Ala Ala
1               5                   10


<210>  316
<211>  12
<212>  PRT
<213>  artificial

<220>
<223>  peptide

<400>  316

Ala Ala Ala Thr Ser Ala Gly Ser Pro Arg Ala Ala
1               5                   10


<210>  317
<211>  12
<212>  PRT
<213>  artificial

<220>
<223>  peptide

<400>  317

Ala Ala Ala Gly Pro Ala His Thr Ser Ser Ala Ala
1               5                   10


<210>  318
<211>  12
<212>  PRT
<213>  artificial

<220>
<223>  peptide

<400>  318

Ala Ala Ala Asn Ala Ala Gly Thr Pro Arg Ala Ala
1               5                   10


<210>  319
<211>  12
<212>  PRT
<213>  artificial

<220>
<223>  peptide

<400>  319

Ala Ala Ala Asn Arg Thr Ala Leu Gly Ser Ala Ala
1               5                   10


<210>  320
<211>  12
<212>  PRT
<213>  artificial

<220>
<223>  peptide

<400>  320

Ala Ala Ala Lys Ser His Ser Ala Ser Pro Ala Ala
1               5                   10

<210>  321
<211>  12
<212>  PRT
<213>  artificial

<220>
<223>  peptide

<400>  321

Ala Ala Ala Arg Gly His Thr Ala Pro Ile Ala Ala
1               5                   10


<210>  322
<211>  12
<212>  PRT
<213>  artificial

<220>
<223>  peptide

<400>  322

Ala Ala Ala Pro Thr Ser Gly Ala Pro Lys Ala Ala
1               5                   10


<210>  323
<211>  12
<212>  PRT
<213>  artificial

<220>
<223>  peptide

<400>  323

Ala Ala Ala Gly Asn Arg Val Gln Ser Thr Ala Ala
1               5                   10


<210>  324
<211>  12
<212>  PRT
<213>  artificial

<220>
<223>  peptide

<400>  324

Ala Ala Ala Asn Thr Ser Leu Thr Ala Arg Ala Ala
1               5                   10


<210>  325
<211>  12
<212>  PRT
<213>  artificial

<220>
<223>  peptide

<400>  325

Ala Ala Ala Arg Asp Arg Ala Thr Ser Pro Ala Ala
1               5                   10


<210>  326
<211>  12
<212>  PRT
<213>  artificial

<220>
<223>  peptide

<400>  326

Ala Ala Ala Gly Ala Glu Arg Thr Tyr Ser Ala Ala
1               5                   10


<210>  327
<211>  12
<212>  PRT
<213>  artificial

<220>
<223>  peptide

<400>  327

Ala Ala Ala Ala Arg Gly Ser Val Ser Pro Ala Ala
1               5                   10


<210>  328
<211>  12
<212>  PRT
<213>  artificial

<220>
<223>  peptide

<400>  328

Ala Ala Ala Thr Gly Arg Ser Ser Gly Asp Ala Ala
1               5                   10


<210>  329
<211>  12
<212>  PRT
<213>  artificial

<220>
<223>  peptide

<400>  329

Ala Ala Ala His Pro Ala Arg Pro Asn Ala Ala Ala
1               5                   10

<210>  330
<211>  12
<212>  PRT
<213>  artificial

<220>
<223>  peptide

<400>  330

Ala Ala Ala Ser Asp His Leu His Pro Arg Ala Ala
1               5                   10


<210>  331
<211>  12
<212>  PRT
<213>  artificial

<220>
<223>  peptide

<400>  331

Ala Ala Ala Thr Val Leu Ser Ser Gln Arg Ala Ala
1               5                   10


<210>  332
<211>  12
<212>  PRT
<213>  artificial

<220>
<223>  peptide

<400>  332

Ala Ala Ala Val Ser Ser Ser Ser Arg Pro Ala Ala
1               5                   10


<210>  333
<211>  12
<212>  PRT
<213>  artificial

<220>
<223>  peptide

<400>  333

Ala Ala Ala Thr Gly Ser Ser Lys Gly Val Ala Ala
1               5                   10


<210>  334
<211>  12
<212>  PRT
<213>  artificial

<220>
<223>  peptide

<400>  334

Ala Ala Ala Ser Pro Gly Gly Pro Ala Arg Ala Ala
1               5                   10


<210>  335
<211>  12
<212>  PRT
<213>  artificial

<220>
<223>  peptide

<400>  335

Ala Ala Ala Gly Arg Ser Glu Gln Ile Arg Ala Ala
1               5                   10


<210>  336
<211>  12
<212>  PRT
<213>  artificial

<220>
<223>  peptide

<400>  336

Ala Ala Ala Val Thr Ala Arg Pro Glu Gly Ala Ala
1               5                   10


<210>  337
<211>  12
<212>  PRT
<213>  artificial

<220>
<223>  peptide

<400>  337

Ala Ala Ala Arg Asp Pro Ser Pro Gly Arg Ala Ala
1               5                   10


<210>  338
<211>  12
<212>  PRT
<213>  artificial

<220>
<223>  peptide

<400>  338

Ala Ala Ala Ala Ala Ser Gly Val Ala Arg Ala Ala
1               5                   10

<210>  339
<211>  12
<212>  PRT
<213>  artificial

<220>
<223>  peptide

<400>  339

Ala Ala Ala Thr Ser Ala Arg Ser Val Pro Ala Ala
1               5                   10


<210>  340
<211>  12
<212>  PRT
<213>  artificial

<220>
<223>  peptide

<400>  340

Ala Ala Ala Ala Arg Gly Gly Ser Ile Pro Ala Ala
1               5                   10


<210>  341
<211>  12
<212>  PRT
<213>  artificial

<220>
<223>  peptide

<400>  341

Ala Ala Ala Arg Ala Ala Gly Gln Val Ala Ala Ala
1               5                   10


<210>  342
<211>  12
<212>  PRT
<213>  artificial

<220>
<223>  peptide

<400>  342

Ala Ala Ala Thr Ser Gln Gln Arg Ile Pro Ala Ala
1               5                   10


<210>  343
<211>  12
<212>  PRT
<213>  artificial

<220>
<223>  peptide

<400>  343

Ala Ala Ala Asn Ser Ala Arg Pro Asn Ser Ala Ala
1               5                   10


<210>  344
<211>  12
<212>  PRT
<213>  artificial

<220>
<223>  peptide

<400>  344

Ala Ala Ala Pro Val Gly Ser Pro Gly Arg Ala Ala
1               5                   10


<210>  345
<211>  12
<212>  PRT
<213>  artificial

<220>
<223>  peptide

<400>  345

Ala Ala Ala Thr Gln Ser Pro Pro Gly Arg Ala Ala
1               5                   10


<210>  346
<211>  12
<212>  PRT
<213>  artificial

<220>
<223>  peptide

<400>  346

Ala Ala Ala Gly Arg Arg Asp Ser Pro Val Ala Ala
1               5                   10


<210>  347
<211>  12
<212>  PRT
<213>  artificial

<220>
<223>  peptide

<400>  347

Ala Ala Ala Asn Gly Ser Pro Val Gly Arg Ala Ala
1               5                   10

<210>  348
<211>  12
<212>  PRT
<213>  artificial

<220>
<223>  peptide

<400>  348

Ala Ala Ala Lys Val Ser Gly His Gln Ala Ala Ala
1               5                   10


<210>  349
<211>  12
<212>  PRT
<213>  artificial

<220>
<223>  peptide

<400>  349

Ala Ala Ala Ala Arg Ser Gln Val His Ala Ala Ala
1               5                   10


<210>  350
<211>  12
<212>  PRT
<213>  artificial

<220>
<223>  peptide

<400>  350

Ala Ala Ala Ala Arg Thr Thr His Ser Pro Ala Ala
1               5                   10


<210>  351
<211>  12
<212>  PRT
<213>  artificial

<220>
<223>  peptide

<400>  351

Ala Ala Ala Asn Leu Ser Arg Ala Ser Pro Ala Ala
1               5                   10


<210>  352
<211>  12
<212>  PRT
<213>  artificial

<220>
<223>  peptide

<400>  352

Ala Ala Ala Asn Thr Pro Gln Ser Ala Arg Ala Ala
1               5                   10


<210>  353
<211>  12
<212>  PRT
<213>  artificial

<220>
<223>  peptide

<400>  353

Ala Ala Ala Gly Arg Pro Gly Ser Gly Tyr Ala Ala
1               5                   10


<210>  354
<211>  12
<212>  PRT
<213>  artificial

<220>
<223>  peptide

<400>  354

Ala Ala Ala Val Pro Ser Ala Pro Gln Arg Ala Ala
1               5                   10


<210>  355
<211>  12
<212>  PRT
<213>  artificial

<220>
<223>  peptide

<400>  355

Ala Ala Ala Ala Pro Ala Asn Leu Pro Arg Ala Ala
1               5                   10


<210>  356
<211>  12
<212>  PRT
<213>  artificial

<220>
<223>  peptide

<400>  356

Ala Ala Ala Leu Gly Arg Pro Ala Gly Pro Ala Ala
1               5                   10

<210>  357
<211>  12
<212>  PRT
<213>  artificial

<220>
<223>  peptide

<400>  357

Ala Ala Ala Val Leu Gly Thr Lys Gly Ala Ala Ala
1               5                   10


<210>  358
<211>  12
<212>  PRT
<213>  artificial

<220>
<223>  peptide

<400>  358

Ala Ala Ala Gly Arg Pro Glu Gln Ile Arg Ala Ala
1               5                   10


<210>  359
<211>  12
<212>  PRT
<213>  artificial

<220>
<223>  peptide

<400>  359

Ala Ala Ala Gly Leu Ala Gly Val Val Arg Ala Ala
1               5                   10


<210>  360
<211>  12
<212>  PRT
<213>  artificial

<220>
<223>  peptide

<400>  360

Ala Ala Ala Ala His Arg Ser Thr Gly Thr Ala Ala
1               5                   10


<210>  361
<211>  12
<212>  PRT
<213>  artificial

<220>
<223>  peptide

<400>  361

Ala Ala Ala Gly Thr Ser Pro Ala Asn Arg Ala Ala
1               5                   10


<210>  362
<211>  12
<212>  PRT
<213>  artificial

<220>
<223>  peptide

<400>  362

Ala Ala Ala Asn Arg Pro Asn Ala Ala Ser Ala Ala
1               5                   10


<210>  363
<211>  12
<212>  PRT
<213>  artificial

<220>
<223>  peptide

<400>  363

Ala Ala Ala Ala Arg Ser Gly Ser Gly Ala Ala Ala
1               5                   10


<210>  364
<211>  12
<212>  PRT
<213>  artificial

<220>
<223>  peptide

<400>  364

Ala Ala Ala Arg His Ser Ser Thr Pro Val Ala Ala
1               5                   10


<210>  365
<211>  12
<212>  PRT
<213>  artificial

<220>
<223>  peptide

<400>  365

Ala Ala Ala Asn Ser Pro Leu Gly Thr Arg Ala Ala
1               5                   10

<210>  366
<211>  12
<212>  PRT
<213>  artificial

<220>
<223>  peptide

<400>  366

Ala Ala Ala Asn Thr Pro Ser Pro Ala Arg Ala Ala
1               5                   10


<210>  367
<211>  12
<212>  PRT
<213>  artificial

<220>
<223>  peptide

<400>  367

Ala Ala Ala Asn Ser Pro Ala Ala Gly Arg Ala Ala
1               5                   10


<210>  368
<211>  12
<212>  PRT
<213>  artificial

<220>
<223>  peptide

<400>  368

Ala Ala Ala Ser Asn Arg Thr Ser Ser Val Ala Ala
1               5                   10


<210>  369
<211>  12
<212>  PRT
<213>  artificial

<220>
<223>  peptide

<400>  369

Ala Ala Ala Ser Val Arg Gly Gly Thr Pro Ala Ala
1               5                   10


<210>  370
<211>  12
<212>  PRT
<213>  artificial

<220>
<223>  peptide

<400>  370

Ala Ala Ala Ser Pro Ala Asp Ser Arg Val Ala Ala
1               5                   10


<210>  371
<211>  12
<212>  PRT
<213>  artificial

<220>
<223>  peptide

<400>  371

Ala Ala Ala Ser Thr Arg Gly Gln Gly His Ala Ala
1               5                   10


<210>  372
<211>  12
<212>  PRT
<213>  artificial

<220>
<223>  peptide

<400>  372

Ala Ala Ala Val Asp Ser Gly Leu Ser Gly Ala Ala
1               5                   10


<210>  373
<211>  12
<212>  PRT
<213>  artificial

<220>
<223>  peptide

<400>  373

Ala Ala Ala Gly Asp Gly Ser Phe Ser Gly Ala Ala
1               5                   10


<210>  374
<211>  12
<212>  PRT
<213>  artificial

<220>
<223>  peptide

<400>  374

Ala Ala Ala Val Ser Gly Pro Pro His Thr Ala Ala
1               5                   10

<210>  375
<211>  12
<212>  PRT
<213>  artificial

<220>
<223>  peptide

<400>  375

Ala Ala Ala Arg His Gly Pro Gln His Pro Ala Ala
1               5                   10


<210>  376
<211>  12
<212>  PRT
<213>  artificial

<220>
<223>  peptide

<400>  376

Ala Ala Ala Arg Asp Arg Ala Ala Ser Pro Ala Ala
1               5                   10


<210>  377
<211>  12
<212>  PRT
<213>  artificial

<220>
<223>  peptide

<400>  377

Ala Ala Ala Gly Asp Arg Ala Thr Ser Pro Ala Ala
1               5                   10


<210>  378
<211>  12
<212>  PRT
<213>  artificial

<220>
<223>  peptide

<400>  378

Ala Ala Ala Arg Gly Arg Ala Thr Ser Pro Ala Ala
1               5                   10


<210>  379
<211>  12
<212>  PRT
<213>  artificial

<220>
<223>  peptide

<400>  379

Ala Ala Ala Arg Asp Arg Val Thr Ser Pro Ala Ala
1               5                   10


<210>  380
<211>  12
<212>  PRT
<213>  artificial

<220>
<223>  peptide

<400>  380

Ala Ala Ala Pro Val Leu Asn Pro Pro Pro Ala Ala
1               5                   10


<210>  381
<211>  12
<212>  PRT
<213>  artificial

<220>
<223>  peptide

<400>  381

Ala Ala Ala Ser Phe Gln Arg Ala Ser Pro Ala Ala
1               5                   10


<210>  382
<211>  12
<212>  PRT
<213>  artificial

<220>
<223>  peptide

<400>  382

Ala Ala Ala Arg Asp Arg Ala Thr Thr Pro Ala Ala
1               5                   10


<210>  383
<211>  12
<212>  PRT
<213>  artificial

<220>
<223>  peptide

<400>  383

Ala Ala Ala Gln Ser Arg Leu Ser Thr Gln Ala Ala
1               5                   10

<210>  384
<211>  12
<212>  PRT
<213>  artificial

<220>
<223>  peptide

<400>  384

Ala Ala Ala Ser Ser Gly Gly Gly Thr Pro Ala Ala
1               5                   10


<210>  385
<211>  12
<212>  PRT
<213>  artificial

<220>
<223>  peptide

<400>  385

Ala Ala Ala Lys Gly Glu Ala Leu Gly Gly Ala Ala
1               5                   10


<210>  386
<211>  12
<212>  PRT
<213>  artificial

<220>
<223>  peptide

<400>  386

Ala Ala Ala Ser Val Arg Gly Ala Pro Pro Ala Ala
1               5                   10


<210>  387
<211>  12
<212>  PRT
<213>  artificial

<220>
<223>  peptide

<400>  387

Ala Ala Ala Asp His Asp Ser Pro Ser Val Ala Ala
1               5                   10


<210>  388
<211>  12
<212>  PRT
<213>  artificial

<220>
<223>  peptide

<400>  388

Ala Ala Ala Arg Asp Gln Ala Thr Ser Pro Ala Ala
1               5                   10


<210>  389
<211>  12
<212>  PRT
<213>  artificial

<220>
<223>  peptide

<400>  389

Ala Ala Ala Arg Asp Arg Ala Thr Ser Ser Ala Ala
1               5                   10


<210>  390
<211>  12
<212>  PRT
<213>  artificial

<220>
<223>  peptide

<400>  390

Ala Ala Ala Gly Lys Pro Pro Arg Thr Glu Ala Ala
1               5                   10


<210>  391
<211>  12
<212>  PRT
<213>  artificial

<220>
<223>  peptide

<400>  391

Ala Ala Ala Ala His Arg Gly Leu Gln Val Ala Ala
1               5                   10


<210>  392
<211>  12
<212>  PRT
<213>  artificial

<220>
<223>  peptide

<400>  392

Ala Ala Ala Val Ala Ser Leu Ala Ala Arg Ala Ala
1               5                   10

<210>  393
<211>  12
<212>  PRT
<213>  artificial

<220>
<223>  peptide

<400>  393

Ala Ala Ala Arg Asp Arg Thr Thr Ser Pro Ala Ala
1               5                   10


<210>  394
<211>  12
<212>  PRT
<213>  artificial

<220>
<223>  peptide

<400>  394

Ala Ala Ala Arg Asp Trp Ala Thr Ser Pro Ala Ala
1               5                   10


<210>  395
<211>  12
<212>  PRT
<213>  artificial

<220>
<223>  peptide

<400>  395

Ala Ala Ala Arg Asn Arg Ala Thr Ser Pro Ala Ala
1               5                   10


<210>  396
<211>  12
<212>  PRT
<213>  artificial

<220>
<223>  peptide

<400>  396

Ala Ala Ala Arg Asp Arg Ala Thr Ser Leu Ala Ala
1               5                   10


<210>  397
<211>  12
<212>  PRT
<213>  artificial

<220>
<223>  peptide

<400>  397

Ala Ala Ala Lys Asp Arg Ala Thr Ser Pro Ala Ala
1               5                   10


<210>  398
<211>  12
<212>  PRT
<213>  artificial

<220>
<223>  peptide

<400>  398

Ala Ala Ala Ala Arg Tyr Ser Ala Ala Val Ala Ala
1               5                   10


<210>  399
<211>  12
<212>  PRT
<213>  artificial

<220>
<223>  peptide

<400>  399

Ala Ala Ala Gly Ala His Arg Ser Val Ser Ala Ala
1               5                   10


<210>  400
<211>  12
<212>  PRT
<213>  artificial

<220>
<223>  peptide

<400>  400

Ala Ala Ala Trp Asp Arg Ala Thr Ser Pro Ala Ala
1               5                   10


<210>  401
<211>  12
<212>  PRT
<213>  artificial

<220>
<223>  peptide

<400>  401

Ala Ala Ala Glu Arg Pro Tyr Gln Ala Gly Ala Ala
1               5                   10

<210>  402
<211>  12
<212>  PRT
<213>  artificial

<220>
<223>  peptide

<400>  402

Ala Ala Ala Asn Ala Pro Gly Asn Leu Arg Ala Ala
1               5                   10


<210>  403
<211>  12
<212>  PRT
<213>  artificial

<220>
<223>  peptide

<400>  403

Ala Ala Ala Asn Asn Pro Leu Pro Gln Arg Ala Ala
1               5                   10


<210>  404
<211>  12
<212>  PRT
<213>  artificial

<220>
<223>  peptide

<400>  404

Ala Ala Ala Ala Arg Pro Thr Val Pro Tyr Ala Ala
1               5                   10


<210>  405
<211>  12
<212>  PRT
<213>  artificial

<220>
<223>  peptide

<400>  405

Ala Ala Ala Arg Ser Gly Ala Ala Val Ala Ala Ala
1               5                   10


<210>  406
<211>  12
<212>  PRT
<213>  artificial

<220>
<223>  peptide

<400>  406

Ala Ala Ala Asn Thr Pro Asn Pro Leu Arg Ala Ala
1               5                   10


<210>  407
<211>  12
<212>  PRT
<213>  artificial

<220>
<223>  peptide

<400>  407

Ala Ala Ala Gly Gly Thr Thr Leu Gly Arg Ala Ala
1               5                   10


<210>  408
<211>  12
<212>  PRT
<213>  artificial

<220>
<223>  peptide

<400>  408

Ala Ala Ala Ala Ser Thr Gly Leu Pro Arg Ala Ala
1               5                   10


<210>  409
<211>  12
<212>  PRT
<213>  artificial

<220>
<223>  peptide

<400>  409

Ala Ala Ala Ala Thr Gly Val Ile Gln Arg Ala Ala
1               5                   10


<210>  410
<211>  12
<212>  PRT
<213>  artificial

<220>
<223>  peptide

<400>  410

Ala Ala Ala Asn Ser Ala Ala Leu Gly Arg Ala Ala
1               5                   10

<210>  411
<211>  12
<212>  PRT
<213>  artificial

<220>
<223>  peptide

<400>  411

Ala Ala Ala Gly Glu Pro Gln Pro Arg Phe Ala Ala
1               5                   10


<210>  412
<211>  12
<212>  PRT
<213>  artificial

<220>
<223>  peptide

<400>  412

Ala Ala Ala Gly His Ser Ala Arg Ser Ile Ala Ala
1               5                   10


<210>  413
<211>  12
<212>  PRT
<213>  artificial

<220>
<223>  peptide

<400>  413

Ala Ala Ala Val Ser Ser Ser Thr Pro Arg Ala Ala
1               5                   10


<210>  414
<211>  12
<212>  PRT
<213>  artificial

<220>
<223>  peptide

<400>  414

Ala Ala Ala Asn Gln Pro Asn Pro Val Arg Ala Ala
1               5                   10


<210>  415
<211>  12
<212>  PRT
<213>  artificial

<220>
<223>  peptide

<400>  415

Ala Ala Ala Pro Ser Gly Gly Pro Pro Arg Ala Ala
1               5                   10


<210>  416
<211>  12
<212>  PRT
<213>  artificial

<220>
<223>  peptide

<400>  416

Ala Ala Ala Ala Arg Asp Gln Leu Pro Arg Ala Ala
1               5                   10


<210>  417
<211>  12
<212>  PRT
<213>  artificial

<220>
<223>  peptide

<400>  417

Ala Ala Ala Arg Gly Ser His Pro Asn Ile Ala Ala
1               5                   10


<210>  418
<211>  12
<212>  PRT
<213>  artificial

<220>
<223>  peptide

<400>  418

Ala Ala Ala Asn Arg Ala Ser Asn Thr Ser Ala Ala
1               5                   10


<210>  419
<211>  12
<212>  PRT
<213>  artificial

<220>
<223>  peptide

<400>  419

Ala Ala Ala Val Asn Ala Val Pro Gly Arg Ala Ala
1               5                   10

<210>  420
<211>  12
<212>  PRT
<213>  artificial

<220>
<223>  peptide

<400>  420

Ala Ala Ala Pro Ala Asn Thr Pro Gly Arg Ala Ala
1               5                   10


<210>  421
<211>  12
<212>  PRT
<213>  artificial

<220>
<223>  peptide

<400>  421

Ala Ala Ala Ile Ser Ser Ser Thr Ala Arg Ala Ala
1               5                   10


<210>  422
<211>  12
<212>  PRT
<213>  artificial

<220>
<223>  peptide

<400>  422

Ala Ala Ala Ala Arg Ala Gly Arg Asp Leu Ala Ala
1               5                   10


<210>  423
<211>  12
<212>  PRT
<213>  artificial

<220>
<223>  peptide

<400>  423

Ala Ala Ala Arg Gly Gly Glu Val Pro Arg Ala Ala
1               5                   10


<210>  424
<211>  12
<212>  PRT
<213>  artificial

<220>
<223>  peptide

<400>  424

Ala Ala Ala Val Asn Leu Val Pro Ser Arg Ala Ala
1               5                   10


<210>  425
<211>  12
<212>  PRT
<213>  artificial

<220>
<223>  peptide

<400>  425

Ala Ala Ala Leu Ala Arg Asn Gln Ser Gly Ala Ala
1               5                   10


<210>  426
<211>  12
<212>  PRT
<213>  artificial

<220>
<223>  peptide

<400>  426

Ala Ala Ala Ser Ser Ser Gly Ser Pro Arg Ala Ala
1               5                   10


<210>  427
<211>  12
<212>  PRT
<213>  artificial

<220>
<223>  peptide

<400>  427

Ala Ala Ala Ser Pro Gly Ile Asn Leu Arg Ala Ala
1               5                   10


<210>  428
<211>  12
<212>  PRT
<213>  artificial

<220>
<223>  peptide

<400>  428

Ala Ala Ala Ala Val Ala Gly Ser Pro Arg Ala Ala
1               5                   10

<210>  429
<211>  12
<212>  PRT
<213>  artificial

<220>
<223>  peptide

<400>  429

Ala Ala Ala Arg Ser Pro Asp Ser Ala Arg Ala Ala
1               5                   10


<210>  430
<211>  12
<212>  PRT
<213>  artificial

<220>
<223>  peptide

<400>  430

Ala Ala Ala Asn Gln Pro Val Pro Asn Arg Ala Ala
1               5                   10


<210>  431
<211>  12
<212>  PRT
<213>  artificial

<220>
<223>  peptide

<400>  431

Ala Ala Ala Pro Ser Ser Pro Val Ala Arg Ala Ala
1               5                   10


<210>  432
<211>  12
<212>  PRT
<213>  artificial

<220>
<223>  peptide

<400>  432

Ala Ala Ala Lys Asp Ala Gln Leu Gly Arg Ala Ala
1               5                   10


<210>  433
<211>  12
<212>  PRT
<213>  artificial

<220>
<223>  peptide

<400>  433

Ala Ala Ala Val Thr Ser Ser Ser Gly Lys Ala Ala
1               5                   10


<210>  434
<211>  12
<212>  PRT
<213>  artificial

<220>
<223>  peptide

<400>  434

Ala Ala Ala Ala Ala Asn Met Thr Ala Arg Ala Ala
1               5                   10


<210>  435
<211>  12
<212>  PRT
<213>  artificial

<220>
<223>  peptide

<400>  435

Ala Ala Ala Ser Ser Ile Asn Pro Pro Arg Ala Ala
1               5                   10


<210>  436
<211>  12
<212>  PRT
<213>  artificial

<220>
<223>  peptide

<400>  436

Ala Ala Ala Ala Tyr Ala Gly Thr Pro Lys Ala Ala
1               5                   10


<210>  437
<211>  12
<212>  PRT
<213>  artificial

<220>
<223>  peptide

<400>  437

Ala Ala Ala Arg Gly Ser Ser Pro Val Arg Ala Ala
1               5                   10

<210>  438
<211>  12
<212>  PRT
<213>  artificial

<220>
<223>  peptide

<400>  438

Ala Ala Ala Gly Gly Gly Leu Thr Pro Arg Ala Ala
1               5                   10


<210>  439
<211>  12
<212>  PRT
<213>  artificial

<220>
<223>  peptide

<400>  439

Ala Ala Ala Gly Ala Ser Ser Val Ser Arg Ala Ala
1               5                   10


<210>  440
<211>  12
<212>  PRT
<213>  artificial

<220>
<223>  peptide

<400>  440

Ala Ala Ala Val His Leu Ser Pro Gly Arg Ala Ala
1               5                   10


<210>  441
<211>  12
<212>  PRT
<213>  artificial

<220>
<223>  peptide

<400>  441

Ala Ala Ala Asn Ser Pro Asn Val Ala Arg Ala Ala
1               5                   10


<210>  442
<211>  12
<212>  PRT
<213>  artificial

<220>
<223>  peptide

<400>  442

Ala Ala Ala Gly Gly Arg Asp Gln Leu Arg Ala Ala
1               5                   10


<210>  443
<211>  12
<212>  PRT
<213>  artificial

<220>
<223>  peptide

<400>  443

Ala Ala Ala Ala Ala Gln Gly Met Pro Lys Ala Ala
1               5                   10


<210>  444
<211>  12
<212>  PRT
<213>  artificial

<220>
<223>  peptide

<400>  444

Ala Ala Ala Arg Arg Asp Leu Asp Val Arg Ala Ala
1               5                   10


<210>  445
<211>  12
<212>  PRT
<213>  artificial

<220>
<223>  peptide

<400>  445

Ala Ala Ala Val Thr Ser Thr Ser Ala Arg Ala Ala
1               5                   10


<210>  446
<211>  12
<212>  PRT
<213>  artificial

<220>
<223>  peptide

<400>  446

Ala Ala Ala Arg His Gly Ser Pro Gly Ser Ala Ala
1               5                   10

<210>  447
<211>  12
<212>  PRT
<213>  artificial

<220>
<223>  peptide

<400>  447

Ala Ala Ala Asn Ser Pro Ser Gly Pro Arg Ala Ala
1               5                   10


<210>  448
<211>  12
<212>  PRT
<213>  artificial

<220>
<223>  peptide

<400>  448

Ala Ala Ala Arg Asp Gly Ile Thr Ser Arg Ala Ala
1               5                   10


<210>  449
<211>  12
<212>  PRT
<213>  artificial

<220>
<223>  peptide

<400>  449

Ala Ala Ala Ser Asn Ala Pro Ser Ala Arg Ala Ala
1               5                   10


<210>  450
<211>  12
<212>  PRT
<213>  artificial

<220>
<223>  peptide

<400>  450

Ala Ala Ala Val Met Asn Gly Gln Leu Arg Ala Ala
1               5                   10


<210>  451
<211>  12
<212>  PRT
<213>  artificial

<220>
<223>  peptide

<400>  451

Ala Ala Ala Tyr Ser Ala Gly Ser Pro Lys Ala Ala
1               5                   10


<210>  452
<211>  12
<212>  PRT
<213>  artificial

<220>
<223>  peptide

<400>  452

Ala Ala Ala Ser Asp Asn His Thr Ala Arg Ala Ala
1               5                   10


<210>  453
<211>  12
<212>  PRT
<213>  artificial

<220>
<223>  peptide

<400>  453

Ala Ala Ala Arg Asp Arg Ala Gly Pro Pro Ala Ala
1               5                   10


<210>  454
<211>  12
<212>  PRT
<213>  artificial

<220>
<223>  peptide

<400>  454

Ala Ala Ala Arg Val Arg Ala Thr Ser Pro Ala Ala
1               5                   10


<210>  455
<211>  12
<212>  PRT
<213>  artificial

<220>
<223>  peptide

<400>  455

Ala Ala Ala Ser Thr Arg Gly His Gly Ala Ala Ala
1               5                   10

<210>  456
<211>  12
<212>  PRT
<213>  artificial

<220>
<223>  peptide

<400>  456

Ala Ala Ala Ser Thr Arg Gly Arg Gly Ala Ala Ala
1               5                   10


<210>  457
<211>  12
<212>  PRT
<213>  artificial

<220>
<223>  peptide

<400>  457

Ala Ala Ala Asn Asp Val Arg Ala Pro Pro Ala Ala
1               5                   10


<210>  458
<211>  12
<212>  PRT
<213>  artificial

<220>
<223>  peptide

<400>  458

Ala Ala Ala Ser Asn Gly Gly Ser Pro Val Ala Ala
1               5                   10


<210>  459
<211>  12
<212>  PRT
<213>  artificial

<220>
<223>  peptide

<400>  459

Ala Ala Ala Met His Ala Ser Pro Ser Gly Ala Ala
1               5                   10


<210>  460
<211>  12
<212>  PRT
<213>  artificial

<220>
<223>  peptide

<400>  460

Ala Ala Ala Ala Ala Ser His Pro Asn Gln Ala Ala
1               5                   10


<210>  461
<211>  12
<212>  PRT
<213>  artificial

<220>
<223>  peptide

<400>  461

Ala Ala Ala Asp Thr Gly Ser His Ala Leu Ala Ala
1               5                   10


<210>  462
<211>  12
<212>  PRT
<213>  artificial

<220>
<223>  peptide

<400>  462

Ala Ala Ala Ser Pro Ser Asp Arg Phe Ser Ala Ala
1               5                   10


<210>  463
<211>  12
<212>  PRT
<213>  artificial

<220>
<223>  peptide

<400>  463

Ala Ala Ala Gly Pro Asn Ala Thr Ala Ser Ala Ala
1               5                   10


<210>  464
<211>  12
<212>  PRT
<213>  artificial

<220>
<223>  peptide

<400>  464

Ala Ala Ala Gly Gly Pro Gly Tyr Thr Ala Ala Ala
1               5                   10

<210>  465
<211>  12
<212>  PRT
<213>  artificial

<220>
<223>  peptide

<400>  465

Ala Ala Ala Gly Ser Asp Ala Thr Ala Ser Ala Ala
1               5                   10


<210>  466
<211>  12
<212>  PRT
<213>  artificial

<220>
<223>  peptide

<400>  466

Ala Ala Ala Ala Gln Tyr Ser Ala Ala Ala Ala Ala
1               5                   10


<210>  467
<211>  12
<212>  PRT
<213>  artificial

<220>
<223>  peptide

<400>  467

Ala Ala Ala Val Pro Thr Leu Arg Pro Ala Ala Ala
1               5                   10


<210>  468
<211>  12
<212>  PRT
<213>  artificial

<220>
<223>  peptide

<400>  468

Ala Ala Ala Ala Gly Thr Gly Ile Pro Val Ala Ala
1               5                   10


<210>  469
<211>  12
<212>  PRT
<213>  artificial

<220>
<223>  peptide

<400>  469

Ala Ala Ala Gly Ser Asn Ala Thr Ala Gly Ala Ala
1               5                   10


<210>  470
<211>  12
<212>  PRT
<213>  artificial

<220>
<223>  peptide

<400>  470

Ala Ala Ala Gly Pro Ala Asn Ser Ser His Ala Ala
1               5                   10


<210>  471
<211>  12
<212>  PRT
<213>  artificial

<220>
<223>  peptide

<400>  471

Ala Ala Ala Ala Ala Arg Ser Thr Asp Leu Ala Ala
1               5                   10


<210>  472
<211>  12
<212>  PRT
<213>  artificial

<220>
<223>  peptide

<400>  472

Ala Ala Ala Ser His Gly Gly Leu Ser Glu Ala Ala
1               5                   10


<210>  473
<211>  12
<212>  PRT
<213>  artificial

<220>
<223>  peptide

<400>  473

Ala Ala Ala Gly Gln Ala Met Ser Gly Gly Ala Ala
1               5                   10

<210>  474
<211>  12
<212>  PRT
<213>  artificial

<220>
<223>  peptide

<400>  474

Ala Ala Ala Gly Thr Asn Ala Thr Ala Ser Ala Ala
1               5                   10


<210>  475
<211>  12
<212>  PRT
<213>  artificial

<220>
<223>  peptide

<400>  475

Ala Ala Ala Phe Thr Ala Ala Asn Asn Gln Ala Ala
1               5                   10


<210>  476
<211>  12
<212>  PRT
<213>  artificial

<220>
<223>  peptide

<400>  476

Ala Ala Ala Ser Ser Asn Ala Thr Ala Ser Ala Ala
1               5                   10


<210>  477
<211>  12
<212>  PRT
<213>  artificial

<220>
<223>  peptide

<400>  477

Ala Ala Ala Gly Ser Asn Thr Thr Ala Ser Ala Ala
1               5                   10


<210>  478
<211>  12
<212>  PRT
<213>  artificial

<220>
<223>  peptide

<400>  478

Ala Ala Ala Val Ser Pro Asn His Ala Ala Ala Ala
1               5                   10


<210>  479
<211>  12
<212>  PRT
<213>  artificial

<220>
<223>  peptide

<400>  479

Ala Ala Ala Gly Ser Asn Ala Ala Ala Ser Ala Ala
1               5                   10


<210>  480
<211>  12
<212>  PRT
<213>  artificial

<220>
<223>  peptide

<400>  480

Ala Ala Ala Asn Val Arg Pro His Pro Ala Ala Ala
1               5                   10


<210>  481
<211>  12
<212>  PRT
<213>  artificial

<220>
<223>  peptide

<400>  481

Ala Ala Ala Asn Ser Glu Arg Ser Val Gln Ala Ala
1               5                   10


<210>  482
<211>  12
<212>  PRT
<213>  artificial

<220>
<223>  peptdie

<400>  482

Ala Ala Ala Thr Val Gly Arg Asp Ser Val Ala Ala
1               5                   10

<210>  483
<211>  12
<212>  PRT
<213>  artificial

<220>
<223>  peptide

<400>  483

Ala Ala Ala Pro Ser Asp Ser Thr Asp Arg Ala Ala
1               5                   10


<210>  484
<211>  12
<212>  PRT
<213>  artificial

<220>
<223>  peptide

<400>  484

Ala Ala Ala Pro Ala Asn Thr Pro Pro Arg Ala Ala
1               5                   10


<210>  485
<211>  12
<212>  PRT
<213>  artificial

<220>
<223>  peptide

<400>  485

Ala Ala Ala Gly Ala Asn Arg Pro Gly Leu Ala Ala
1               5                   10


<210>  486
<211>  12
<212>  PRT
<213>  artificial

<220>
<223>  peptide

<400>  486

Ala Ala Ala Thr Asn His Ala Gly Ala Ala Ala Ala
1               5                   10


<210>  487
<211>  12
<212>  PRT
<213>  artificial

<220>
<223>  peptide

<400>  487

Ala Ala Ala Ala Ser Thr Gly Pro Arg Thr Ala Ala
1               5                   10


<210>  488
<211>  12
<212>  PRT
<213>  artificial

<220>
<223>  peptde

<400>  488

Ala Ala Ala Ser Ser Tyr Ala Pro Ser Ser Ala Ala
1               5                   10


<210>  489
<211>  12
<212>  PRT
<213>  artificial

<220>
<223>  peptide

<400>  489

Ala Ala Ala Ala Gln Pro Pro Gln Pro Pro Ala Ala
1               5                   10


<210>  490
<211>  12
<212>  PRT
<213>  artificial

<220>
<223>  peptide

<400>  490

Ala Ala Ala Ala Pro Thr Gly Pro His Thr Ala Ala
1               5                   10


<210>  491
<211>  12
<212>  PRT
<213>  artificial

<220>
<223>  peptide

<400>  491

Ala Ala Ala Ala His Leu Ser Gly Thr Ser Ala Ala
1               5                   10

<210>  492
<211>  12
<212>  PRT
<213>  artificial

<220>
<223>  peptde

<400>  492

Ala Ala Ala Thr Arg Ser Gly Asp Ser Ser Ala Ala
1               5                   10


<210>  493
<211>  12
<212>  PRT
<213>  artificial

<220>
<223>  peptide

<400>  493

Ala Ala Ala Arg Thr Gly Arg Thr Ser Pro Ala Ala
1               5                   10


<210>  494
<211>  12
<212>  PRT
<213>  artificial

<220>
<223>  peptide

<400>  494

Ala Ala Ala Arg Thr Gly Arg Asp Ser Pro Ala Ala
1               5                   10


<210>  495
<211>  12
<212>  PRT
<213>  artificial

<220>
<223>  peptide

<400>  495

Ala Ala Ala Asp Asp Phe Gln Arg Ala Arg Ala Ala
1               5                   10


<210>  496
<211>  12
<212>  PRT
<213>  artificial

<220>
<223>  peptide

<400>  496

Ala Ala Ala Arg Asp Arg Ala Asp Ser Pro Ala Ala
1               5                   10


<210>  497
<211>  12
<212>  PRT
<213>  artificial

<220>
<223>  peptide

<400>  497

Ala Ala Ala Arg Asp Arg Ala Ile Ser Pro Ala Ala
1               5                   10


<210>  498
<211>  12
<212>  PRT
<213>  artificial

<220>
<223>  peptide

<400>  498

Ala Ala Ala Arg Asp Arg Arg Thr Ser Pro Ala Ala
1               5                   10


<210>  499
<211>  12
<212>  PRT
<213>  artificial

<220>
<223>  peptide

<400>  499

Ala Ala Ala Gly Ala Arg Arg Ser Asp Ser Ala Ala
1               5                   10


<210>  500
<211>  12
<212>  PRT
<213>  artificial

<220>
<223>  peptide

<400>  500

Ala Ala Ala Arg Thr Gly Gly Thr Ser Pro Ala Ala
1               5                   10

<210>  501
<211>  12
<212>  PRT
<213>  artificial

<220>
<223>  peptide

<400>  501

Ala Ala Ala Arg Thr Gly Ala Thr Ser Pro Ala Ala
1               5                   10


<210>  502
<211>  12
<212>  PRT
<213>  artificial

<220>
<223>  peptide

<400>  502

Ala Ala Ala Gly Ala His Arg Pro Asp Ser Ala Ala
1               5                   10


<210>  503
<211>  12
<212>  PRT
<213>  artificial

<220>
<223>  peptide

<400>  503

Ala Ala Ala Arg Arg Ser Gly Gly Thr Pro Ala Ala
1               5                   10


<210>  504
<211>  12
<212>  PRT
<213>  artificial

<220>
<223>  peptide

<400>  504

Ala Ala Ala Pro Pro Ser Ala Ala Ala His Ala Ala
1               5                   10


<210>  505
<211>  12
<212>  PRT
<213>  artificial

<220>
<223>  peptide

<400>  505

Ala Ala Ala Ser Ala His Arg Ser Asp Ser Ala Ala
1               5                   10


<210>  506
<211>  12
<212>  PRT
<213>  artificial

<220>
<223>  peptide

<400>  506

Ala Ala Ala Gly Ala His Arg Ser Asp Thr Ala Ala
1               5                   10


<210>  507
<211>  12
<212>  PRT
<213>  artificial

<220>
<223>  peptide

<400>  507

Ala Ala Ala Ala Gln Tyr Gly Ala Ala Val Ala Ala
1               5                   10


<210>  508
<211>  12
<212>  PRT
<213>  artificial

<220>
<223>  peptide

<400>  508

Ala Ala Ala Gly Ala His Arg Ser Asp Pro Ala Ala
1               5                   10


<210>  509
<211>  12
<212>  PRT
<213>  artificial

<220>
<223>  peptide

<400>  509

Ala Ala Ala Lys Gly Gly Arg Ser Ala Val Ala Ala
1               5                   10

<210>  510
<211>  12
<212>  PRT
<213>  artificial

<220>
<223>  peptide

<400>  510

Ala Ala Ala Pro Pro Ala Asp Ser Arg Val Ala Ala
1               5                   10


<210>  511
<211>  12
<212>  PRT
<213>  artificial

<220>
<223>  peptide

<400>  511

Ala Ala Ala Gly Ala Asn Arg Ser Asp Ser Ala Ala
1               5                   10


<210>  512
<211>  12
<212>  PRT
<213>  artificial

<220>
<223>  peptide

<400>  512

Ala Ala Ala Arg Asp Arg Ala Thr Leu Pro Ala Ala
1               5                   10


<210>  513
<211>  12
<212>  PRT
<213>  artificial

<220>
<223>  peptide

<400>  513

Ala Ala Ala Ser Ile Ser Ala Thr Ala Arg Ala Ala
1               5                   10


<210>  514
<211>  12
<212>  PRT
<213>  artificial

<220>
<223>  peptide

<400>  514

Ala Ala Ala Thr Ala Asn Pro Gly Leu Arg Ala Ala
1               5                   10


<210>  515
<211>  12
<212>  PRT
<213>  artificial

<220>
<223>  peptide

<400>  515

Ala Ala Ala Arg Gly Gly Ala Leu Gly Gly Ala Ala
1               5                   10


<210>  516
<211>  12
<212>  PRT
<213>  artificial

<220>
<223>  peptide

<400>  516

Ala Ala Ala Pro Pro Asp Ala Tyr Arg Met Ala Ala
1               5                   10


<210>  517
<211>  12
<212>  PRT
<213>  artificial

<220>
<223>  peptide

<400>  517

Ala Ala Ala Ser Arg Ala Ser Pro Val Ala Ala Ala
1               5                   10


<210>  518
<211>  12
<212>  PRT
<213>  artificial

<220>
<223>  peptide

<400>  518

Ala Ala Ala Arg Asp Arg Ala Thr Phe Pro Ala Ala
1               5                   10

<210>  519
<211>  12
<212>  PRT
<213>  artificial

<220>
<223>  peptide

<400>  519

Ala Ala Ala Arg Asp Arg Arg Asp Ser Pro Ala Ala
1               5                   10


<210>  520
<211>  12
<212>  PRT
<213>  artificial

<220>
<223>  peptide

<400>  520

Ala Ala Ala Ser Gly Pro Gly Gly Thr Pro Ala Ala
1               5                   10


<210>  521
<211>  12
<212>  PRT
<213>  artificial

<220>
<223>  peptide

<400>  521

Ala Ala Ala Arg Arg Pro Gly Gly Thr Pro Ala Ala
1               5                   10


<210>  522
<211>  12
<212>  PRT
<213>  artificial

<220>
<223>  peptide

<400>  522

Ala Ala Ala Met Arg Pro Gly Asn Ser Ile Ala Ala
1               5                   10


<210>  523
<211>  12
<212>  PRT
<213>  artificial

<220>
<223>  peptide

<400>  523

Ala Ala Ala Ala Gly Arg Ser Pro Val Ser Ala Ala
1               5                   10


<210>  524
<211>  12
<212>  PRT
<213>  artificial

<220>
<223>  peptide

<400>  524

Ala Ala Ala Ala Ala Arg Ser Tyr Ser Ser Ala Ala
1               5                   10


<210>  525
<211>  12
<212>  PRT
<213>  artificial

<220>
<223>  peptide

<400>  525

Ala Ala Ala Gln Thr Asn Pro Val Arg His Ala Ala
1               5                   10


<210>  526
<211>  12
<212>  PRT
<213>  artificial

<220>
<223>  peptide

<400>  526

Ala Ala Ala Glu Gly Gly Ala Leu Gly Gly Ala Ala
1               5                   10


<210>  527
<211>  12
<212>  PRT
<213>  artificial

<220>
<223>  peptide

<400>  527

Ala Ala Ala Leu Ala Ala Arg Asp Ala Arg Ala Ala
1               5                   10

<210>  528
<211>  12
<212>  PRT
<213>  artificial

<220>
<223>  peptide

<400>  528

Ala Ala Ala Met Pro Ser Gly Thr Thr Arg Ala Ala
1               5                   10


<210>  529
<211>  12
<212>  PRT
<213>  artificial

<220>
<223>  peptide

<400>  529

Ala Ala Ala Gly Ala His Arg Ser Gly Ser Ala Ala
1               5                   10


<210>  530
<211>  12
<212>  PRT
<213>  artificial

<220>
<223>  peptide

<400>  530

Ala Ala Ala Gly Thr His Arg Ser Asp Ser Ala Ala
1               5                   10


<210>  531
<211>  12
<212>  PRT
<213>  artificial

<220>
<223>  peptide

<400>  531

Ala Ala Ala Glu Gly Pro Gly Asp Ser Pro Ala Ala
1               5                   10


<210>  532
<211>  12
<212>  PRT
<213>  artificial

<220>
<223>  peptide

<400>  532

Ala Ala Ala Arg Asp Arg Ala Ser Ser Pro Ala Ala
1               5                   10


<210>  533
<211>  12
<212>  PRT
<213>  artificial

<220>
<223>  peptide

<400>  533

Ala Ala Ala Glu Gly Pro Gly Thr Ser Pro Ala Ala
1               5                   10


<210>  534
<211>  12
<212>  PRT
<213>  artificial

<220>
<223>  peptide

<400>  534

Ala Ala Ala Lys Gly Gly Ala Thr Ser Pro Ala Ala
1               5                   10


<210>  535
<211>  12
<212>  PRT
<213>  artificial

<220>
<223>  peptide

<400>  535

Ala Ala Ala Glu Asp Arg Ala Thr Ser Pro Ala Ala
1               5                   10


<210>  536
<211>  12
<212>  PRT
<213>  artificial

<220>
<223>  peptide

<400>  536

Ala Ala Ala Ser Arg Gly Ala Pro Thr Thr Ala Ala
1               5                   10

<210>  537
<211>  12
<212>  PRT
<213>  artificial

<220>
<223>  peptide

<400>  537

Ala Ala Ala Asp Ser Arg Leu Lys Gln Ser Ala Ala
1               5                   10


<210>  538
<211>  12
<212>  PRT
<213>  artificial

<220>
<223>  peptide

<400>  538

Ala Ala Ala Ala Asn Thr Val Leu Arg Pro Ala Ala
1               5                   10

## Description

The present invention relates in a first aspect to a mutated adeno-associated virus (AAV) capsid protein or fragment thereof having an insert composed of an oligopeptide optionally having further amino acids representing linker sequences at both sites of said oligopeptide. These mutated AAV capsid proteins are more efficient in transferring genetic material into hepatic tissue, hepatocytes, hepatic cells and cell lines or hepatocellular carcinoma (HCC) partially with improved tropism for these cell types. Further, a mutated AAV particle (uniform or as hybrid) comprising the mutated AAV capsid protein according to the present invention is provided. In addition, a nucleic acid encoding the mutated AAV capsid protein according to the present invention is identified together with a corresponding nucleic acid vector, in particular, a plasmid. In addition, a host cell containing the nucleic acid vector or the nucleic acid molecule according to the present invention. Further, a use of the AAV particle or the nucleic acid or the nucleic acid vector according to the present invention in the manufacture of a medicament for gene therapy. Moreover, the described protein, particle molecules as well as nucleic acid vectors for use in targeting hepatocytes and/or HCC are described. In particular, said components for use in treating diseases involving hepatocytes or for treating HCC are disclosed, in particular, for use in gene therapy, e.g. for use in a transfer of a gene of interest into hepatocytes, hepatic tissue or HCC.

### Background of the invention

The liver represents an essential organ with complex metabolic, immunological and hemostatic functions. The liver is affected by a broad range of monogenic disorders and chronic metabolic conditions, see Marcellin, P. and Kutala, B. K., Liver International, 2018, 38(S1), 2-6. Consequently, liver has become a key target for the development of gene therapy strategies in particular with AAV vectors as summarized in Baruteau, J., Journal of Inherited Metabolic Disease, 2017, 497-517.

As reviewed recently, promising results, for example, have been obtained for patients suffering from hemophilia, see Pierce, G. F. and lorio, A., Hamophilia, 1018, 24(Suppl 6), 60-67. However, still high vector doses are required to compensate for the low efficacy of AAV vectors in human patients. While efficacy is sufficient to obtain the moderate physiological levels of blood clotting factors required to transform severe hemophilic phenotypes into mild forms, other liver diseases are in urgent need for more effective AAV vectors. In addition, patients establish neutralizing capsid antibodies upon the first AAV vector dose, as described by Fitzpatrick et al., Molecular Therapy: Methods & Clinical Development. Elsevier Ltd., 9 (June), 119-129. This impairs re-administration of AAV vectors with the same or cross-reacting AAV capsids for improving transgene expression levels. Therefore, AAV vectors with novel capsid properties for efficient liver-directed gene transfer are of utmost importance for improving current and future gene therapeutic approaches.

Beside the above-mentioned diseases, liver is prone to develop cancer, in particular hepatocellular carcinoma (HCC), or to being the "home" for metastasis originating from tumors other than HCC. With regard to HCC, it is reported to be the third most common cause of cancer-related deaths worldwide. It is a severe cancer disease with poor prognosis at advanced HCC stages. So far potentially curative treatments are limited to surgical procedures like tumor resection, orthotopic liver transplantation, or percutaneous radio frequency ablation, see Waghray, A. et al., WJH, 2015, 7(8), 1020-1029. The majority of patients are diagnosed at advanced stages of HCC for which current therapeutic options are very poor. Therefore, there is a strong need for novel therapeutic approaches including AAV vector-based cancer gene therapy. AAV vectors may be used to deliver suicide genes, tumor-suppressor genes or immunotherapeutic transgenes to the tumor site, e.g. as discussed in Dhungel, B. et al., 2017, 7544. To provide efficient and safe delivery of transgene for cancer gene therapy, engineering of novel AAV vectors is required which are directed to the HCC target tissue and are capable of efficient therapeutic transgene expression.

AAV vectors are based on the adeno-associated virus (AAV), a non-pathogenic member of the parvoviridae and the genus Dependoparvovirus. AAV are composed of a single-stranded DNA genome of about 4.7 kB packaged into a non-enveloped icosahedral protein capsid. It is a small (25 nm in diameter) virus, consisting of 60 monomers forming the icosahedral capsid. These monomers are composed of the viral capsid proteins (VP) VP1, VP2, and VP3 in a 1:1:10 ratio. The 4.5 kB single stranded coding DNA genome (plus inverted terminal repeats (ITRs)) is bearing two genes (rep and *cap*) encoding for non-structural (Rep proteins and assembly activating protein) and structural proteins (VP1, VP2, VP3). The AAV genome is flanked by ITRs, which serve as packaging and replication signals. AAV infection biology is mainly based on the interaction of the AAV capsid with the host cell. Specifically, the capsid is interacting with cell surface receptors of the host cell mediating cell entry and intracellular trafficking as well as processing of the virion. AAV has not been associated with any disease; rather, discussion arises about being tumor protective. It is unique in being dependent on the presence of a helper virus like adenovirus for progeny production. Further, AAV is able to transduce mitotic and postmitotic tissues and shows a broad tissue tropism. In fact, for *in vivo* application a major drawback of AAV vectors, in particular, AAV serotype 2-based vectors, is their broad tropism. Since AAV vectors are able to transduce a wide range of cell types, transduction efficacy of a distinct target organ is lowered, and administration of higher vector doses are required to achieve therapeutic target cell transduction levels.

However, AAV, in particular, AAV2, has gained tremendous popularity as vector for gene therapy, but also as platform for vaccine development and as tool in preclinical research. AAV vectors are considered as gene delivery system of choice for *in vivo* gene therapy. Advantageous features of AAV include its apathogenicity, high stability, ability to transduce both dividing and non-dividing cells, long-term gene expression in post mitotic or slowly proliferating cells and low immunogenicity. In addition, AAV vectors lack an intrinsic genome integration activity and are therefore defined as non-integrating vector system. This is a main advantage compared to retro/lentiviral vectors as it significantly reduces the risk of insertional mutagenesis. Further, vectors can be produced to high titer and purity owing to its stability.

To date, 13 different natural human and non-human primate AAV serotypes and over 100 natural variants have been isolated. Most of the serotypes have been vectorized as alternatives to AAV serotype 2, the prototype AAV vector, because they differ in tropism and in epitopes recognized by the immune system. For vectorization viral genomes are replaced by a transgene cassette. Usage of serotypes is simplified by the pseudopackaging technology allowing to package ITR2-flanked AAV vector genomes into non-AAV2 capsids. This enables the production of AAV vectors differing in capsid but delivering the same vector genome. The AAV vector capsid and genome can be modified and optimized for efficient and directed gene delivery *in vitro* and *in vivo.*

Pseudotyping (pseudopackaging) represents a strategy to change the tropism of the AAV vector system. However, it does not allow to re-direct the tropism towards a distinct cell type. In addition, its potency of increasing efficiency of AAV vector-mediated cell transduction is limited. Therefore, alternative strategies summarized as capsid-engineering technologies have been developed to tailor the host-AAV interaction including the first step of cell transduction. Consequently, non-permissive cell types became susceptible towards AAV transduction and transduction efficiencies for permissive cells could be improved. In addition, it became possible to redirect viral vector *in vivo* tropism towards a defined cell surface structure (Büning, H. et al., Current Opinion in Pharmacology, 2015, 24, 94-104).

In genetic capsid engineering approaches which focus on cell surface targeting, peptide ligands are inserted into a suitable position of the capsid. This position needs to be exposed on the capsid surface to influence target cell infection properties. To date, peptide insertions for tropism modification are mainly performed by the following techniques: i) fusion to the N-terminus of VP2 or ii) insertion of peptides at the tip of variable region (VR)-VIII, for AAV2: insertion at amino acid position 587 and insertion at position 588, or insertion at VR-IV, for AAV2: insertion at amino acid position 453 as described in Büning et al., see above; and Büning and Svrivastava, Mol Ther Methods Clin Dev, 2019, 12, 248-265. For example, EP 2 158 211 B1 identifies structure protein insertions having a length of 4 to 13 amino acids, which represent ligands mediating cell targeting accordingly. In addition, approaches have been conducted for substituting specific amino acids present in the capsid proteins.

Since for a lot of target cells or tissues of therapeutic interest suitable peptide ligands that would mediate target cell transduction are unknown, high-throughput screenings of AAV peptide display libraries have been described. For example, libraries have been disclosed in Müller, O. J. et al., Nature Biotechnology, 2003, 21(9), 1040-1046 and Perabo, L. et al., Molecular Therapy, 2003, 8(1), 151-157. The AAV peptide display library established by Perabo et al. is composed of >4 E6 capsid variants. The library has been generated by insertion of a random 7-mer peptide into amino acid position 587 (VP1 numbering) of AAV2 capsid proteins. The random peptide is flanked by linker sequences to help optimal presentation. This library is described for example in EP 1 456 383 B1 and also in EP 2 363 487 A2. Selection of this library on target cells has been used to identify AAV capsid variants with novel and improved transduction properties.

In US 2002/0192823 A1 an AAV capsid variant and methods have been described whereby said AAV capsid variant is derived from a shuffled AAV capsid library based on AAV2, AAV8, and AAV9.

Further strategies include attachment of foreign molecules to the outer surface of the vector that can mediate specific interaction with cellular receptors expressed on the membrane of target cell types. Chemical modifications of amino acids present on the surface or on the exterior of the capsid surface have been discussed. These modifications include binding of biotin for allowing coupling of further components, e.g. via streptavidin. Further, introduction of unnatural moieties have been disclosed. For example, WO 2012/149160 A2 describes the strategy for modification of virus capsid proteins based on introducing unnatural amino acid moieties which eventually allows to introduce further groups.

As noted, a main problem of the AAV vector system is the *in vivo* tropism as well as the transduction efficiency of specific tissue types, thus, requiring the use of high vector doses.

### Summary of the present invention

Consequently, it is an object of the present invention to provide AAV vectors and capsid proteins overcoming the problems described in the art, namely, the problem of broad tropism and low transduction efficiency.

The present invention relates in a first aspect to a mutated adeno-associated virus (AAV) capsid protein or a fragment thereof wherein an insert is inserted after at least one of the amino acids having the amino acid numbers 139, 161, 261, 381, 447, 453, 459, 534, 570, 573, 584, 585, 586, 587, 588, 589 of SEQ ID No. 2 corresponding to the capsid protein of AAV2 or the homolog capsid protein of the other serotypes of AAV, namely, AAV1, AAV3b to AAV11, and AAV bovine, the insert comprises an oligonucleotide of at least four amino acids and at most thirty amino acids, like consisting of five to twelve amino acids, in particular, seven amino acids, said insert may optionally contain further amino acids representing a linker sequence at both sites of said oligopeptide, said linker sequence having independently from one another a size of 1 to 3 amino acids, if present; wherein the mutated AAV capsid protein or a fragment thereof or its homologs being more efficient in targeting hepatic tissue, hepatocytes, hepatic cells and cell lines or hepatocellular carcinoma (HCC) with higher specificity.

In a second aspect, the present invention relates to a mutated AAV particle comprising the AAV capsid protein or fragment thereof according to the present invention. Further, the present invention relates to a nucleic acid encoding the AAV virus capsid protein or fragment thereof according to the present invention. In addition, a nucleic acid vector, in particular, a plasmid comprising the nucleic acid molecules according to the present invention is disclosed as well as host cells containing said nucleic acid vector or said nucleic acid according to the present invention.

Moreover, the use of the AAV particle according to the present invention or the nucleic acid molecule according to the present invention or the nucleic acid vector according to the present invention in the manufacture of an AAV particle, or a manufacture of a medicament for gene therapy. Moreover, the components described herein including the mutated AAV capsid protein or a fragment thereof or a homolog as well as the mutated AAV particle or the nucleic acid molecule or the nucleic acid vector are suitable for use in targeting hepatocytes and/or HCC and/or improving transduction efficiency of AAV vectors in hepatocytes and/or HCC. Further, these components are suitable for use in transfer of a gene of interest into hepatocytes or HCC, in particular, for use in gene therapy.

That is, the present inventors identified new variants of mutated AAV capsid proteins or fragments thereof being more efficient in transducing hepatic tissue, hepatocytes, hepatic cells and cell lines, and HCC, respectively, with higher specificity and/or efficiency.

### Brief description of the drawings

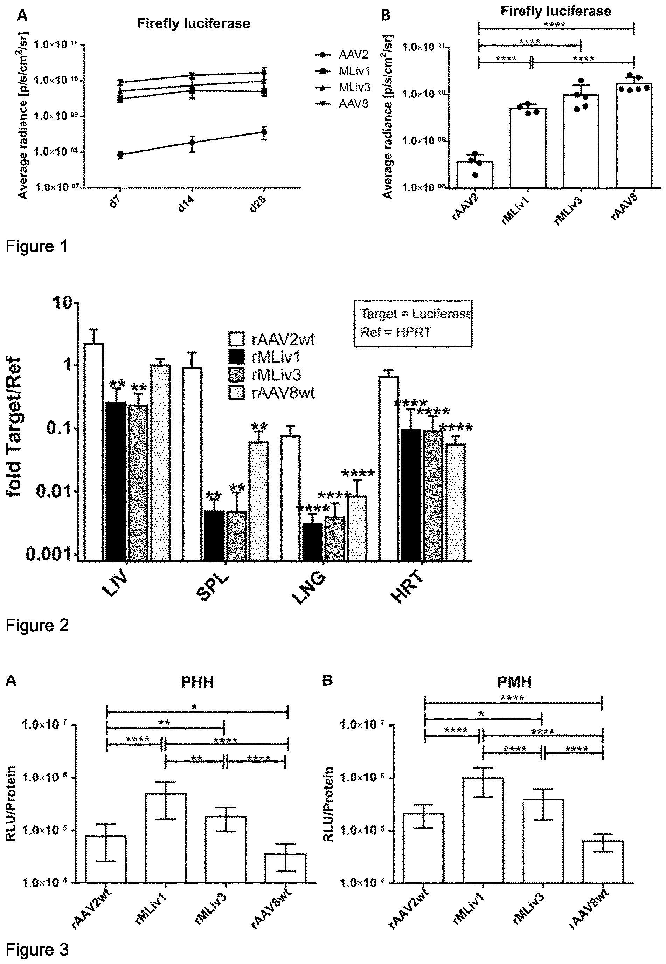

Figure 1: Mean transgene expression of rAAV vectors in Balb/c mice (A) over the course of 28 days and (B) at day 28. Animals received 4.8E11 particles of indicated AAV vectors by tail vein injection. After 7, 15, and 28 days mice were analyzed for vector transduction. Specifically, animals received 1.5 mg of D-Luciferin by tail vein injection and were positioned within IVIS® instrument. After 5 min images were taken with 5 min exposure. Radiance represents firefly luciferase-induced luminescence signal intensity measured as photons/second/cm²/steradiant[p/s/cm²/sr]. Statistics: Error bars show SD. Ordinary Two-way ANOVA and Tukey's multiple comparisons test were performed. *p<0.05; **p<0.01, ***p<0.001, ****p<0.00001;
Figure 2: Biodistribution of rAAV vector genomes determined by qPCR quantification. For determining vector genome content in liver compared to main-off target tissues (LIV=liver; SPL=spleen; LNG=lung; HRT=heart), total DNA was isolated from animals of the above-described *in vivo* experiment (Figure 1). Indicated DNA samples were analyzed by relative qPCR quantification using firefly luciferase as target and mHPRT as reference gene. The biodistribution is shown as relative transgene content for the different tissue samples. The target gene contents are defined as cTarget/Reference ratios and represent Target/Reference ratios calibrated by an inter-plate calibrator. Error bars show SD. Mean of rAAV8 relative vector genome content in liver tissue was set as one. Statistics: Error bars show SD. Shown significances relate to rAAV2. One-Way ANOVA und Tukeys Multiple Comparison Test: **p<0.01; ****p<0.0001);
Figure 3: Transduction of primary human and murine hepatocytes with rMLIV1 and rMLIV3 compared to parental rAAV2 and hepatotropic rAAV8 controls. Primary human (A) or murine (B) hepatocytes were seeded and subsequently transduced with a vector particle per cell ratio (GOI) of 10000. 72 hrs post transduction, cells were lysed and a luciferase assay was performed to determine level of transduction as means of luciferase activity. Luminescence signal shown in relative luminescence units (RLU). Mean RLUs were normalized to protein content measured by Bradford assay. Statistics: Error bars show SD. One-Way ANOVA und Tukeys Multiple Comparison Test: *p≤0.05, **p<0.01; ***p<0.001; ****p<0.0001);

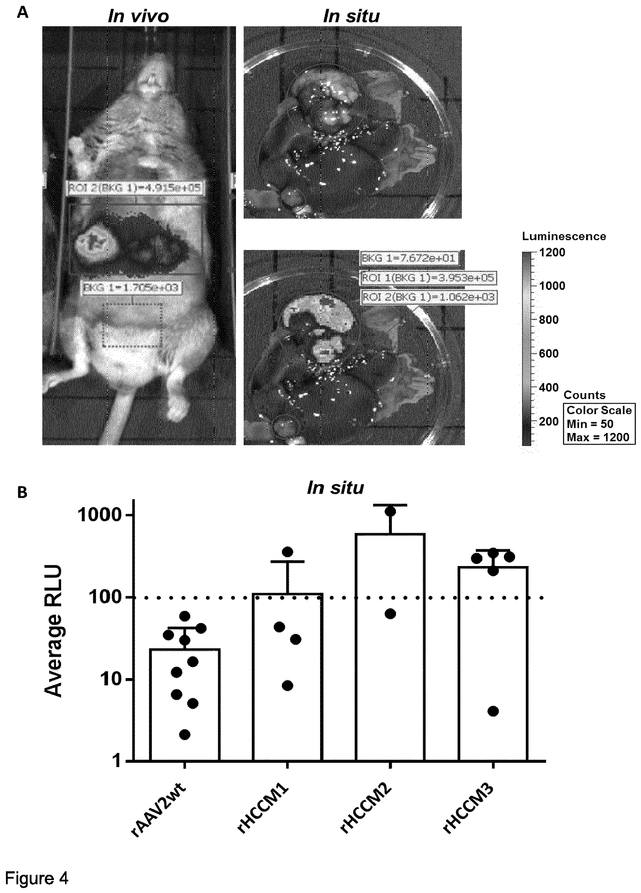

Figure 4: HCC transduction efficiency of novel AAV2-based capsid variants (rHCCM1, rHCCM2, and rHCCM3) compared to parental rAAV2wt control. A) Representative images of *in vivo* and *in situ* imaging of luciferase activity/luminescence signal 3 days post vector injection into the TGFα/c-myc HCC mice. B) Quantification of *in situ* luciferase activity/luminescence of tumor nodules; Animals received 1.5E11 particles of indicated vectors by tail vein injection. Three days post injection, animals were prepared for *in vivo* imaging. 5 min after D-luciferin injection (1 mg per animal) a series of images was taken with 3 min of exposure time by IVIS®. One hour after the first measurement, animals received a second injection of D-luciferin. Animals were sacrificed after 5 min, livers were isolated and then imaged with 3 min of exposure time. Counts represent firefly luciferase-induced luminescence signal intensity measured as relative light units (RLU). Signal overlay images (A) generated with a color scale of 50 to 1200 counts. 100 RLU defined as back ground level. Dashed rectangle marks background (BKG) ROI on animal skin; RLU values represent background-corrected signals. Circles represent ROIs marking tumor nodules. Dashed circle mark background (BKG) ROI on non-tissue area; ROI (BKG) tags show BKG-corrected total RLU values measured within ROIs; BKG tags show total RLU values of measured within BKG ROI;

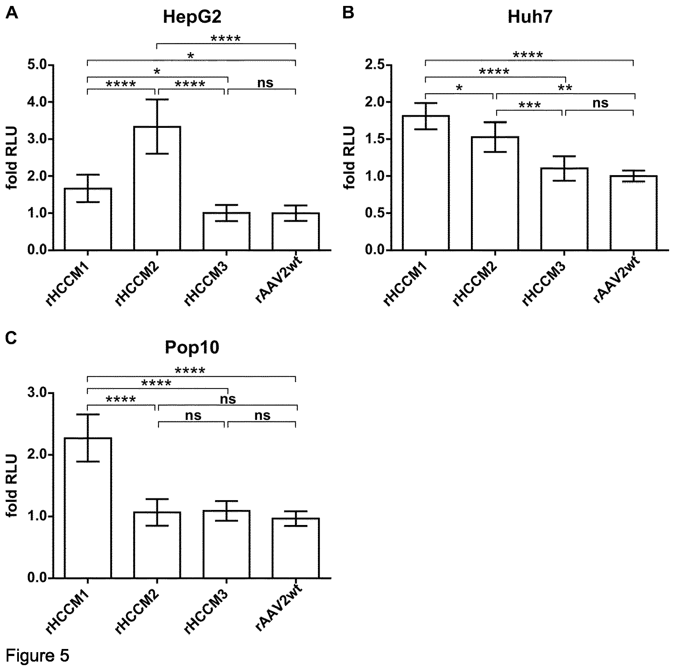

Figure 5: Transduction of human hepatoma and human hepatocyte cell lines by indicated novel capsid variants compared to parental rAAV2wt control. A) HepG2, Huh7 B) and C) Pop10 were transduced with a particle per cell ratio (GOI) of 1000. 24 hrs post transduction, cells were lysed and luciferase assay was performed. Luciferase activity as luminescence signal is shown in relative luminescence units (RLU). Mean RLU of rAAV2 was set as one. Statistics: One-Way ANOVA und Tukeys Multiple Comparison Test: *p≤0.05, **p<0.01; ***p<0.001; ****p<0.0001);

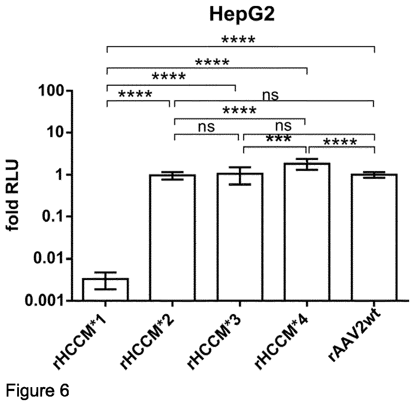

Figure 6: Transduction of human hepatoma cell line with indicated capsid variants compared to parental rAAV2wt control. HepG2 were transduced with GOI 1000 and harvested 24 hrs post transduction. Cells were lysed and luciferase assay was performed to detect luciferase activity as luminescence signal shown in relative luminescence units (RLU). Mean RLU of rAAV2 (rAAV2wt control) was set as one. Statistics: One-Way ANOVA und Tukeys Multiple Comparison Test: *p≤0.05, **p<0.01; ***p<0.001; ****p<0.0001).

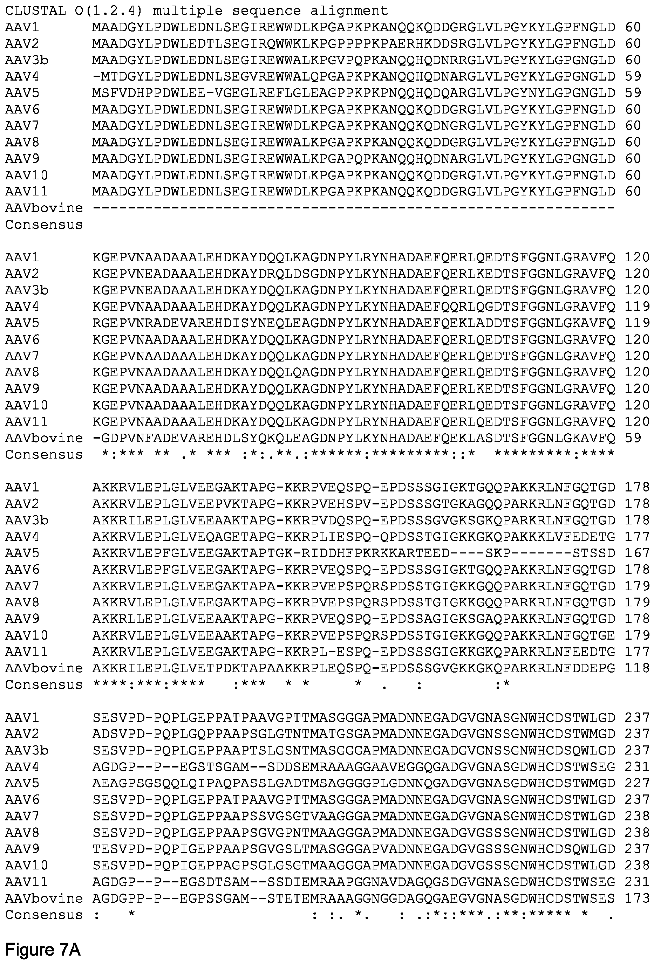

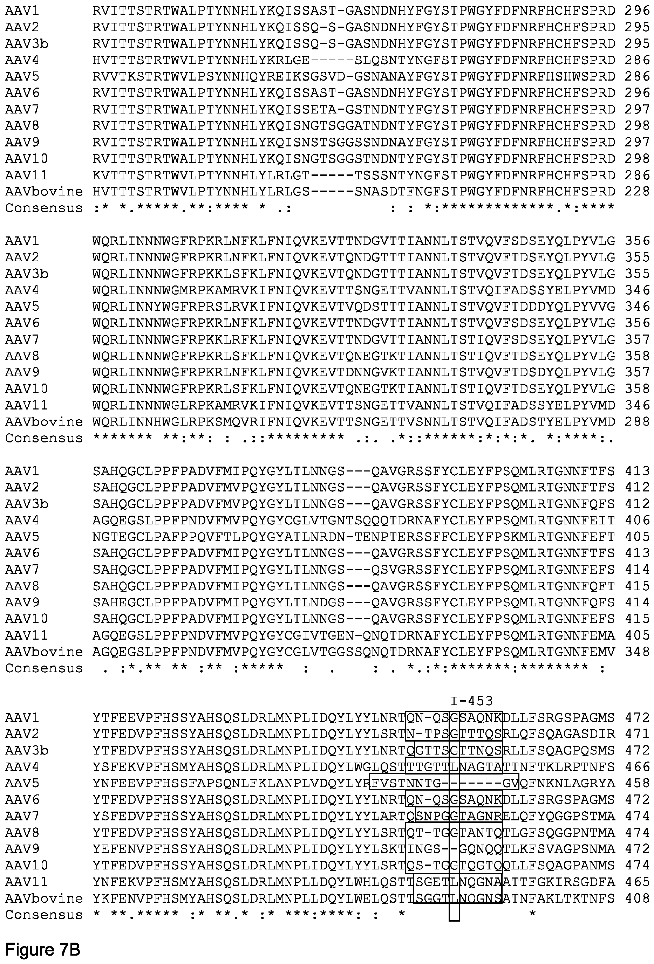

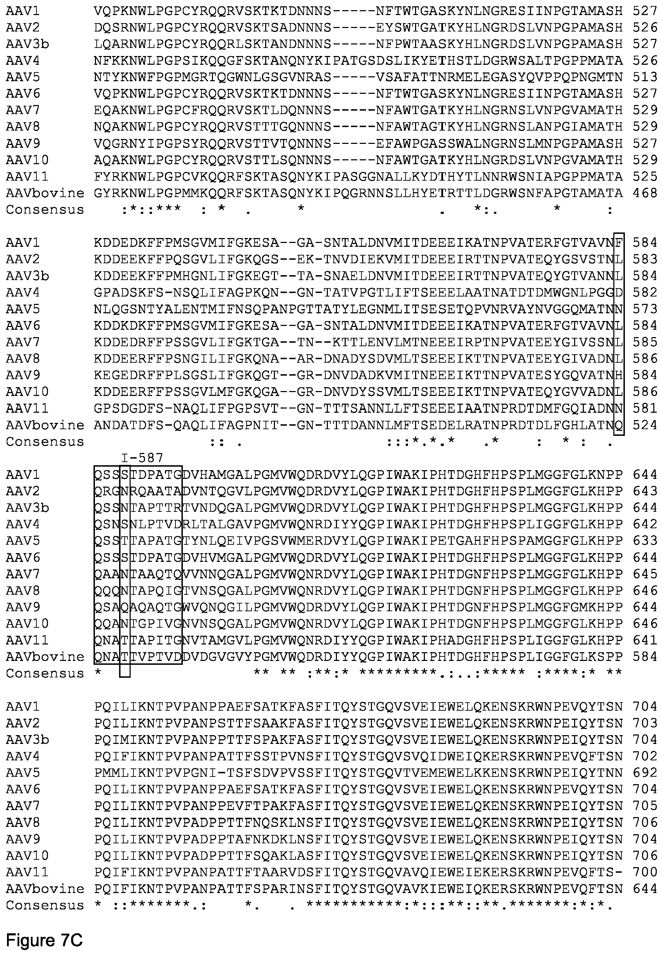

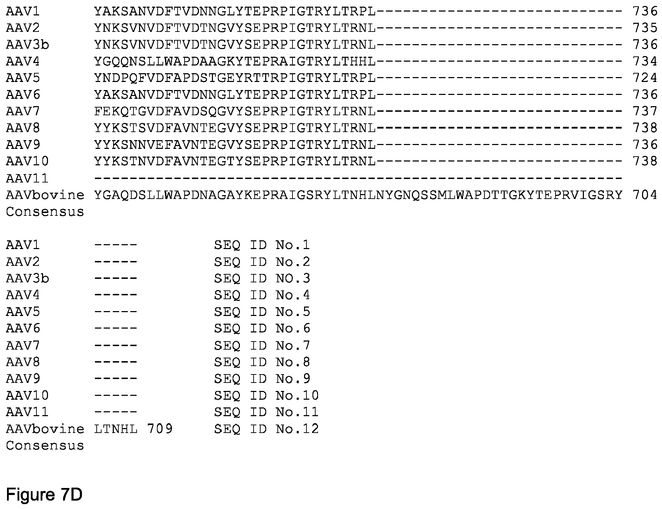

Figure 7A-D: VP1 amino acid sequence alignment of AAV serotypes AAV1, AAV2, AAV3b, AAV4, AAV5, AAV6, AAV7, AAV9, AAV10, AAV11 and AAVbovine. I-587 and I-453 represent preferred homologous insertion sites after amino acid positions 587 and 453 (AAV2 VP1 numbering).

### Detailed description of the present invention

The present inventors identified new mutant AAV capsid protein or fragment thereof being more efficient and partially more specific in targeting hepatic tissue, hepatocytes, hepatic cells and cell lines or hepatocellular carcinoma (HCC).

The vector particles as well as the capsid proteins are characterized in containing insertions of an insert after at least one of the amino acids having the amino acid numbers 139, 161, 261, 381, 447, 453, 459, 534, 570, 573, 584, 585, 586, 587, 588, 589 of SEQ ID No. 2 corresponding to the capsid protein of AAV, or the homolog capsid protein of the other serotypes of AAV, namely, AAV1, AAV3 to AAV11, and AAV bovine, the insert comprises an oligopeptide of at least four amino acids and at most 30 amino acids, like consisting of five to 12 amino acids, in particular, seven amino acids. The insert may optionally contain further amino acids representing a linker sequence at both sites of said oligopeptide, said linker sequence having independently from one another a size of one to three amino acids, if present.

As used herein, the term "fragment thereof" refers to a protein or a polypeptide derived from the mutated adeno-associated virus capsid protein. Typically, the size of the fragment is at least 50 %, like at least 70 %, as 80 %, 90 %, like 95 % of the size or length of the sequence it refers to.

The term "mutated AAV capsid protein" means a capsid protein containing an insert composed of amino acids at the distinct positions identified herein referring to the sequence of SEQ ID No. 2 corresponding to the capsid protein VP1 of AAV2.

The term "homolog capsid protein" refers to the homolog capsid protein VP1 of the other serotypes of AAV, namely, AAV1 (SEQ ID No. 1), AAV3 (SEQ ID No. 3), AAV4 (SEQ ID No. 4), AAV5 (SEQ ID No. 5), AAV6 (SEQ ID No. 6), AAV7 (SEQ ID No. 7), AAV8 (SEQ ID No. 8, AAV9 (SEQ ID No. 9), AAV10 (SEQ ID No. 10), AAV11 (SEQ ID No. 11) and AAV bovine (SEQ ID No. 12). That is, the homologs are serotype capsid proteins derived from the other AAV serotypes. An overview is given in Vance, M. A. et al., DOI: 10.5772/61988. The serotype alignment is shown in figure 7A-D. That is, the homologous insertion sites in other AAV serotypes corresponding to the AAV2 insertion sites can be easily determined by the skilled person,

The term "outer surface of the capsid" identifies the exterior of the capsid, thus, displaying the insert for allowing interaction with an interacting partner. The term "outer surface of the capsid" is used interchangeably with the term "exterior of the capsid".

The term "linker" refers to an amino acid sequence which is optionally present in the mutated AAV capsid protein according to the present invention. Said linker is a linking element between the oligopeptide present in the insert of the mutated AAV capsid protein and the wild type amino acid sequence of the VP1 protein.

The term "particle" or "AAV particle", identify a capsid either without DNA or containing DNA. The term "vector particle", or "vector" identify a capsid containing DNA. The term "AAV" refers to the adeno-associated virus or the derivatives thereof including recombinant AAV vector particles wherein the term "AAV wild type particle" designates the adeno-associated virus capsid as it occurs in nature without DNA or containing DNA. The term "recombinant AAV" or "recombinant vector particle" is an AAV wherein the genome of the virus is substituted with a vector genome, namely, a foreign DNA to be introduced into the cell and is also termed "rAAV". The term "AAV" or the respective recombinant AAV vector particle and AAV wild type particle include the at least 13 different serotypes known in the art. The AAV of human serotype 2 is also mentioned as AAV2 or AAV2 particle or AAV2 vector particle.

The term "targeting" refers to the improvement of specificity for the target tissues or target cells and/or the improvement of transduction efficiency in the target tissues or target cells by genetic modification of the AAV capsid. The term "targeting molecule" refers to a molecule allowing targeting of AAV particle as described herein to target cells.

The term "target cells" or "target tissue" as used herein refers to specific cell types or tissues representing the target as defined by the targeting molecule present in the mutated AAV capsid proteins or fragments thereof according to the present invention. Namely, the target cells or target tissue is hepatic tissue, hepatocytes, hepatic cells and cell lines, or hepatocellular carcinoma.

The present inventors recognized that inserting the insert at the distinct positions of the capsid protein allows to provide capsid proteins and, eventually, mutated adeno-associated virus particles being more efficient in targeting hepatic tissue, hepatocytes, hepatic cells and cell lines or hepatocellular carcinoma with partially higher specificity.

In an embodiment, the mutated AAV capsid protein, the fragment thereof or a homolog thereof is a mutated AAV capsid protein being derived from AAV type 1, 2, 3b, 4, 5, 6, 7, 8, 9, 10, 11 and bovine. In an embodiment, the mutated AAV capsid protein is derived from the VP1 of serotype AAV2.

In a further embodiment of the present invention, the mutated AAV capsid protein, the fragment thereof or a homolog thereof is a protein wherein the insert is at the position following at least one of the amino acids of 261, 453, 534, 570, 573, 587 and 588. In a preferred embodiment, the position wherein the insert is introduced after positions 453 and 587, respectively. That is, the insertion is in between positions 453 and 454 of SEQ ID No. 2 and in between 587 and 588 of SEQ ID No. 2.

The sequence of SEQ ID No. 2 corresponds to the encoding sequence of the VP1 protein of AAV2. In another embodiment, the mutated AAV capsid protein, a fragment thereof, or a homolog thereof according to the present invention is a mutated AAV capsid protein, a fragment thereof, or the homolog thereof comprising at least one further mutation selected from a point mutation, an internal or terminal deletion, a second insertion and a mutation.

For example, the additional mutation may be a mutation described in the art. Further, the additional mutation may be a mutation as described in undisclosed EP 17193742.8.

The mutated AAV capsid protein according to the present invention were obtained by screening the library *in vivo* in two different orthotopic HCC mouse models following a pre-clearing step for variants that bind heparin since this feature correlates with unspecific binding to a wide variety of different cell types, which express heparan sulfate proteoglycane (HSPG), the AAV2 primary attachment cell surface receptor. One of the HCC mouse models was generated by transplanting a murine hepatoma cell line (Hepa129) into the liver of C3H mice (Schmitz et al, J. Hepatol. 2004. 787-797). A further mouse model represents a transgenic mouse model over-expressing TGF-a and c-myc regulated by ZnCl₂-and albumin-inducible promoters, respectively (Haupenthal J. et al., Neoplasia, 2012, 14(5), 410-419).

The mutated AAV capsid proteins containing the insertion at the predetermined positions as disclosed herein are characterized in displaying the introduced insert from the capsid at the outer surface of the capsid when the capsid is assembled as an AAV particle. That is, the present inventors aim to improve the AAV vector system with regard to efficiency and selectivity of *in vivo* gene transfer into hepatocytes and its malignant counterpart, hepatocellular carcinoma cells, based on the mutated AAV capsid proteins according to the present invention. That is, superior transduction efficiencies of hepatocytes and HCC have been achieved with higher cell entry rates and more efficient vector genome accessibility for transcription. The variants described herein transduced hepatocytes and HCC more efficiently compared to wild type AAV2 or other known mutated capsid strains. In addition, the mutated AAV capsid proteins according to the present invention are found to be superior regarding expression efficiency, i.e. transcripts per vector copy, arguing for a more efficient intercellular processing again compared to the wild type AAV2.

That is, the displayed insert present in the outer surface of the capsid when the capsid is assembled as an AAV particle increases efficiency of transfection, thus, lower vector doses are required to achieve therapeutic target cell transduction levels in hepatocytes and HCC, respectively.

In an embodiment of the present invention, the mutated AAV capsid protein, the fragment thereof or a homolog thereof according to the present invention are a capsid protein or a protein fragment or homolog thereof wherein the linker sequences in an amino acid sequence is containing or consisting of amino acids G, S and/or A. In a preferred embodiment, the linker consists of at least one of the amino acids A or S, in particular, having amino acids AAA N-terminally and AA C-terminally or ASA N-terminally and AA C-terminally, flanking the amino acid sequence of the oligopeptide inserted at the positions identified herein.

In an embodiment, the insert does not contain any stop codons. That is, it is clear that the mutated AAV capsid protein according to the present invention contains an insert continued with the wild type amino acid present after the amino acid wherein the insert is introduced. In addition, the nucleic acid molecule according to the present invention does not encode a stop codon, thus, stopping translation of the encoded peptide.

In an embodiment, the linker, in particular, the linker mentioned above, are flanking the oligopeptide of the at least five amino acids and at most 12 amino acids, like the seven amino acids described herein.

In an embodiment, the mutated AAV capsid protein, a fragment thereof, or a homolog thereof are a capsid protein, a protein fragment or homolog thereof wherein an oligonucleotide having a size of seven amino acids having a sequence according to any one of SEQ ID No. 13 to 275. In another embodiment, the mutated AAV capsid protein, a fragment thereof, or a homolog thereof are containing the insert having a sequence of SEQ ID No. 276 to 538. The sequences according to any one of SEQ ID No. 13 to 275 are amino acid sequences showing the insert without any linker group while the insert of any one of SEQ ID No. 276 to 538 show the amino acid sequences of suitable insert to be present as inserts in the mutated AAV capsid proteins according to the present invention containing linker sequences at both sites of the peptide sequences shown in SEQ ID Nos. 13 to 275.

In a further aspect of the present invention, a mutated adeno-associated virus particle, also named vector particle, comprising the mutated adeno-associated virus capsid protein or a fragment thereof, or a homolog thereof according to the present invention is provided. This AAV particle is more efficient in targeting hepatic tissue, hepatocytes, hepatic cells and cell lines or hepatocellular carcinoma with partially higher specificity. That is, the efficiency of *ex vivo* or *in vivo* transduction of the target cell or target tissue as well as the applicability of AAV as a delivery tool is increased by increasing the capacity of the mutated AAV particle to transduce the cell with higher specificity and/or efficiency.

For example, this mutated AAV particle is useful in the manufacture of AAV particle in the manufacture of a medicament for gene therapy. For example, in case of a medicament for gene therapy, the AAV particle according to the present invention carries a functional nucleic acid fragment or a nucleic acid fragment of a molecule of interest. Said molecule of interest includes a nucleic acid fragment encoding a molecule of interest. Being more efficient and having partially higher specificity of targeting the target cells or target tissue, here targeting hepatic tissue, hepatocytes, hepatic cells and cell lines, or HCC, allows to increase efficacy of gene therapy as well as vaccination strategies for preventing or treating malignancies of the mentioned targeted cells and target tissue.

In particular, the mutated AAV particle according to the present invention is suitable for use in targeting hepatocytes and/or HCC.

The mutated AAV particle according to the present invention is particularly useful in the transfer of a gene of interest into hepatocytes or HCC, in particular, for use in gene therapy.

That is, the mutated AAV particles according to the present invention are useful in the expression of therapeutically effective transgenes. For example, metabolic factors, like enzymes may be expressed to overcome defects like genetic defects. In case of e.g. HCC the expression of tumor suppressor genes, suicide genes, pro-inflammatory factors, etc. may be envisaged.

Moreover, the mutated AAV particles are suitable to induce immunologic tolerance against transgenes or antigens introduced into the cells. This is possible particularly in the target cells and target tissue of the liver. Tolerance may help to treat autoimmune disease.

In a further aspect, the present invention relates to a nucleic acid molecule encoding the mutated AAV capsid protein or fragment thereof, or a homolog thereof according to the present invention. That is, the nucleic acid molecule according to the present invention is a nucleic acid molecule encoding the AAV capsid protein or fragment thereof as defined herein, for example, containing the oligopeptide of any one of SEQ ID No. 13 to 275 or the insert according to any one of SEQ ID Nos. 276 to 538. Of course, depending on the host cell, a codon optimization may be effected.

A further aspect relates to a nucleic acid vector, in particular, a plasmid comprising the nucleic acid molecule according to the present invention. The skilled person is well-aware of suitable plasmid and vectors. In particular, based on the host cell to be transfected, a plasmid or vector is selected accordingly. Further the nucleic acid vector may contain further genetic AAV helper elements e.g. the rep open reading frame.

In a further aspect, a host cell containing the nucleic acid vector according to the present invention or the nucleic acid molecule according to the present invention is provided.

The host cell may contain further helper plasmids or nucleic acid molecules containing e.g. the adeno-viral helper genes E4, E2A and VA for the production of the recombinant AAV particles. In an embodiment of the present invention, the host cell is a producer for the production of the AAV particle according to the present invention.

In a further aspect, the present invention relates to the mutated AAV particle according to the present invention or the nucleic acid molecule according to the present invention or the nucleic acid vector according to the present invention or the composition as described herein with suitable transgene DNA components. Said transgene DNA components may be known transgene DNA or DNA encoding suitable peptides or proteins accordingly. In another embodiment, a composition is provided comprising the mutated AAV particle according to the present invention, e.g. the composition is a pharmaceutical composition for usage in transducing hepatic tissue, hepatocytes, hepatic cells and cell lines or hepatocellular carcinoma. Further, the present invention relates to the use of the AAV particle according to the present invention, the nucleic acid molecules according to the present invention or the nucleic acid vector according to the present invention in the manufacture of an AAV particle or the manufacture of a medicament for gene therapy.

The mutated AAV capsid protein, a fragment thereof, or a homolog according to the present invention, the mutated AAV particle according to the present invention, the nucleic acid molecule according to the present invention or the nucleic acid vector according to the present invention are particularly useful in targeting hepatocytes and/or HCC as demonstrated herein.

Another aspect of the present invention relates to the mutated AAV capsid protein or a fragment thereof or a homolog thereof according to the present invention, the mutated AAV particle according to the present invention, the nucleic acid molecule according to the present invention or the nucleic acid vector according to the present invention for use in treating diseases involving hepatocytes or for treating HCC: bleeding disorders including Hemophilia A, Hemophilia B, Von Willebrand disease, metabolic disorders including Alpha-1-antitrypsin deficiency, familial hypercholesterolemia, ornithine transcarbamylase deficiency, crigler-najjar syndrome, acute intermittent porphyria, glycogen storage disease type 1a, liver cancer including hepatocellular carcinoma, hepatoblastoma, collangiocarcinoma, autoimmune disorders including multiple sclerosis, latent autoimmune diabetes, induction of tolerance towards allogenic transplants including liver, kidneys, heart, stem cells, surrogate liver-directed gene therapy including lipoproteinlipase deficiency, diabetes and ornithine transcarbamylase deficiency.

That is, the components described herein including the mutated AAV capsid protein, fragment thereof or homolog thereof, the mutated AAV particle, the nucleic acid molecule or the nucleic acid vector are suitable for use in a method of treating an individual in need thereof. Hence, another aspect relates to a method of treating an individual in need thereof comprising the step of administering a mutated AAV particle according to the present invention or a nucleic acid molecule encoding the mutated AAV virus capsid protein, a fragment thereof, or a homolog thereof according to the present invention, and/or a nucleic acid vector according to the present invention to an individual in need thereof.

Moreover, administration can be conducted by known means. In particular, administration can be conducted by intravenous route, or injection into the (hepatic) portal vein, or intrahepatically, or intratumorally, respectively.

In particular, the method of therapeutic treatment according to the present invention is a method for therapeutic treatment of diseases involving hepatocytes or for treating HCC. Examples include bleeding disorders including Hemophilia A, Hemophilia B, Von Willebrand disease, metabolic disorders including Alpha-1-antitrypsin deficiency, familial hypercholesterolemia, ornithine transcarbamylase deficiency, Crigler-Najjar syndrome, acute intermittent porphyria, glycogen storage disease type 1a, liver cancer including hepatocellular carcinoma, hepatoblastoma, collangiocarcinoma, autoimmune disorders including multiple sclerosis, latent autoimmune diabetes, induction of tolerance towards allogenic transplants including liver, kidneys, heart, stem cells, surrogate liver-directed gene therapy including lipoproteinlipase deficiency, diabetes and ornithine transcarbamylase deficiency.

In an embodiment of the present invention, the method of prophylactic or therapeutic treatment is a gene therapy including the step of administering the AAV particle according to the present invention or comprising at least one of the mutated AAV capsid protein or fragment thereof, or homolog thereof according to the present invention, a nucleic acid molecule according to the present invention and/or a nucleic acid vector according to the present invention , e.g. in form of a pharmaceutical composition. Said pharmaceutical composition or medicament may contain other suitable components including diluents, excipients or carriers.

Further, a method for *ex vivo* or *in vivo* transfer of a gene of interest into hepatocytes or HCC, in particular, by gene therapy, namely, liver directed AAV gene therapy, is disclosed.

The invention will be described further by way of examples without limiting the same thereto.

### Examples

### Characterization of MLIV capsid variants

Prior to AAV peptide display library selection the library was pre-cleared from HSPG binding capsid variants by heparin affinity column purification. Capsid variants that accumulated in liver tissue after an *in vivo* AAV peptide display library selections in the two different mouse models were named MLIV* library (Hepa129 grafted) and MLIV library (TGF-α/c-myc transgenic), respectively. 92 candidate variants for the grafted and 84 candidate variants for the transgenic mouse model have been identified. The most abundant variants of each of the two libraries were named MLIV1 (MLIV*) (SEQ ID No. 13) and MLIV3 (MLIV) (SEQ ID NO. 14) and characterized in detail. For *in vivo* evaluation, MLIV1 and MLIV3 were produced as recombinant (r)AAV vectors expressing a luciferase transgene cassette. Transgene expression was controlled by the CMV promoter, a ubiquitously expressed promoter. In detail, 4.8E11 rAAV vector particles were applied to healthy mice via tail vein injection. As control and for comparison, mice received rAAV2 and rAAV8 with natural serotype capsids delivering the same vector genome. 7, 14, and 28 days post injection, mice were injected with D-luciferin (1.5 mg per animal) and the luciferase activity in various body regions was monitored by *in vivo* imaging using a luminescence signal detecting camera. In all mouse cohorts a significant luciferase activity was detected in the upper abdomen, the liver region, of the animals. The novel capsid variants rMLIV1 and rMLIV3 demonstrated a clear and significant improvement in murine liver transduction efficiency (up to 26-fold) compared to rAAV2 (Fig. 1, A, B). Murine liver transduction efficiency of the new capsid variants is similar to the control vector (rAAV8), which is currently in use in liver-directed human clinical gene therapy trials. Regarding the biodistribution of rMLIV1 and rMLIV3 as determined by qPCR analyses of vector genomes, a clear tropism for murine liver was observed. Specifically, the amount of vector genomes detected in liver was up to 51-fold higher for the novel capsid variants than in spleen, up to 18-fold higher than in the lung, and 3-fold higher than in the heart (Fig. 2). In particular, the observed de-targeting from murine spleen, one of the main off-target tissues of AAV2 (in the murine off-target tissues: rAAV2 genome content > rMLIV capsid variant genome content (182-fold higher in spleen; up to 24-fold higher in lung, 7-fold higher in heart)) (Fig. 2), is an important novel feature of the variants according to the present invention with regard to *in vivo* gene therapy.

It is well known that the non-human primate serotype rAAV8 shows high transduction efficiency in liver tissue of mice. However, this does not correlate with the transduction efficiency obtained for human liver. In comparison, human serotype rAAV2 vectors are taken up and processed more efficiently in human hepatocytes. To investigate the efficiency of the newly developed capsid variants in the context of human hepatic tissue, primary human hepatocytes were transduced with rMLIV1 and rMLIV3 (and the controls rAAV2 and rAAV8). In addition, the same vectors were assayed on primary murine hepatocytes (GOI 1E4 rAAV vector particles per cell, 72 hrs). For quantification of luciferase expression, luciferase activity was measured in cell lysates by adding luciferin followed by luminescent signal detection (Promega Luciferase Assay kit). Here, rAAV2 and the novel capsid variants rMLIV1 and rMLIV3 were clearly superior to rAAV8, which is used in human clinical trials (Nathwani 2014 doi: 10.1056/NEJMoa1407309; Pierce 2018, doi: 10.1111/hae.13489) (Fig. 3, A and B).

Due to the strong liver tropism across species and high hepatic transduction efficiency, MLIV1 and MLIV3 represent promising new developments for *in vivo* gene therapy. Furthermore, the significantly higher efficiency compared to rAAV8 in primary human hepatocytes which have been observed following *ex vivo* transduction and which is currently confirmed in humanized mice are an excellent basis for moving these novel vectors forward as tools for liver-directed human clinical gene therapy trials.

### Characterization of HCCM capsid variants

Prior to AAV peptide display library selection the library was pre-cleared from HSPG binding capsid variants by heparin affinity column purification. Capsid variants that accumulated most abundantly in HCC tissue after *in vivo* AAV peptide display library selections in the two different mouse models were named HCCM* (selected in Hepa129 grafted mouse model) and HCCM (selected in TGF-α/c-myc transgenic mouse model). For the HCCM* selection route, before *in vivo* selection, the AAV peptide display library was pe-selected *in vitro* on target cells Hepa129. We identified 103 candidate variants in the grafted and 89 candidate variants in the transgenic mouse model. These capsid variants were further scored with regard to enrichment in HCC (target) compared to liver (main off-target) tissue. The best scored capsid variants were ranked regarding enrichment in HCC compared to all analyzed off-target tissues (liver, heart, spleen, skeleton muscle, kidney, and pancreas). The best scored capsid variants were named HCCM1 (SEQ ID No.15), HCCM2 (SEQ ID No.16), and HCCM3 (SEQ ID No.17) (HCCM library) and HCCM*1 (SEQ ID No.18), HCCM*2 (SEQ ID No.19), HCCM*3 (SEQ ID No.20), and HCCM*4 (SEQ ID No.21) (HCCM* library). They were produced as rAAV vectors expressing a luciferase reporter transgene cassette for characterization. Expression of the transgene was controlled by the SFFV promoter.

HCCM1, HCCM2, and HCCM3 were administered to TGF-a/c-myc mice via tail vein injection (1.5E11 rAAV vector particles per animal). For comparison the parental AAV (rAAV2 vector with natural occurring serotype delivering the same vector genome) was applied. 3 days post injection, mice were injected with D-luciferin (1 mg per animal) and after 5 min luciferase activity in the different body regions was measured by *in vivo* imaging analysis using a luminescence signal detecting camera (3 min exposure). In addition, from all animals tumor bearing livers were isolated 5 min after repeated administration of luciferin and the luminescence signal was determined *in situ* (3 min exposure). Exclusively, mouse cohorts treated with the novel rHCCM capsid variants demonstrated significant luciferase activity in the tumors, whereas in the rAAV2 cohort luminescence signals remained below background level (100 RLU) (Fig. 4, A, B). The novel capsid variants rHCCM1, rHCCM2, and rHCCM3 demonstrated a clear improvement in HCC transduction efficiency compared to control.

rAAV2 is very efficient in transducing human hepatoma and hepatocytes cell lines (GOI of 1000 AAV vector particles per cell, 24 hrs). To estimate the efficiency of the newly developed HCCM and HCCM* capsid variants in the context of human hepatic tissue, several human hepatoma/hepatocyte cell lines were transduced with the newly developed capsid variants rHCCM1, rHCCM2, rHCCM3, rHCCM*1, rHCCM*2, rHCCM*3, and rHCCM*4 as well as the parental control (rAAV2). rAAV vectors expressed Renilla luciferase controlled by CMV promoter. Transgene expression level was quantified by luciferase assay (Promega Renilla Luciferase Assay kit). The novel capsid variants rHCCM1, rHCCM2, rHCCM3 (Fig. 5, A to B), rHCCM*2, rHCCM*3, and rHCCM*4 (Fig. 6) demonstrated transduction efficiencies comparable or even superior to rAAV2 in these human hepatoma cell lines (Fig. 5, A and B).

To date, treatment of HCC per se and thus also AAV-based gene therapy targeting HCC is limited by low efficiency. The novel HCCM capsid variants show clearly improved transduction of HCC nodules following intravenous injection and thus represent promising candidates for novel treatment strategies employing the AAV vector system.

## Claims

1. A mutated adeno-associated virus (AAV) capsid protein or a fragment thereof wherein an insert is inserted after at least one the amino acids having the amino acid numbers 139, 161, 261, 381, 447, 453, 459, 534, 570, 573, 584, 585, 586, 587, 588, 589 of SEQ ID No. 2 corresponding to the capsid protein of AAV type 2 or the homolog capsid protein of the other serotypes of AAV, namely, AAV1, AAV3b, AAV4, AAV5, AAV6, AAV7, AAV8, AAV9, AAV10,AAV11, and AAV bovine, the insert comprises an oligopeptide of at least four amino acids and at most thirty amino acids, like consisting of five to twelve amino acids, in particular, seven amino acids, said insert may optionally has further amino acids representing a linker sequence at both sites of said oligopeptide, said linker sequence having independently from one another a size of 1 to 3 amino acids, if present; wherein the mutated AAV capsid protein or a fragment thereof or its homologs being more efficient in targeting hepatic tissue, hepatocytes, hepatic cells and cell lines or hepatocellular carcinoma (HCC) with higher specificity.

2. The mutated AAV capsid protein, a fragment thereof or a homolog thereof according to claim 1 wherein the AAV is type 1, 2, 3b, 5, 6, 7, 8, 9, 10, 4, 11, and bovine.

3. The mutated AAV capsid protein, a fragment thereof or a homolog thereof according to claim 1 wherein the insert are at the positions following at least one of the amino acids of 261, 453, 534, 570, 573, 587 and 588, more preferably 453 and 587.

4. The mutated AAV capsid protein, a fragment thereof or a homolog thereof according to any one of the preceding claims wherein the mutated AAV capsid protein, the fragment thereof, or the homolog thereof comprise at least one further mutation selected from a point mutation, an internal or terminal deletion, a second insertion and a substitution.

5. The mutated AAV capsid protein, a fragment thereof, or a homolog thereof according to any one of the preceding claims when the linker sequence is an amino acid sequence containing or consisting of the amino acids G, S and/or A, in particular, wherein the linker consists of at least one of amino acids A or S, in particular, having amino acids AAA N-terminally and AA C-terminally, or ASA N-terminally and AA C-terminally, flanking the oligopeptide of at least four to at most thirty amino acids, like at least five amino acids and at most twelve amino acids, like seven amino acids.

6. The mutated AAV capsid protein, a fragment thereof, or a homolog thereof according to any one of the preceding claims wherein the oligopeptide having a size of seven amino acids is a sequence according to any one of SEQ ID No. 13 to 275.

7. The mutated AAV capsid protein, a fragment thereof, or a homolog thereof according to any one of the preceding claims wherein the insert is any one of SEQ ID No. 276 to 538.

8. A mutated adeno-associated virus (AAV) particle comprising the mutated AAV capsid protein, a fragment thereof or a homolog thereof according to any one of claims 1 to 7.

9. A nucleic acid molecule encoding the mutated AAV capsid protein, a fragment thereof or a homolog thereof according to any one of claims 1 to 8.

10. A nucleic acid vector, in particular, a plasmid comprising the nucleic acid molecule according to claim 9.

11. A host cell containing the nucleic acid vector according to claim 10 or a nucleic acid molecule according to claim 9.

12. A use of the AAV particle according to claim 8 or the nucleic acid molecule according to claim 9 or the nucleic acid vector according to claim 10 in the manufacture of an AAV particle, or in manufacture of a medicament for gene therapy.

13. The mutated AAV capsid protein, a fragment thereof or a homolog according to any one of claims 1 to 7, the mutated AAV particle according to claim 8, the nucleic acid molecule according to claim 9, nucleic acid vector according to claim 10, or the nucleic acid vector according to claim 11 for use in transducing hepatocytes and/or HCC *in vivo* or *ex vivo.*

14. The mutated AAV capsid protein, a fragment thereof or a homolog according to any one of claims 1 to 7, the mutated AAV particle according to claim 8, the nucleic acid molecule according to claim 9, or the nucleic acid vector according to claim 10 for use in treating diseases involving hepatocytes: bleeding disorders including Hemophilia A, Hemophilia B, Von Willebrand disease, metabolic disorders including Alpha-1-antitrypsin deficiency, familial hypercholesterolemia, ornithine transcarbamylase deficiency, crigler-najjar syndrome, acute intermittent porphyria, glycogen storage disease type 1a, liver cancer including hepatocellular carcinoma, hepatoblastoma, collangiocarcinoma, autoimmune disorders including multiple sclerosis, latent autoimmune diabetes, induction of tolerance towards allogenic transplants including liver, kidneys, heart, stem cells, surrogate liver-directed gene therapy including lipoproteinlipase deficiency, diabetes and ornithine transcarbamylase deficiency; or for treating HCC

15. The mutated AAV capsid protein, a fragment thereof or a homolog according to any one of claims 1 to 7, the mutated AAV particle according to claim 8, the nucleic acid molecule according to claim 9, he nucleic acid vector according to claim 10, or the nucleic acid vector according to claim 11 for use in transfer of a gene of interest into hepatocytes or HCC, in particular, for use in gene therapy.
